# EUROPEAN PATENT SPECIFICATION

(11) **EP 3 217 980 B1**
(45) Date of publication and mention of the grant of the patent: **14.10.2020**
(21) Application number: 15859268.3
(22) Date of filing: 10.11.2015
(51) Int. Cl.: A61K 31/661, A61K 31/663, A61K 31/675, A61K 31/4412, A61K 31/4418, A61P 35/00

(54) **CICLOPIROX, CICLOPIROX OLAMINE, OR A CICLOPIROX PRODRUG FOR USE IN THE TREATMENT OF BLADDER CANCER**
CICLOPIROX, CICLOPIROX-OLAMIN ODER EINEM CICLOPIROX-PRODRUG ZUR VERWENDUNG IN DER BEHANDLUNG VON BLASENKREBS
CICLOPIROX, CICLOPIROX OLAMINE OU UN PROMÉDICAMENT À BASE DE CICLOPIROX POUR UTILISATION DANS LE TRAITEMENT DU CANCER DE LA VESSIE

(30) Priority: 11.11.2014 US 201462078069 P; 18.03.2015 US 201562134747 P
(43) Date of publication of application: 20.09.2017
(73) Proprietor: The University of Kansas, Lawrence, KS 66045 (US)
(72) Inventor: WEIR, Scott J., Overland Park Kansas 66221 (US); ANANT, Shrikant, Kansas 66223 (US)
(74) Representative: Gill Jennings & Every LLP
(86) International application number: PCT/US2015/059955
(87) International publication number: WO 2016/077346

(56) References cited:
- WO-A2-2004/007676
- US-A1- 2012 142 637
- ZHOU HONGYU ET AL: "The antitumor activity of the fungicide ciclopirox", INTERNATIONAL JOURNAL OF CA, JOHN WILEY & SONS, INC, US, vol. 127, no. 10, 1 January 2010 (2010-01-01), pages 2467-2477, XP008132831, ISSN: 0020-7136
- HO ET AL.: 'Normal and neoplastic urothelial stem cells: getting to the root of the problem' NAT.REV.UROL. vol. 9, no. 10, 01 October 2012 - 14 August 2012, pages 583 - 594, XP055441075 DOI: 10.1038/NRUROL.2012.142
- EDER ET AL.: 'Expression of transforming growth factors beta-1, beta-2 and beta-3 in human bladder carcinomas' BRITISH JOURNAL OF CANCER vol. 75, no. 12, 01 January 1997 - 01 June 1997, pages 1753 - 1760, XP055441076 DOI: 10.1038/BJC.1997.299
- HARSHMAN ET AL.: 'Ribonucleotide reductase subunit M1 expression in resectable, muscle- invasive, urethelial cancer correlates with survival in younger patients' BJU INTERNALTIONAL vol. 106, no. 11, 01 December 2010 - 15 November 2010, pages 1805 - 1811, XP055441077 DOI: 10.1111/J.1464-410X.2010.09327.X

## Description

### BACKGROUND

The molecule 6-cyclohexyl-1-hydroxy-4-methylpyridin-2(1H)-one, also known as ciclopirox, is a commercially available topical antifungal agent. Ciclopirox has been used to treat superficial mycoses and *Tinea versicolor.* Commercially available topical drug products contain the olamine salt of ciclopirox. Following oral administration to humans, ciclopirox olamine is completely absorbed followed by rapid and complete drug elimination. The oral bioavailability of ciclopirox olamine in humans has not been reported. In animals, however, the absolute bioavailability of oral ciclopirox olamine is low. Repeat oral administration of the olamine salt of ciclopirox has been associated with gastrointestinal toxicity in both animals and humans. Lastly, the olamine salt of ciclopirox does not possess water solubility required for formulation as an injectable drug product. As such, it would be beneficial to configure ciclopirox for improved water solubility in order to administer the drug by injection.

Bladder cancer is the fifth most common cancer diagnosis in the US. Because patients have a high risk of recurrence and progression, bladder cancer is the most expensive cancer to treat on a per patient lifetime basis. Despite its incidence and prevalence, bladder cancer research is woefully underfunded, resulting in little progress in improving the treatment of bladder cancer. Bladder cancer is a spectrum of two diseases, non-muscle invasive bladder cancer (NMIBC), historically referred to as "superficial bladder cancer", and muscle invasive bladder cancer (MIBC). NMIBC is treatable with endoscopic resection and topical vaccine and drug treatment administered by bladder instillation (intravesical administration). MIBC requires more aggressive intervention, usually in the form of radical cystectomy and/or systemic chemotherapy. There are three types of NMIBC lesions, noninvasive papillary carcinoma (Ta), tumor invasion of the lamina propria (T1), and carcinoma in situ (Tis). Ta and T1 tumors are classified as low-grade and high-grade tumors. High-grade Ta and T1, as well as Tis tumors have higher recurrence rates with moderate to high risk of progression to MIBC. Fifty percent of NMIBC will recur within 12 months after endoscopic resection and intravesical therapies. The five-year recurrence or new occurrence rate for NMIBC ranges from 31-78% depending on tumor type and grade, despite surgical, immunotherapy and chemotherapy interventions. Accordingly, it would be advantageous to have improved treatments of NMIBC, to prevent recurrence and progression to MIBC.

### SUMMARY

The present invention is defined in the appended claims. In one embodiment, a method of treating bladder cancer is provided. The method of treating bladder cancer can include: providing a pharmaceutical composition having ciclopirox or ciclopirox olamine or a ciclopirox-POM prodrug having a structure of one of the formulae provided herein or derivative thereof or stereoisomer thereof or pharmaceutically acceptable salt thereof; and administering the pharmaceutical composition to a subject having the bladder cancer. The ciclopirox or ciclopirox olamine or a ciclopirox-POM prodrug can be administered in a therapeutically effective amount. In one aspect, the therapeutically effective amount is sufficient for inhibiting the NOTCH signaling pathway, the Υ-secretase complex, and Notch downstream target proteins, Cyclin D1 and c-Myc. In one aspect, the therapeutically effective amount is sufficient for inhibiting the TGFβ signaling pathway. In one aspect, the therapeutically effective amount is sufficient for inhibiting the STAT3 signaling pathway. In one aspect, the therapeutically effective amount is sufficient for inhibiting growth of bladder cancer cells. In one aspect, the therapeutically effective amount is sufficient for inhibiting DNA repair. In one aspect, the therapeutically effective amount is sufficient for inhibiting ribonucleotide reductase. In one aspect, the therapeutically effective amount is sufficient for inhibiting growth of bladder cancer stem cells. In one aspect, the therapeutically effective amount is sufficient for inhibiting growth of bladder cancer cells at S-phase. In one aspect, the therapeutically effective amount is sufficient for inhibiting bladder cancer spheroid formation. In one aspect, the therapeutically effective amount is sufficient for inhibiting bladder cancer cell proliferation.

In one embodiment, a method of treating bladder cancer disease in the upper urinary tract is provided by delivering ciclopirox systemically. The method can include: providing a pharmaceutical composition having ciclopirox, ciclopirox olamine, or a ciclopirox prodrug represented by a structure of any formulae provided herein or derivative thereof or stereoisomer thereof or pharmaceutically acceptable salt thereof; and administering the pharmaceutical composition to a subject having bladder cancer disease in the upper urinary tract.

### BRIEF DESCRIPTION OF THE FIGURES

The foregoing and following information as well as other features of this disclosure will become more fully apparent from the following description and appended claims, taken in conjunction with the accompanying drawings. Understanding that these drawings depict only several embodiments in accordance with the disclosure and are, therefore, not to be considered limiting of its scope, the disclosure will be described with additional specificity and detail through use of the accompanying drawings, in which:
Figure 1 illustrates a schematic representation of a method of preparing 6-cyclohexyl-1-hydroxy-4-methylpyridin-2(1H)-one, which is referred to herein as Ciclopirox (Scheme 1).
Figure 2 illustrates a schematic representation of a method of preparing di-tert-butyl(choloromethyl)phosphate (Scheme 2).
Figure 3 illustrates a schematic representation of a method of preparing a prodrug of 6-cyclohexyl-1-hydroxy-4-methylpyridin-2(1H)-one (Scheme 3).
Figure 3A illustrates a schematic representation of a sub-reaction process identified as Scheme 3A, which can be a sub-process of Scheme 3 of Figure 3.
Figure 3B illustrates a mass spectroscopy chromatograph that shows the reaction products of Scheme 3A of Figure 3A.
Figure 3C illustrates a mass spectrometry chromatograph that shows the presence of the free acid form of ((6-cyclohexyl-4-methylpyridin-1(2H)-yl)oxy)methyl dihydrogen phosphate.
Figure 3D illustrates a dimer-type form of ciclopirox-POM prodrug.
Figure 3E illustrates a mass spectrometry chromatograph of the dimer-type form of Ciclopirox-POM prodrug of Figure 3D.
Figure 4 illustrates a schematic representation of salting ((6-cyclohexyl-4-methylpyridin-1(2H)-yl)oxy)methyl dihydrogen phosphate to obtain ((6-cyclohexyl-4-methylpyridin-1(2H)-yl)oxy)methyl phosphate disodium salt (Scheme 4); however, synthesis can result in any other pharmaceutically acceptable salt of the compound.
Figure 4A illustrates a mass spectrometry chromatograph that shows the presence of the ((6-cyclohexyl-4-methylpyridin-1(2H)-yl)oxy)methyl phosphate disodium salt of Figure 4.
Figure 5 illustrates a schematic representation of a method of preparing dibenzyl(choloromethyl)phosphate (Scheme 5).
Figure 6 illustrates a schematic representation of a method of preparing dibenzyl (((6-cyclohexyl-4-methyl-2-oxopyridin-1(2H)-yl)oxy)methyl) phosphate (Scheme 6).
Figure 7 illustrates a schematic representation of a method of preparing ((6-cyclohexyl-4-methylpyridin-1(2H)-yl)oxy)methyl phosphate disodium salt (Scheme 7).
Figure 8 illustrates a schematic representation of a method of preparing the POM reagent dibenzyl(choloromethyl)phosphate (Scheme 8).
Figure 9 includes a graph that shows ciclopirox (CPX) inhibits NMIBC (T24) bladder cancer cell proliferation across a small concentration range.
Figure 10 includes images that show CPX inhibits bladder cancer spheroid formation.
Figure 11 includes graphs that show ciclopirox (CPX) inhibits NMIBC (T24) and MIBC (253JBV) bladder cancer cell proliferation across a larger concentration range.
Figure 12A shows the mean plasma ciclopirox (CPX) concentration-time rectilinear profiles following administration of 10 mg/kg IV ciclopirox olamine (CPX-O), 10 mg/kg IV CPX-POM, 30 mg/kg PO CPX-O and 30 mg/kg PO CPX-POM to six mice per time point treatment group.
Figure 12B shows the mean plasma ciclopirox (CPX) concentration-time rectilinear profiles following administration of 10 mg/kg IV and 50 mg/kg IP CPX-POM to three mice per time point per treatment group.
Figure 13A shows the mean plasma ciclopirox (CPX) concentration-time semi-logarithmic profiles following administration of 10 mg/kg IV CPX-POM and CPX-O to six rats per treatment group.
Figure 13B shows the mean plasma ciclopirox (CPX) concentration-time rectilinear profiles following administration of 20 mg/kg IV CPX-POM, 30 mg/kg SQ CPX-POM, and 30 mg/kg CPX-O to six rats per treatment group.
Figure 14 shows the mean plasma ciclopirox (CPX) concentration-time rectilinear profiles following administration of 3 mg/kg IV CPX-POM and CPX-O to four male beagle dogs.
Figure 15 shows the mean plasma ciclopirox (CPX) concentration-time rectilinear profiles following administration of 3 mg/kg intravenous (IV) CPX-POM, 9 mg/kg subcutaneous (SQ) CPX-POM, and 9 mg/kg Oral (PO) CPX-O to four male beagle dogs.
Figure 16 includes Western blots showing apoptosis of T24 bladder cancer cells from CPX.
Figure 17 includes Western blots showing apoptosis of T24 bladder cancer cells from CPX.
Figure 18 includes a graph that shows CPX formation from CPX-POM compared to incubation time.
Figure 19 includes images that show the difference of cell growth of T24 cells in the presence of CPX compared to controls.
Figure 20 includes images that show the difference of cell growth of 253JBV cells in the presence of CPX compared to controls.
Figure 21 includes graphs that show CPX inhibits spheroid formation both in 253JBV and T24 cell lines.
Figure 22 includes images that show CPX induces cell death in T24 bladder cancer cell lines.
Figures 23A-23C include graphs that show CPX inhibits intracellular Notch, Wnt, and Hedgehog signaling pathways in T24 bladder cancer cells.
Figure 24 includes graphs and an image that shows the effect of CPX on cell proliferation in WT and NCID overexpressing T24 cells, as well as the Notch 1 and Actin levels.
Figures 25A-25D include graphs that show the effect of CPX on bladder cancer cells.
Figure 26A includes a graph that shows the bladder weight per treatment with CPX-POM.
Figure 26B includes a graph that shows the tumor stage per treatment with CPX-POM dosing.
Figure 27A includes images that show CPX-POM inhibits Notch1 signaling and γ-secretase complex proteins *in vivo.*
Figure 27B includes images that show CPX-POM inhibits Notch downstream target proteins, Cyclin D1 and c-Myc in BBN induced bladder tumor model.
Figure 28 shows CPX-POM inhibits proliferating cell nuclear antigen (PCNA) both *in vitro* and *in vivo.*

### DETAILED DESCRIPTION

In the following detailed description, reference is made to the accompanying drawings, which form a part hereof. In the drawings, similar symbols typically identify similar components, unless context dictates otherwise. The illustrative embodiments described in the detailed description, drawings, and claims are not meant to be limiting. Other embodiments may be utilized, and other changes may be made, without departing from the spirit or scope of the subject matter presented herein. It will be readily understood that the aspects of the present disclosure, as generally described herein, and illustrated in the figures, can be arranged, substituted, combined, separated, and designed in a wide variety of different configurations, all of which are explicitly contemplated herein.

Generally, the present invention relates to 6-cyclohexyl-1-hydroxy-4-methylpyridin-2(1H)-one, also known as ciclopirox, and prodrugs thereof. The ciclopirox and ciclopirox prodrugs described herein can be used in treating bladder cancer, as supported by the data of this document. The ciclopirox prodrugs can include a phosphoryloxymethyl (POM) moiety in order to deliver the active moiety, ciclopirox, to the target sites of action by improved water solubility and ability to administer the drug by injection. The ciclopirox-POM prodrug of the present invention can also include derivatives of ciclopirox that include the POM prodrug moiety, which derivative prodrugs can be referred to herein generally under the term ciclopirox-POM or may be specifically referenced as ciclopirox-POM derivatives. The ciclopirox-POM prodrugs of the present invention can be prepared with traditional chemistry techniques, such as those described herein. The ciclopirox may be used as ciclopirox olamine in the bladder cancer treatment.

The ciclopirox-POM prodrug has been shown to improve the aqueous solubility of ciclopirox, and thereby afford an opportunity to selectively deliver significant concentrations of ciclopirox to the entire urinary tract by injection of ciclopirox-POM. By delivering the biologically active ciclopirox to the entire urinary tract, administration of the ciclopirox-POM prodrug by injection overcomes the limitations of topical delivery via bladder instillation in that drug is delivered to the upper urinary tract including the ureters, collecting system, and kidneys. Accordingly, the ciclopirox-POM prodrug overcomes limitations with the formulation and/or delivery route of ciclopirox or other forms of ciclopirox, such as a ciclopirox salt like ciclopirox olamine. The improved solubility of the ciclopirox-POM prodrug can now allow for improved pharmaceutical compositions for administering effective amounts of ciclopirox by injection. The pharmaceutical compositions can be formulated to be suitable for injectable routes of administration, such as intravenous, subcutaneous, and intramuscular.

The ciclopirox-POM prodrug is configured so that the POM moiety is cleaved off by phosphatase enzymes in order to produce the original parent drug ciclopirox that is biologically active. Metabolic converstion of ciclopirox-POM to the biologically active ciclopirox is rapid and complete. As such, the ciclopirox-POM can be administered to a living subject, and then be enzymatically processed into bioactive ciclopirox within the body of the subject. While the subject usually will be human, the ciclopirox-POM prodrugs may be found to be suitable for a wide variety of animals, such as mammals, birds, reptiles, or the like.

While ciclopirox has traditionally been used as a topical antifungal agent, the different formulations available for the ciclopirox-POM prodrugs of the present invention may provide therapeutically effective amounts of ciclopirox for other maladies that have been shown or that may be developed to be suitable for ciclopirox treatment (ZHOU HONGYU et al., "The antitumor activity of the fungicide cyclopirox", Int. J. Cancer, vol. 127, no. 10, 2010, pages 2467-2477; WO2004/007676 A2). Accordingly, the ciclopirox-POM prodrugs may be used in therapies for: inhibition, treatment, and/or prevention of a fungus; treatment, prevention, and/or control of cancer (e.g., bladder cancer or others); inhibition, treatment, and/or prevention of dermatitis; inhibition, treatment, and/or prevention of superficial mycoses; for inhibition, treatment, and/or prevention of inflammation (e.g., as an anti-inflammatory, or NSAID); inhibition, treatment, and/or prevention of one or more of tinea pedis, tinea cruris, and tinea corporis, Trichophyton rubrum, Trichophyton mentagrophytes, Epidermophyton floccosum, and Microsporum canis, candidiasis (moniliasis), Candida albicans, tinea (pityriasis) vesicolor, or Malassezia furfur, and for inhibition, treatment, and/or prevention of viral infection including HIV. Now, it has been found that ciclopirox, such as ciclopirox olamine or ciclopirox prodrug, can be used in the treatments of bladder cancer. That is, the agent can be used without a prodrug moiety or with a prodrug moiety as a prodrug. Such use of ciclopirox can be administered as described herein. Also, the ciclopirox can be included in a topical delivery system to be delivered directly to the bladder cancer via bladder instillation. It has also been found that that CPX selectively eliminates viral infection by HIV-1, which can be delivered as ciclopirox, ciclopirox-POM prodrug, or ciclopirox olamine.

The ciclopirox-POM prodrug improves the solubility of the prodrug such that increased systemic and urinary tract exposure to ciclopirox is achieved. That is, more ciclopirox can be available throughout a subject as well as within individual cells of a subject. The increased amount of available ciclopirox improves the bioactivity and therapeutic potential, as well as improves the ability to modulate cellular processes. Accordingly, the ciclopirox-POM prodrug can be used for disrupting DNA repair, cell division, or intranuclear transport in a cell. Moreover, ciclopirox-POM can be used to inhibit Notch signaling, thereby affecting stemness and promote the invasion and metastasis of bladder cancer. The cells may be *in vivo, ex vivo,* or *in vitro.* Accordingly, the ciclopirox-POM can be used against a broad spectrum of cancer lines by disrupting DNA repair, cell division, or intranuclear transport in a cell, by inhibiting Notch signaling, and inhibiting any other biological pathway described herein.

While prodrugs of ciclopirox have been described, such prodrugs have been limited to conventional esters formed with available hydroxyl and/or amino groups, such as by acylation of activated acids in the presence of a base. The esters described can include prodrugs that have phenyl esters, aliphatic (C₈-C₂₄) esters, acyloxymethyl esters, carbamates, and amino acid esters (see WO 2010/048712.

Additionally, prodrugs with increased solubility have been created for compounds that include secondary and tertiary amine containing drugs. Examples of such prodrugs include N-phosphoryloxymethyl (POM) prodrugs where the methyl group of the POM moiety is coupled to a secondary or tertiary amine (see US 5,985,856).

It has now been found that alternative chemistry techniques can couple a POM prodrug entity to ciclopirox in order to form the ciclopirox-POM prodrugs of the present invention (US2012/142637 A1). The chemical synthesis protocols can conjugate ciclopirox, shown in below, through its hydroxyl group rather than through a secondary or tertiary amine. While ciclopirox does include a cyclic nitrogen, it has been found that such a nitrogen is undesirable to be linked to a POM due to the hydroxyl group or oxygen linked to the nitrogen possibly being important for the biological activity of ciclopirox. As such, the present invention provides chemistry techniques to conjugate a POM moiety to the hydroxyl group that is linked to the ring nitrogen of ciclopirox.

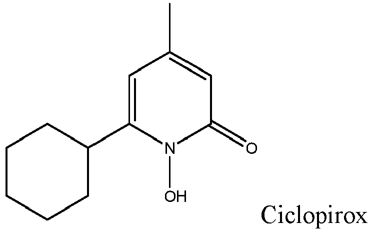

In one embodiment, the present invention includes a prodrug of ciclopirox or its derivatives. The prodrug can include a structure of Formula 1 or derivative thereof or stereoisomer thereof as well as a pharmaceutically acceptable salt thereof.

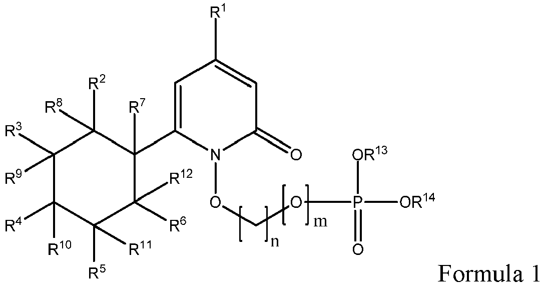

In the formulae, R¹-R¹⁴ each independently can include a hydrogen, halogens, hydroxyls, alkoxys, straight aliphatics, branched aliphatics, cyclic aliphatics, substituted aliphatics, unsubstituted aliphatics, saturated aliphatics, unsaturated aliphatics, aromatics, polyaromatics, substituted aromatics, hetero-aromatics, amines, primary amines, secondary amines, tertiary amines, aliphatic amines, carbonyls, carboxyls, amides, esters, amino acids, peptides, polypeptides, derivatives thereof, substituted or unsubstituted, or combinations thereof as well as other well-known chemical substituents. R¹³ and/or R¹⁴ can be a positive ion such as a sodium ion, such that the compound forms a salt. Also, R¹³ and/or R¹⁴ independently can include a positive ion to form a salt with the prodrug or one or more of hydrogen, halogens, hydroxyls, alkoxys, straight aliphatics, branched aliphatics, cyclic aliphatics, substituted aliphatics, unsubstituted aliphatics, saturated aliphatics, unsaturated aliphatics, aromatics, polyaromatics, substituted aromatics, hetero-aromatics, amines, primary amines, secondary amines, tertiary amines, aliphatic amines, carbonyls, carboxyls, amides, esters, amino acids, peptides, polypeptides, or combinations thereof. The methyl linker can be expanded to a larger aliphatic group that is substituted or unsubstituted if desired, such that n can be about 0-20, more preferably 1-10, and most preferably 1-4. The linker may also have one or two oxygen atoms, such that m can be 0, 1 or 2. For all formula show herein, n can be 1-4 and m is 1, or n and m can both be 1.

In one embodiment, R¹³ and/or R¹⁴ may independently be any one of various positive ions. In one example, R¹³ and/or R¹⁴ can independently include ions with positive charge of +1. The R¹³ and/or R¹⁴ can be an alkali metal. Also, the R¹³ and/or R¹⁴ can independently be a group 1 ion, such as a sodium ion. Accordingly, the bond between the oxygen and R¹³ and/or R¹⁴ can be covalent or ionic.

In one embodiment, the R¹³ and/or R¹⁴ can independently be a protecting group. Examples of protecting groups can include tert-butyl and benzyl; however, other organic-based protecting groups can be used. These protecting groups may be left on the prodrug for administration, or removed prior to administration.

In one embodiment, R¹ is a short aliphatic, such as a methyl or other alkyl.

In one embodiment, the R²-R¹² are all hydrogen.

In one embodiment, the R¹³ and/or R¹⁴ are each independently a hydrogen.

In one embodiment, the R¹³ and/or R¹⁴ are each independently a benzyl or tert-butyl.

In one embodiment, the R¹³ or R¹⁴ includes a Ciclopirox-POM and the other is hydrogen, which is shown in Formula 10 below.

In one embodiment, the present invention can include compounds with a structure of Formula 2 or derivative thereof or stereoisomer thereof. The n, m, R¹, and R¹³ and/or R¹⁴ can be the same as recited above for Formula 1.

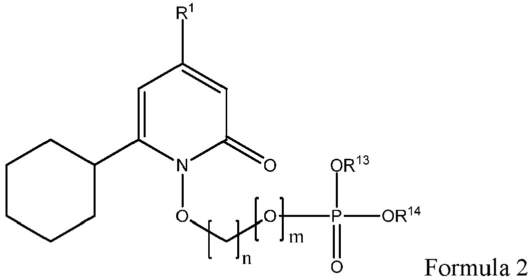

In one embodiment, the compounds can include the structure of Formula 3 or derivative thereof or stereoisomer thereof. The n, m, R¹, and R¹³ and/or R¹⁴ can be the same as recited above for Formula 1.

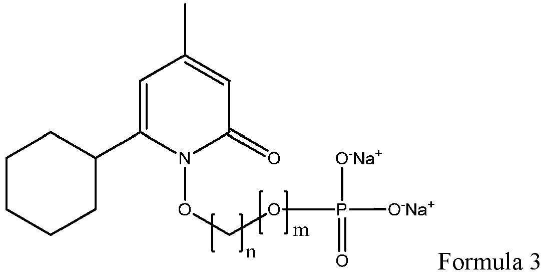

In one embodiment, the compounds can include the structure of Formula 4 or derivative thereof or stereoisomer thereof. The n and m can be the same as recited above for Formula 1.

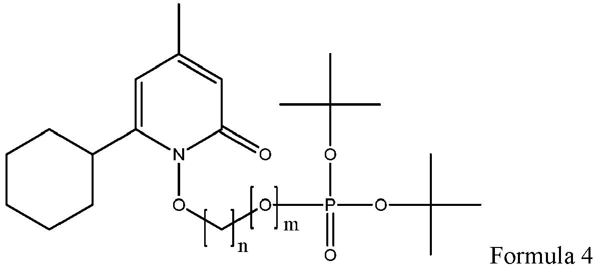

In one embodiment, the compounds can include the structure of Formula 5 or derivative thereof or stereoisomer thereof. The n and m can be the same as recited above for Formula 1.

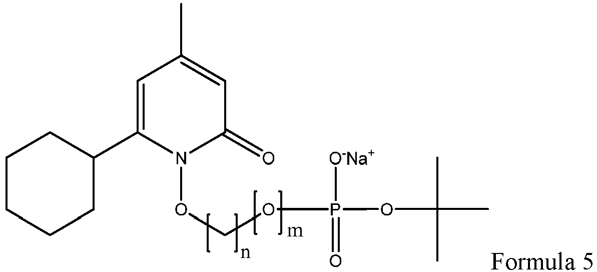

In one embodiment, the compounds can include the structure of Formula 6 or derivative thereof or stereoisomer thereof. The n and m can be the same as recited above for Formula 1.

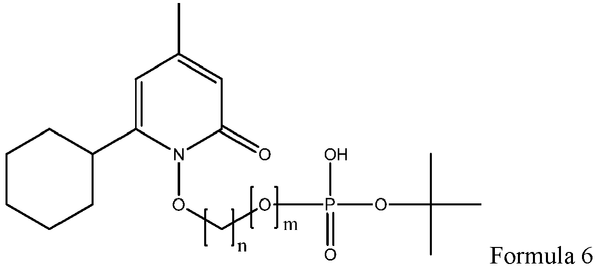

In one embodiment, the compounds can include the structure of Formula 7 or derivative thereof or stereoisomer thereof. The n and m can be the same as recited above for Formula 1.

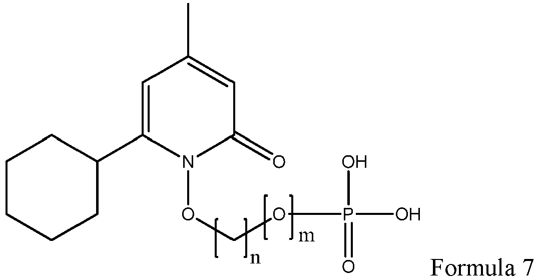

In one embodiment, the compounds can include the structure of Formula 8 or derivative thereof or stereoisomer thereof. The n and m can be the same as recited above for Formula 1.

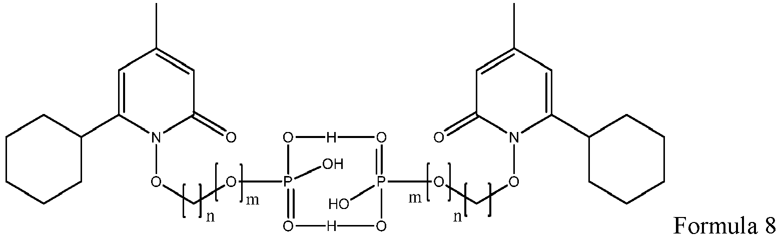

In one embodiment, the compounds can include the structure of Formula 9 or derivative thereof or stereoisomer thereof. The n and m can be the same as recited above for Formula 1.

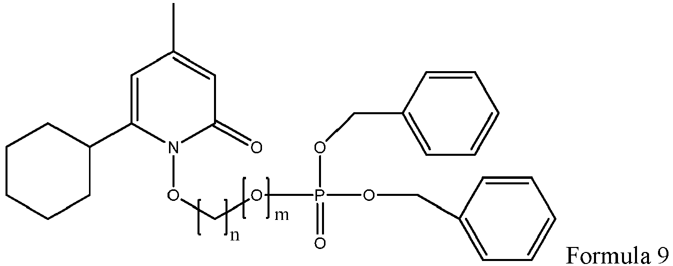

In one embodiment, the compounds can include the structure of Formula 10 or derivative thereof or stereoisomer thereof.

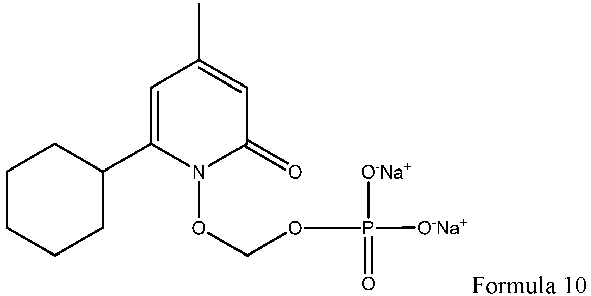

In one embodiment, the compounds can include the structure of Formula 11 or derivative thereof or stereoisomer thereof. Here, the compound has two oxygen atoms with a negative charge, which can be ionically associated with a cation so as to form a salt, such as any pharmaceutically acceptable salt.

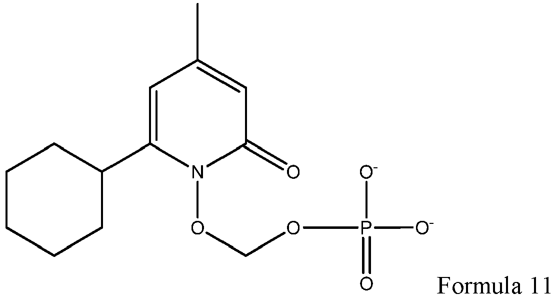

In one embodiment, the compounds can include the structure of Formula 12 or derivative thereof or stereoisomer thereof. Here, the compound has two oxygen atoms associated with a cation so as to form a salt, such as any pharmaceutically acceptable salt.

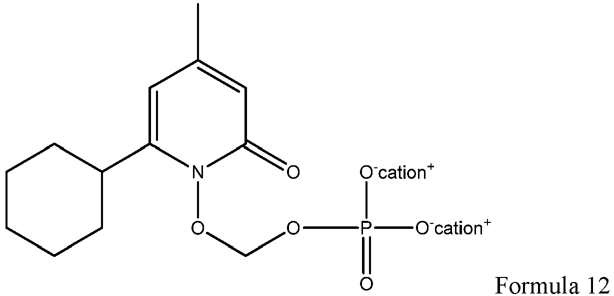

In one embodiment, the compounds can include the structure of Formula 13 or derivative thereof or stereoisomer thereof. Here, the compound has two oxygen atoms that can lose the hydrogen and then become associated with a cation so as to form a salt, such as any pharmaceutically acceptable salt.

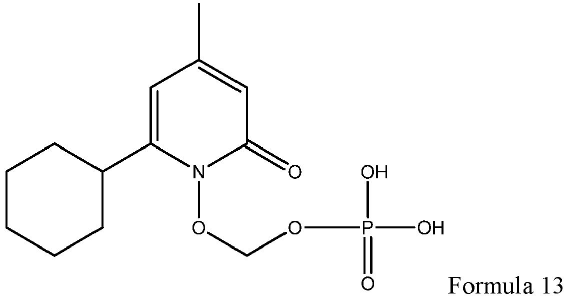

Any of the compounds may be prepared as pharmaceutically acceptable salts, if possible. Any common pharmaceutically acceptable salt ion can be used. Examples of such salts can include alkali and alkaline earth metal salts such as lithium, sodium, potassium, magnesium, calcium, strontium, and hydrates thereof; more preferably sodium, magnesium, calcium, and hydrates thereof; and still more preferably magnesium, calcium and hydrates thereof. The salts can also include the corresponding salts of various organic acids such as formic acid, acetic acid, propionic acid, trifluoroacetic acid, tartaric acid, malic acid, maleic acid, fumaric acid, succinic acid, oxalic acid, etc. and of various inorganic acids such as hydrochloric acid, hydrobromic acid, sulfuric acid, phosphoric acid and so on. Any other salt may be suitable.

Additionally, any of the compounds described herein and represented by the chemical formulae can have any of the R groups (R¹-R¹⁴) independently selected from substituents selected from the group of hydrogen, C₁-C₂₄ alkyl, C₂-C₂₄ alkenyl, C₂-C₂₄ alkynyl, C₅-C₂₀ aryl, C₆-C₂₄ alkaryl, C₆-C₂₄ aralkyl, halo, hydroxyl, sulfhydryl, C₁-C₂₄ alkoxy, C₂-C₂₄ alkenyloxy, C₂-C₂₄ alkynyloxy, C₅-C₂₀ aryloxy, acyl (including C₂-C₂₄ alkylcarbonyl (-CO-alkyl) and C₆-C₂₀ arylcarbonyl (-CO-aryl)), acyloxy (-O-acyl), C₂-C₂₄ alkoxycarbonyl (-(CO)-O-alkyl), C₆-C₂₀ aryloxycarbonyl (-(CO)-O-aryl), halocarbonyl (-CO)-X where X is halo), C₂-C₂₄ alkylcarbonato (-O-(CO)-O-alkyl), C₆-C₂₀ arylcarbonato (-O-(CO)-O-aryl), carboxy (-COOH), carboxylato (-COO⁻), carbamoyl (-(CO)-NH₂), mono-(C₁-C₂₄ alkyl)-substituted carbamoyl (-(CO)-NH(C₁-C₂₄ alkyl)), di-(C₁-C₂₄ alkyl)-substituted carbamoyl (-(CO)-N(C₁-C₂₄ alkyl)₂), mono-substituted arylcarbamoyl (-(CO)-NH-aryl), thiocarbamoyl (-(CS)-NH₂), carbamido (-NH-(CO)-NH₂), cyano(-C≡N), isocyano (-N⁺≡C⁻), cyanato (-O-C≡N), isocyanato (-O-N⁺≡C⁻), isothiocyanato (-S-C≡N), azido (-N=N⁺ =N⁻), formyl (-(CO)-H), thioformyl (-(CS)-H), amino (-NH₂), mono- and di-(Ci -C₂₄ alkyl)-substituted amino, mono- and di-(C₅-C₂₀ aryl)-substituted amino, C₂-C₂₄ alkylamido (-NH-(CO)-alkyl), C₆-C₂₀ arylamido (-NH-(CO)-aryl), imino (-CR=NH where R is hydrogen, C₁-C₂₄ alkyl, C₅-C₂₀ aryl, C₆-C₂₄ alkaryl, C₆-C₂₄ aralkyl, etc.), alkylimino (-CR=N(alkyl), where R=hydrogen, alkyl, aryl, alkaryl, aralkyl, etc.), arylimino (-CR=N(aryl), where R=hydrogen, alkyl, aryl, alkaryl, etc.), nitro (-NO₂), nitroso (-NO), sulfo (-SO₂-OH), sulfonato (-S₂-O⁻)' C₁-C₂₄ alkylsulfanyl (-S-alkyl; also termed "alkylthio"), arylsulfanyl (-S-aryl; also termed "arylthio"), C₁-C₂₄ alkylsulfinyl (-(SO)-alkyl), C₅-C₂₀ arylsulfinyl (-(SO)-aryl), C₁-C₂₄ alkylsulfonyl (-SO₂-alkyl), C₅-C₂₀ arylsulfonyl (-SO₂-aryl), phosphono (-P(O)(OH)₂), phosphonato (-P(O)(O⁻)₂), phosphinato (-P(O)(O-)), phospho (-PO₂), phosphino (-PH₂), derivatives thereof, and combinations thereof.

### PHARMACEUTICAL COMPOSITIONS

In one embodiment, the ciclopirox-POM prodrug can be included in a pharmaceutical composition. The pharmaceutical compositions can be formulated to be suitable for injectable routes of administration, such as intravenous, subcutaneous, and intramuscular. The administration can be into the kidney, or into the bladder, or into the urethra, or upstream in any blood vessel thereto.

In one embodiment, the ciclopirox or ciclopirox olamine can be included in a pharmaceutical composition. The pharmaceutical compositions can be formulated to be suitable for injectable routes of administration, such as intravenous, subcutaneous, and intramuscular. In one example, ciclopirox or ciclopirox olamine or CPX-POM can be included a local delivery system, such as a polymeric carrier that can be administered as a depot to the bladder cancer or adjacent thereto. The polymeric carrier can be nanoparticle, microparticles, hydrogels, or other that can be delivered to a local site and allow for the ciclopirox or ciclopirox olamine or CPX-POM to be delivered topically to the bladder cancer cells via bladder instillation. The delivery system can be a biodegradable delivery system or allow for diffusion of the agents therefrom.

In one embodiment, a pharmaceutical composition can include the ciclopirox-POM prodrug as described herein. For example, the pharmaceutical composition can include a pharmaceutically acceptable carrier. Since the ciclopirox-POM prodrug is now highly water soluble, the pharmaceutically acceptable carrier can include water. However, the carrier can be sufficient in order to administer the ciclopirox-POM prodrug so that it reaches the aqueous environment of a subject to which it is administered, such as a human of the cells thereof.

In one embodiment, the pharmaceutical composition can include the ciclopirox or ciclopirox olamine or ciclopirox-POM prodrug being present in an amount greater than about 0.77%, more preferably greater than about 1%; or most preferably greater than about 2%.

In one embodiment, the pharmaceutical composition can include water as a carrier.

In one embodiment, the ciclopirox or olamine thereof or ciclopirox-POM prodrug can be delivered in accordance with known delivery of ciclopirox or olamine thereof. However, local administration directly to the bladder cancer cells can be advantageous. Also, injectable routes of administration, such as intravenous, subcutaneous, and intramuscular can be suitable.

The compositions described herein can be prepared by known methods for the preparation of pharmaceutically acceptable compositions that can be administered to subjects, such that an effective quantity of the active substance is combined in a mixture with a pharmaceutically acceptable vehicle. Suitable vehicles are described, for example, in Remington's Pharmaceutical Sciences. On this basis, the compositions include, albeit not exclusively, solutions of the substances in association with one or more pharmaceutically acceptable vehicles or diluents, and contained in buffered solutions with a suitable pH and iso-osmotic with the physiological fluids.

In one embodiment, the effective amount of ciclopirox, ciclopirox olamine, or ciclopirox-POM prodrug is within the range of about 0.01 to about 200 mg/kg body weight of a subject. In one aspect, the effective amount of ciclopirox, ciclopirox olamine, or ciclopirox-POM prodrug is within the range of about 0.5 to about 50 mg/kg body weight. The ciclopirox, ciclopirox olamine, or ciclopirox-POM prodrug can be prepared into an injectable dosage form that contains from about 1 mg to about 1000 mg of ciclopirox, ciclopirox olamine, or ciclopirox-POM prodrug. In one aspect, the composition can include about 1 mg to about 200 mg of ciclopirox, ciclopirox olamine, or ciclopirox-POM prodrug per kg body weight of subject, and can be formulated into, a liquid dosage form, or an injectable dosage. However, other amounds can be used. For example, the drug can be administered over a wide range of doses, such as 0.5 mg/m² to 300 mg/m², 1 mg/m² to 200 mg/m², 5 mg/m² to 150 mg/m², 10 mg/m² to 75 mg/m², or any suitable amount.

Pharmaceutical compositions include, without limitation, lyophilized powders or aqueous or non-aqueous sterile injectable solutions or suspensions, which may further contain antioxidants, buffers, bacteriostats and solutes that render the compositions substantially compatible with the tissues or the blood of an intended recipient. Other components that may be present in such compositions include water, surfactants (e.g., Tween®), alcohols, polyols, glycerin and vegetable oils, for example. Extemporaneous injection solutions and suspensions may be prepared from sterile powders, granules, tablets, or concentrated solutions or suspensions. The composition may be supplied, for example but not by way of limitation, as a lyophilized powder which is reconstituted with sterile water or saline prior to administration to the patient.

Suitable pharmaceutically acceptable carriers include essentially chemically inert and nontoxic compositions that do not interfere with the effectiveness of the biological activity of the pharmaceutical composition. Examples of suitable pharmaceutical carriers include, but are not limited to, water, saline solutions, glycerol solutions, ethanol, N-(1 (2,3- dioleyloxy)propyl)N,N,N-trimethylammonium chloride (DOTMA), diolesyl-phosphotidyl-ethanolamine (DOPE), and liposomes. Such compositions should contain a therapeutically effective amount of the compound, together with a suitable amount of carrier so as to provide the form for direct administration to the patient.

The ciclopirox, ciclopirox olamine, or ciclopirox-POM prodrug compositions described herein can be administered for example, by parenteral, intravenous, subcutaneous, intramuscular, intracranial, intraorbital, ophthalmic, intraventricular, intracapsular, intraspinal, intracisternal, intraperitoneal, intranasal, aerosol, transdermal, or oral administration. Common carriers or excipients can be used for preparing pharmaceutical compositions designed for such routes of administration.

Another aspect provides a commercial package comprising a composition described herein, and associated therewith instructions for the use thereof in the treatment of bladder cancer with ciclopirox, ciclopirox olamine, or ciclopirox-POM prodrug. Another embodiment provides a commercial package comprising a composition described herein, and associated therewith instructions for the inducing cell death and/or inhibiting surviving activity or level in treatment of bladder cancer with ciclopirox, ciclopirox olamine, or ciclopirox-POM prodrug.

In one embodiment, the composition is devoid of an olamine. In fact, the prodrug can be devoid of an olamine. Accordingly, the composition can be devoid of a ciclopirox olamine or derivative thereof. In one embodiment, the composition can be devoid of ciclopirox that lacks a prodrug moiety.

In one embodiment, the ciclopirox, ciclopirox olamine, or ciclopirox-POM prodrug can be present in a therapeutically effective amount for use in the treatment of bladder cancer.

### SYNTHESIS

Generally, ciclopirox can be obtained and then reacted through the reaction protocols described herein in order to produce the ciclopirox-POM prodrug. Also, a ciclopirox derivative can be obtained and reacted following the synthetic protocols described herein, where a ciclopirox derivative reagent will result in a corresponding ciclopirox-POM derivative prodrug. In some instances, the ciclopirox or derivative thereof can be obtained in a salt, such as an olamine salt. Accordingly, the reaction scheme can include desalting the ciclopirox prior to conjugation with the POM prodrug moiety.

In the figures and associated following descriptions of chemical reactions and the corresponding reactants, abbreviations are used to describe chemicals, which abbreviations are defined as follows: EDAC is N-(3-Dimethylaminopropyl)-N'-ethylcarbodiimide hydrochloride; DIAD is diisopropyl azodicarboxylate; BBDI is 1-tert-Butoxy-2-tert-butoxycarbonyl-1,2-dihydroisoquinoline; DICD is diisopropyl carbodiimide; and DBP is dibenzyl phosphate.

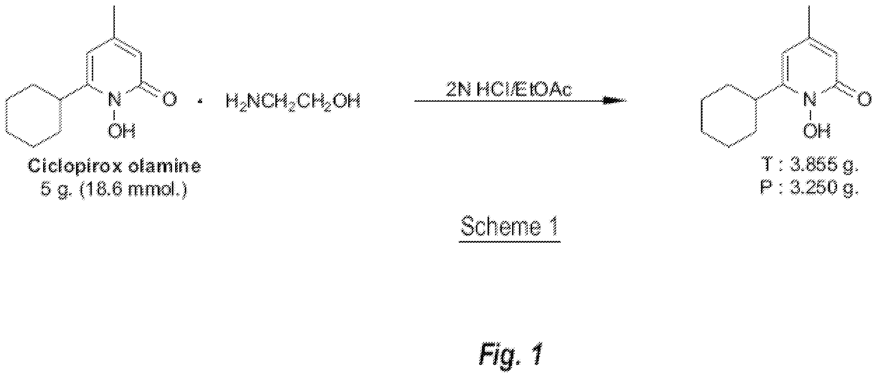

In one embodiment, the present invention provides a method of preparing 6-cyclohexyl-1-hydroxy-4-methylpyridin-2(1H)-one, which method can include reaction Scheme 1 as shown in Figure 1. The reaction of Scheme 1 prepares a ciclopirox from an olamine salt thereof. Briefly, ciclopirox olamine (5 g, 18.6 mmol) can be dissolved in 2 N HCl, extracted with EtOAc, and precipitated with hexane in order to obtain ciclopirox (about an 84% yield).

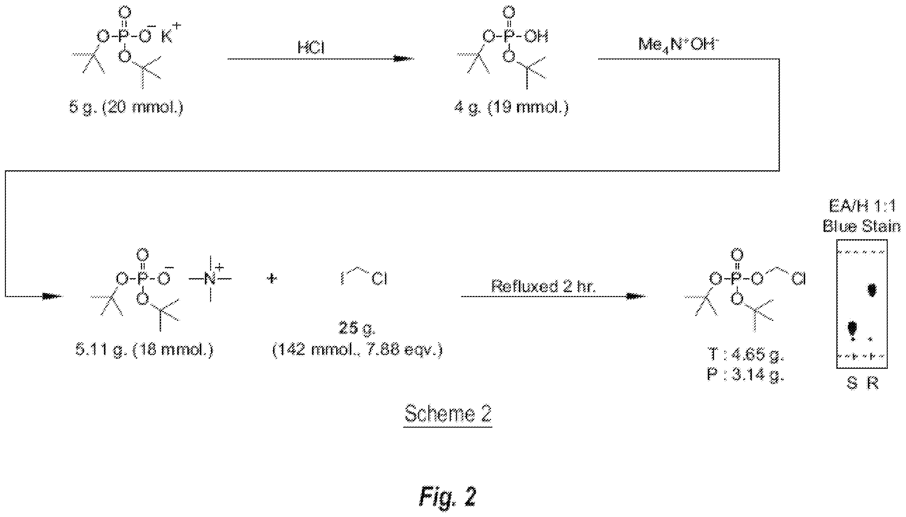

In one embodiment, the present invention provides a method of preparing a reagent for use in preparing a prodrug of 6-cyclohexyl-1-hydroxy-4-methylpyridin-2(1H)-one, which method can include reaction Scheme 2 as shown in Figure 2. The reaction Scheme 2 provides a reagent for preparation of a reagent of the POM prodrug moiety, which is di-tert-butyl(choloromethyl)phosphate. Briefly, potassium di-tert phosphate (5 g, 20 mmol) is dissolved in a minimum amount of cold water and 6 N HCl is added drop-wise in order to form a precipitate, and then the precipitate is washed with cold water, which is then filtered and dried under vacuum in order to form di-tert phosphate. The di-tert-phosphate (4 g, 19 mmol) is then dissolved in about 100 mL acetone with tetramethylammonium hydroxide added drop-wise until reaching about pH 7, and then the solvent is removed and dried under vacuum to produce tetra-methyl ammonium di-tert-butyl phosphate. Tetra-methyl ammonium di-tert-butyl phosphate (5.11 g, 18 mmol) is then reacted with iodocholoro methane (CH₂ClI) (25 g, 142 mmol, 7.88 equivalent) in about 150 ML DME and refluxed for about 2 hours before being filtered to remove the precipitate, removal of the solvent, and then dissolved in EA/H, and then filtered through a silica bed, and the solvent is removed and the product is dried to obtain di-tert-butyl(choloromethyl)phosphate. A TLC is shown to confirm product.

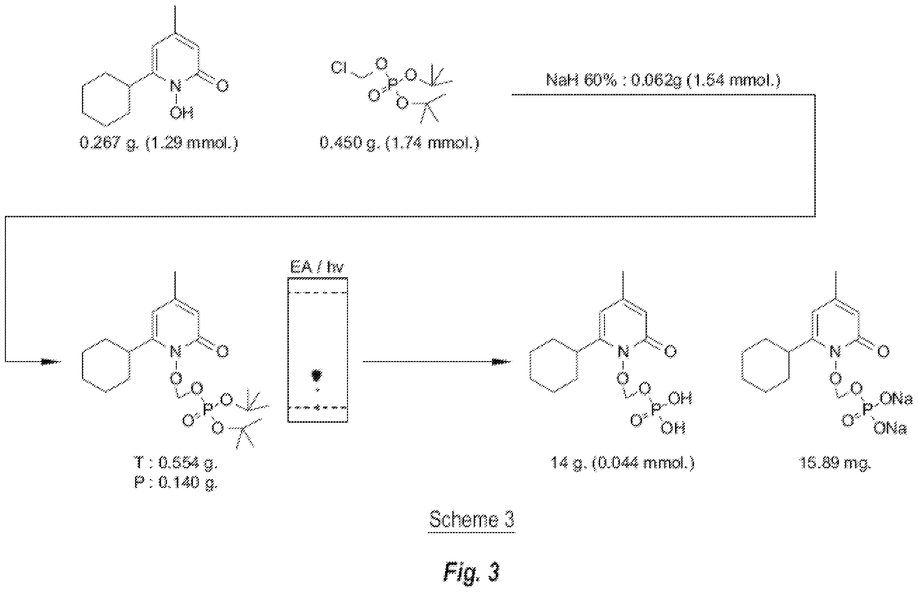

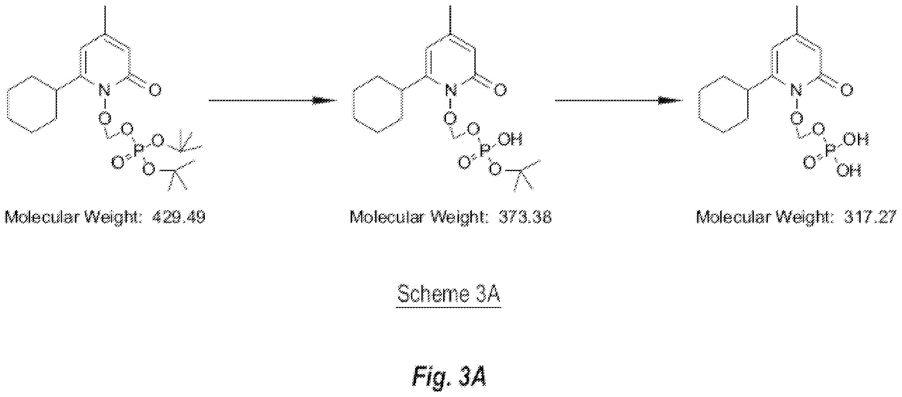

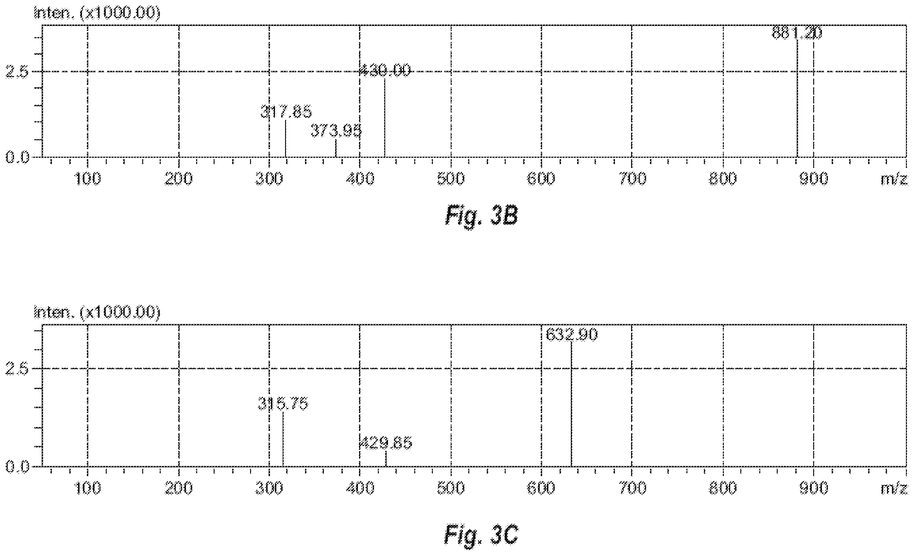

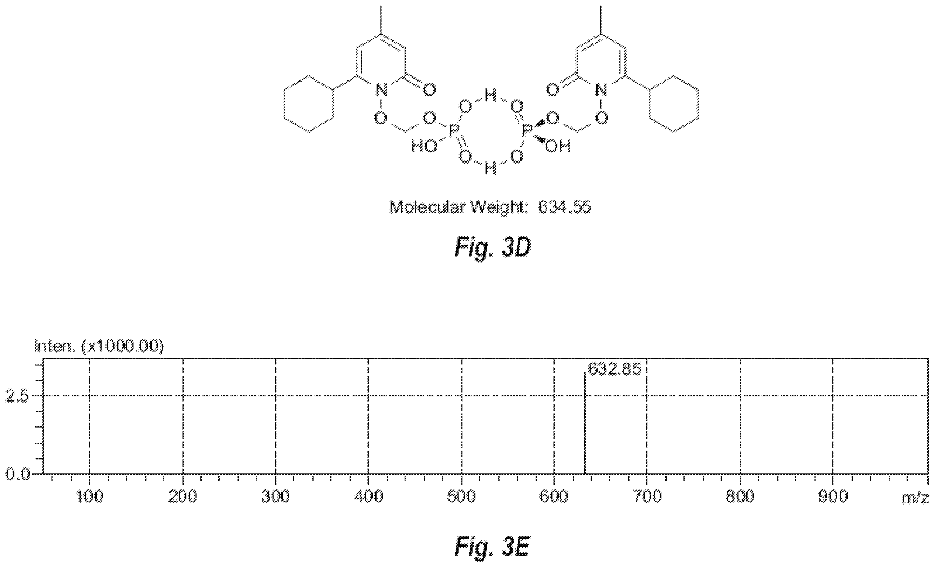

In one embodiment, the present invention provides a method of preparing a prodrug of 6-cyclohexyl-1-hydroxy-4-methylpyridin-2(1H)-one (i.e., ((6-cyclohexyl-4-methylpyridin-1(2H)-yl)oxy)methyl phosphate disodium salt), which method can include reaction Scheme 3 as shown in Figure 3. The reaction Scheme 3 reacts ciclopirox with di-tert-butyl(choloromethyl)phosphate to form a ciclopirox-POM that includes a disodium salt. Briefly, ciclopirox (0.257 g, 1.25 mmol) and di-tert-butyl(choloromethyl)phosphate (0.450 g, 1.74 mmol) are dissolved in 5 mL and reacted with 60% NaH (0.62g, 154. mmol) at 0°C for 30 minutes and then room temperature for 2 hours before being quenched with water, and then the solvent is removed and process through a separation column with EA/H (1:1) to yield di-tert-butyl (((6-cyclohexyl-4-methylpyridin-1(2H)-yl)oxy)methyl) phosphate, which was confirmed via TLC. The di-tert-butyl (((6-cyclohexyl-4-methylpyridin-1(2H)-yl)oxy)methyl) phosphate was then dissolved in 10 mL THF/CH₂Cl₂ (3:1), and incubated at room temperature for 2 hours before the solvent was removed and purified via RPHPLC, and then equamolar Na₂CO₃ in water/ACN was added and lyophilized to obtain ((6-cyclohexyl-4-methylpyridin-1(2H)-yl)oxy)methyl dihydrogen phosphate (14 mg, 0.044 mmol) and ((6-cyclohexyl-4-methylpyridin-1(2H)-yl)oxy)methyl phosphate disodium salt (15.89 mg), which is referred to herein as the primary ciclopirox-POM prodrug.

Figure 3A shows a sub-reaction process identified as Scheme 3A, which can be a sub-process of Scheme 3. Scheme 3A shows the di-tert-butyl (((6-cyclohexyl-4-methylpyridin-1(2H)-yl)oxy)methyl) phosphate converting to tert-butyl (((6-cyclohexyl-4-methylpyridin-1(2H)-yl)oxy)methyl) hydrogen phosphate, which converts to ((6-cyclohexyl-4-methylpyridin-1(2H)-yl)oxy)methyl dihydrogen phosphate. Figure 3B shows a mass spectrometry chromatograph that shows the presence of these chemical entities.

The free acid form of the ciclopirox-POM prodrug (i.e., ((6-cyclohexyl-4-methylpyridin-1(2H)-yl)oxy)methyl dihydrogen phosphate) was also purified by RPHPLC as shown in Figure 3C. The purified product was shown to have a mass spectrometry chromatograph as shown in Figure 3C.

Additionally, another dimer-type form of ciclopirox-POM prodrug has been created as shown in Figure 3D, which is referred to as the ciclopirox-POM dimer prodrug. The ciclopirox-POM dimer prodrug is confirmed with mass spectrometry chromatograph as shown in Figure 3E.

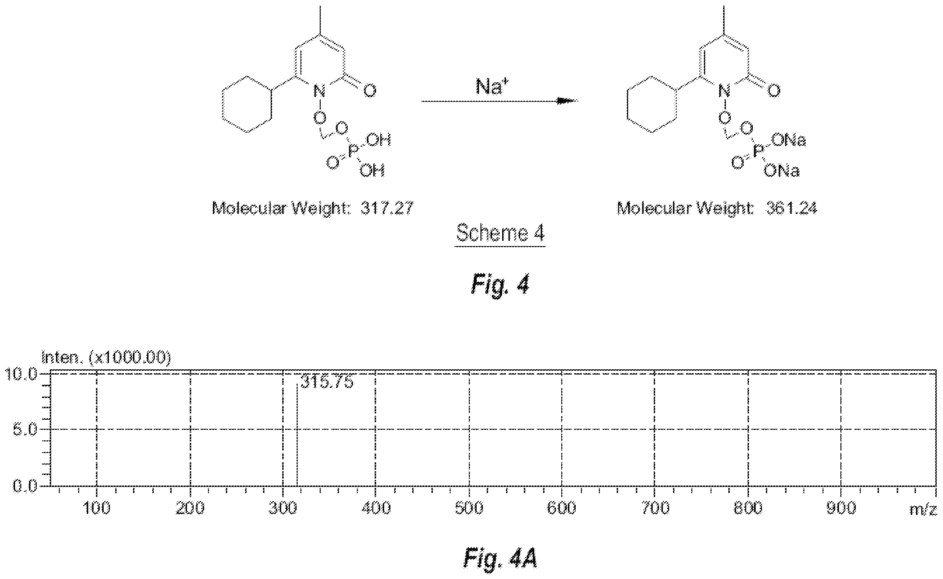

Figure 4 illustrates Scheme 4 which is the process of salting ((6-cyclohexyl-4-methylpyridin-1(2H)-yl)oxy)methyl dihydrogen phosphate with sodium ions in order to obtain ((6-cyclohexyl-4-methylpyridin-1(2H)-yl)oxy)methyl phosphate disodium salt, which is confirmed with mass spectrometry chromatograph as shown in Figure 4A.

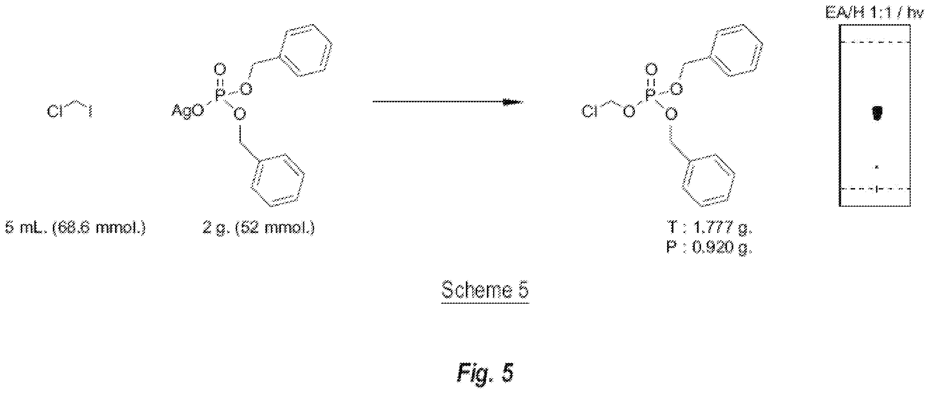

In one embodiment, the present invention can include a method of preparing the POM reagent dibenzyl(choloromethyl)phosphate, as shown in reaction Scheme 5 of Figure 5. Briefly, CH₂ClI (5 mL; 68.6 mmol) is reacted with silver dibenzyl phosphate (2 g, 52 mmol) in about 25 mL toluene and refluxed for about 1 hour before the solvent is removed and processed through a separation column with 1/1EA/H in order to produce dibenzyl(choloromethyl)phosphate as confirmed via TLC.

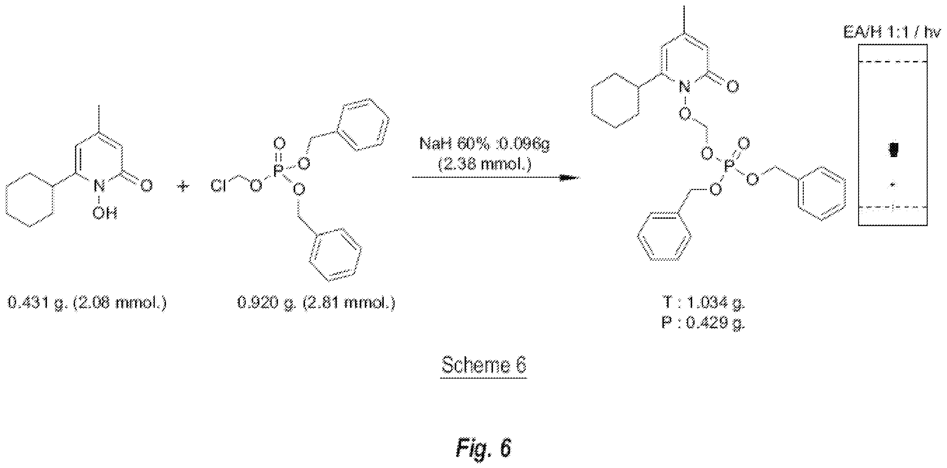

In one embodiment, the present invention provides a method of preparing a prodrug dibenzyl (((6-cyclohexyl-4-methyl-2-oxopyridin-1(2H)-yl)oxy)methyl) phosphate, which is shown by Scheme 6 of Figure 6. Briefly, ciclopirox (0.431 g, 2.08 mmol) is dissolved in 10 mL DMF with dibenzyl(choloromethyl)phosphate (0.920 g, 2.81 mmol) along with 60% NaH (0.096 g, 2.38 mmol) and incubated at 0°C for 30 minutes and then at room temperature for 1 hour before being quenched with water. The solvent is then removed and processed through a separation column with EA/H 1:1 in order to yield dibenzyl (((6-cyclohexyl-4-methyl-2-oxopyridin-1(2H)-yl)oxy)methyl) phosphate, which is confirmed via the TLC as shown.

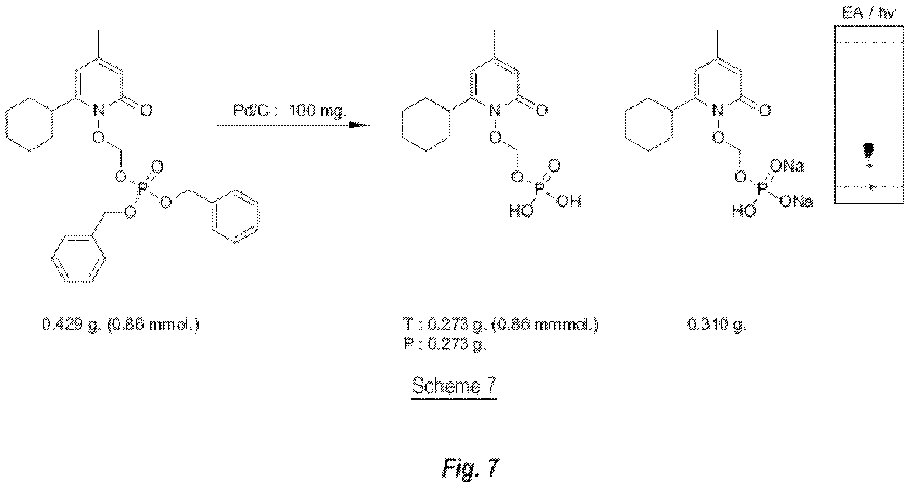

In one embodiment, dibenzyl (((6-cyclohexyl-4-methyl-2-oxopyridin-1(2H)-yl)oxy)methyl) phosphate is converted into ((6-cyclohexyl-4-methylpyridin-1(2H)-yl)oxy)methyl phosphate disodium salt as shown in Scheme 7 of Figure 7. Briefly, dibenzyl (((6-cyclohexyl-4-methyl-2-oxopyridin-1(2H)-yl)oxy)methyl) phosphate is dissolved in 25 mL THF in the presence of 100 mg Pd/C, and under hydrogen (H₂) at room temperature for 3 hours before the solvent was removed and the product dissolved in ACN. Na₂CO₃ in water is then added and lyophilized. The product is determined to be a minor amount of ((6-cyclohexyl-4-methylpyridin-1(2H)-yl)oxy)methyl dihydrogen phosphate (about 0.273 g, 0.86 mmol) and the major product being ((6-cyclohexyl-4-methylpyridin-1(2H)-yl)oxy)methyl phosphate disodium salt (0.310 g), which was confirmed via TLC as shown.

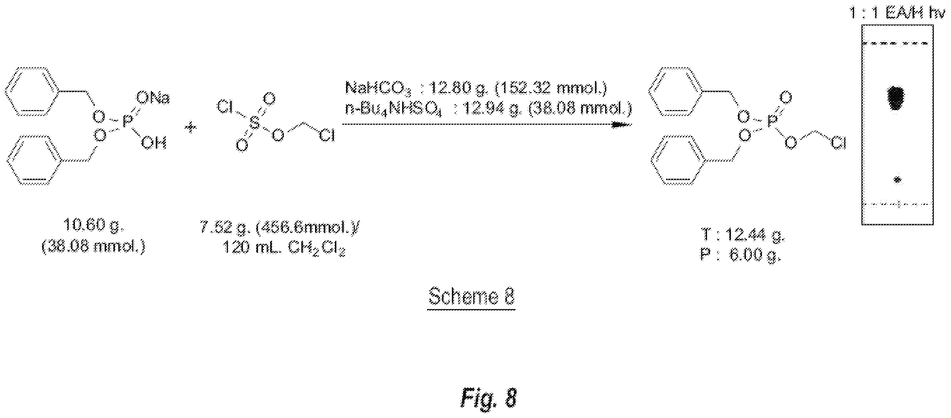

In one embodiment, another method of preparing the POM reagent dibenzyl(choloromethyl)phosphate, as shown in reaction Scheme 8 of Figure 8. Briefly, dibenzylphosphate (10.6 g; 38.08 mmol) and chloromethyl sulfochloridate (7.52 g, 456.6 mmol/120 mL CH₂Cl₂) are dissolved in 320 mL water and 200 mL CH₂Cl₂ with NaHCO₃ (12.80 g, 152.32 mmol) and n-Bu₄NHSO₄ (12.94 g, 38.08 mmol) at 0°C to room temperature overnight, and then processed through a separation column with ½ EA/H, EA. This reaction yields about 48% of dibenzyl(choloromethyl)phosphate (6 g), which is confirmed with TLC as shown.

In one embodiment, the present invention provides another method of preparing a prodrug dibenzyl (((6-cyclohexyl-4-methyl-2-oxopyridin-1(2H)-yl)oxy)methyl) phosphate, which is shown by Scheme 6 of Figure 6. Briefly, Ciclopirox (1 g, 4.82 mmol) is dissolved in 20 mL DMF with dibenzyl(choloromethyl)phosphate (2 g, 6.12 mmol) along with 60% NaH (0.375 g) and incubated at 0°C for 30 minutes and then at room temperature for 1 hour before being quenched with water. The solvent is then removed, and the product is dissolved in EtOAc, washed with water, solvent is removed, and processed through a separation column with EA/H 1:2, and RPHPLC in order to yield 67% dibenzyl (((6-cyclohexyl-4-methyl-2-oxopyridin-1(2H)-yl)oxy)methyl) phosphate (1.616 g).

### EXPERIMENTAL

The data in the figures and tables indicates that ciclopirox-POM prodrug as described herein can be an improvement for administration to subjects. It was found that ciclopirox was readily bioavailable when given IV as the prodrug, compared to IV administration of ciclopirox olamine to mice, rats and dogs, and thereby the ciclopirox-POM is an improvement in chemical formulation. Following oral administration to mice, however, bioavailability is low, 21% following administration of the olamine salt, and 12% following oral administration of ciclopirox-POM prodrug. Accordingly, ciclopirox is readily bioavailable when given IV as the prodrug. Based on the inability to detect the prodrug in plasma and urine following IV, oral, subcutaneous and intraperitoneal administration, it appears the prodrug is rapidly and completely metabolized to ciclopirox when it reaches the systemic circulation. Thus, ciclopirox-POM prodrug has advantages over ciclopirox olamine, from a physicochemical properties standpoint, and can be formulated into a suitable injectable product. Based on the data, it is reasonable to believe that the ciclopirox-POM prodrug is rapidly converted to ciclopirox.

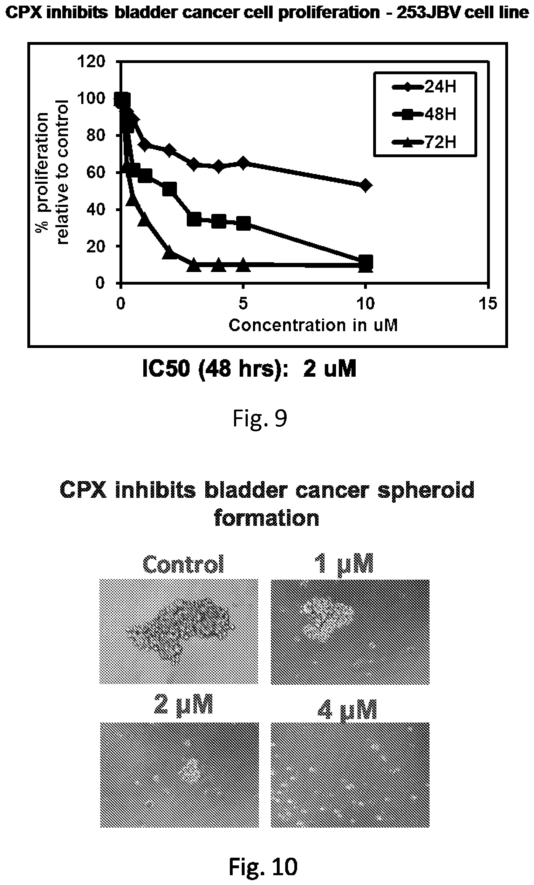

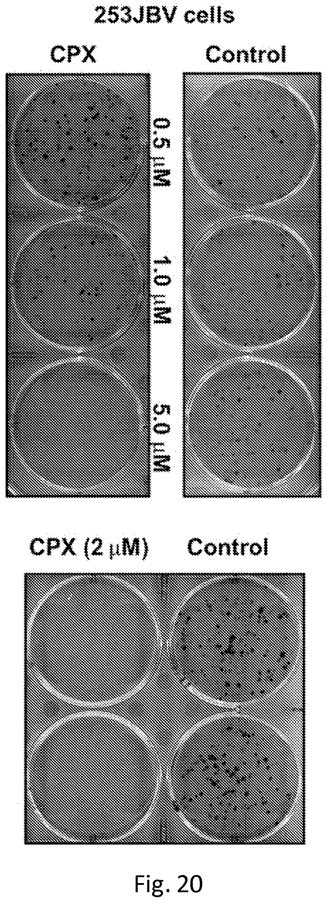

It has now been found that the compounds of the present invention are useful for treating bladder cancer. The bladder cancer can include NMIBC (non-muscle invasive bladder cancer) and MIBC (Muscle invasive bladder cancer), as well as other bladder cancers. See Figures 9-28. The compounds (Formula 10) have been shown to have anti-bladder cancer activity. Giving the prodrug by systemic injection (e.g., intravenous, intramuscular, subcutaneous, etc.) results in active, clinically relevant concentrations (e.g., significant concentrations) of the active agent ciclopirox being selectively delivered to the entire urinary tract. This can treat non-muscle invasive or superficial bladder cancer.

The prodrug converts to the active ciclopirox compound that effectively suppresses stem cells in bladder cancer and specifically targets the Notch-1 signaling pathway. It has been demonstrated that in the presence of the compound, signaling is significantly suppressed and the compound is affecting bladder cancer through this mechanism. Suppressing Notch is a significant activity for the compound to inhibit bladder and other cancers where Notch signaling is active (e.g. colon cancer, osteosarcoma).

NMIBC starts out by a cancer of the outer lining (urothelium) of the urinary tract (e.g., the lining that's up against the urine), and the cancer cells that are on the outer lining start invading inwardly into the muscle of the bladder, and then the cancer spreads throughout the body. However, the activity of ciclopirox and the delivery as the prodrug allows the drug to inhibit a pathway that is associated with the invasiveness of this particular cancer.

The ciclopirox-POM prodrug, based on activity of ciclopirox, may also be used to induce autophagy, inhibit mTOR, downregulate cyclins A, B1, D1 and E as well as CDK2 and CDK4, and other activites.

The methods of treating bladder cancer with the prodrug can include administering ciclopirox-POM prodrug (e.g., prodrug) systemically for the treatment of bladder cancer, which provides surprising and unexpected results, such as described herein.

It has been found that systemic administration of ciclopirox-POM prodrug results in the selective delivery of clinically significant concentrations of the active, anticancer agent, ciclopirox (free acid) to the entire urinary tract.

Current treatment of non-muscle invasive bladder cancer (NMIBC, formerly referred to as superficial bladder cancer) is via bladder instillation. There are no current drug therapies for NMIBC administered by any route other than bladder instillation. Bladder instillation of anticancer agents only exposes bladder cancer to anticancer agents for the period of time patients are able to retain fluids in their bladders (e.g., two hours). Secondly, bladder instillation does not deliver drug to the entire urinary tract, i.e., the ureters, collecting system, and kidneys that anatomically sits above the bladder. Up to 10% of NMIBC patients have upper urinary tract disease. Contrary thereto, the present invention includes systemic delivery (e.g., injection) that provides active drug to the entire urinary tract. However, the therapeutics may also be delivered by bladder installation.

Bladder cancer presents as two diseases, NMIBC and muscle invasive bladder cancer (MIBC). Approximately three-fourths of newly diagnosed bladder cancer patients present with NMIBC. NMIBC, as it invades into bladder muscle, becomes MIBC. MIBC often metastasizes, requiring systemically administered chemotherapy agents. It has now been surprising and unexpectedly demonstrated that ciclopirox possesses anticancer activity in NMIBC and MIBC bladder cancers, and systemic delivery of ciclopirox-POM prodrugprovides the ciclopirox to these bladder cancer cells. The ciclopirox from ciclopirox-POM prodrugdelivery inhibits cell proliferation, colony formation, and spheroid formation in well characterized NMIBC and MIBC cell lines. Although that ciclopirox's fungicidal activity is mediated through its ability to chelate iron, and interrupt iron-dependent intracellular processes, ciclopirox in vitro anti-cancer activity in independent of commercially available iron chelators. For the first time, it was demonstrated that ciclopirox inhibits the Notch-1 signaling pathway, which is a pathway recently identified as being overexpressed in invasive bladder cancer, and a pathway that is overexpressed in cancer stem cells across a number of cancers. Accordingly, ciclopirox-POM prodrug can be used in treatment of all forms of bladder cancer, and in cancer stem cells of many different cancers where the Notch signaling pathway is active.

### Ciclopirox-POM Prodrug Selectively Delivers Clinical Significant Concentrations of Ciclopirox to the Entire Urinary Tract

We have conducted a series of pharmacokinetic studies in mice, rats and dogs demonstrating the following. ciclopirox-POM prodrug rapidly and completely disappears from plasma following systemic administration. The bioavailability of ciclopirox following systemic administration of ciclopirox-POM prodrug is excellent, ranging from 77-137% in mice, rats and dogs. Following systemic administration of ciclopirox-POM prodrug, the dose is eliminated from the body via renal clearance of ciclopirox and the glucuronide metabolite of ciclopirox (CPX-G). Drug elimination is consistent with that reported for ciclopirox olamine (CPX-O). At doses of ciclopirox-POM prodrug well below maximum tolerated dose, urine concentrations of ciclopirox are observed that exceed in vitro IC50 values determined in NMIBC and MIBC cell lines.

Pharmacokinetic studies have been conducted in mice, rats and dogs, demonstrating that ciclopirox-POM prodrug is rapidly and completely metabolized to form ciclopirox. Rapid metabolism of ciclopirox-POM prodrug occurs in vivo to form ciclopirox. Ciclopirox-POM prodrugis not detected in plasma nor urine following IV administration in mice, rats, and dogs. Plasma ciclopirox concentrations are greatest immediately following IV bolus administration (within five minutes), and decline thereafter in mice, rats and dogs. The POM moiety is well understood to be metabolized by phosphatases that circulate in blood. Complete metabolism of ciclopirox-POM prodrug forms ciclopirox. Following administration of IV ciclopirox-POM prodrug to mice, ciclopirox is completely bioavailable compared to IV bolus administration of ciclopirox olamine

### Clopirox-POM Prodrug Pharmacokinetics In Mouse

**Study 208-02. Bioavailability of Ciclopirox Following Oral and Intravenous Administration of Ciclopirox-POM Prodrug in the Mouse.** Pharmacokinetic proof of principle for ciclopirox-POM prodrug was determined in the mouse following administration of ciclopirox-POM prodrug and ciclopirox olamine given orally and intravenously at doses of 30 mg/kg and 10 mg/kg, respectively, to six mice per time point per treatment group. The objectives of this study were to characterize the rate and extent of CPX-POM metabolism and assess the feasibility of orally administered ciclopirox-POM prodrug in support of future cancer proof of principle studies.

Following oral and IV administration of ciclopirox-POM prodrug, the prodrug was not detected in plasma. Further, ciclopirox AUC following IV administration of ciclopirox-POM prodrug was 137%, compared to IV administration of ciclopirox olamine, demonstrating not only rapid but complete metabolism of the prodrug, to ciclopirox presumably by plasma phosphatases. The oral bioavailability of ciclopirox following administration of ciclopirox olamine (21%) and ciclopirox-POM prodrug (12%) was low, suggesting significant first-pass effect associated with this route of administration.

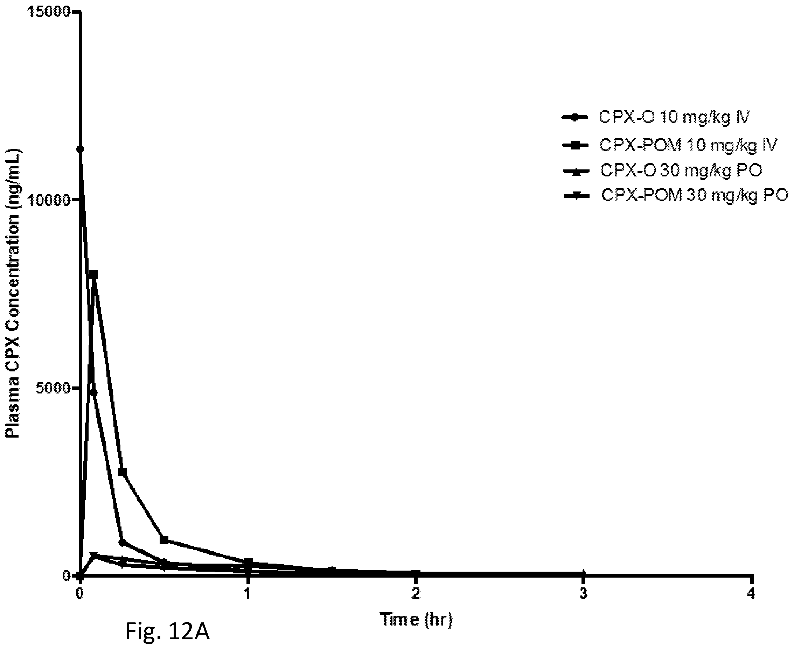

Figure 12A shows mean plasma ciclopirox (CPX) concentration-time rectilinear profiles following administration of 10 mg/kg IV ciclopirox olamine (CPX-O), 10 mg/kg IV ciclopirox-POM prodrug (CPX-POM), 30 mg/kg PO CPX-O and 30 mg/kg PO CPX-POM. Table 1A summarizes the resultant pharmacokinetic data.

**Table 1A. Plasma CPX pharmacokinetic parameters in male C57/B16 mice following single doses of IV and oral CPX-O and CPX-POM**

| **Treatment** | **CPX-O IV** | **CPX-POM IV** | **CPX-O Oral** | **CPX-POM Oral** |
|---|---|---|---|---|
| Mean Body Weight kg) | 0.020 | 0.020 | 0.020 | 0.020 |
| CPX Dose (mg/kg)^{b} | 10 | 10 | 30 | 30 |
| C₀ (ng/mL) | 11338 | | | |
| Cmax (ng/mL) | | 8005 | 548 | 520 |
| Tmax (hr) | | 0.083 | 0.25 | 0.25 |
| Kₑₗ (hr⁻¹) | 1.136 | 1.890 | 0.549 | 0.496 |
| T_{½} (hr) | 0.610 | 0.367 | 1.26 | 1.40 |
| AUC₀∫ⁿ (ng*hr/mL) | 1533 | 2183 | 821 | 476 |
| AUC₀ ∫^{∞} (ng*hr/mL) | 1544 | 2213 | 957 | 575 |
| AUMC₀ ∫ⁿ (ng*hr²/mL) | 358 | | | |
| AUMC₀∫^{∞} (ng*hr²/mL) | 401 | | | |
| MRT (hr) | 0.259 | | | |
| Cl (mL/hr/kg) | 6475 | | | |
| Vd_{z} (mL/kg) | 5700 | | | |
| Vₛₛ (mL/kg) | 1680 | | | |
| F(%)^{d} | | 137 | 20.6 | 12.4 |

| | | | | |
|---|---|---|---|---|
| ^{a} N=6 mice per time point per treatment ^{b} Ciclopirox free acid dose administered, CPX-O was formulated in 25mM Phosphate Buffer pH7 with 50mM Captisol® at a strength of 2.0 mg/mL CPX; CPX-POM disodium salt was formulated in 25mM Phosphate Buffer pH7 at a strength of 2.69 mg/mL ^{c} C₀ corresponds to y-intercept value following IV bolus administration derived from non-parametric pharmacokinetic data analysis ^{d} Absolute bioavailability determined using CPX-O IV as the reference treatment | | | | |

### Study 11177. Ciclopirox-POM Prodrug Absolute Bioavailability following IP Administration in the Mouse.

The plasma and urine pharmacokinetics of ciclopirox (CPX) were characterized in the mouse following 10 mg/kg IV and 50 mg/kg IP administration of ciclopirox-POM prodrug (CPX-POM). The objective of this study was to characterize the absolute bioavailability of ciclopirox-POM prodrug following IP administration to support IP dosing in preclinical proof of principle studies in validated models of bladder cancer. In addition, we characterized the urinary excretion of CPX and ciclopirox glucuronide (CPX-G) to describe urinary tract exposure to CPX following IP administration of CPX-POM. Two treatment groups of 30 C57BL/6 mice each were treated with CPX-POM. Prior to and over six hours post-dose, three animals were sacrificed at each selected plasma sampling time point. Blood (plasma) was collected by cardiac puncture for bioanalysis. Plasma was pooled at each time point. In addition, two groups of six mice each were administered 10 mg/kg IV and 50 mg/kg IP CPX-POM to characterize urine pharmacokinetics. Mice were placed in metabolism cages with urine collected over 24 hours post-dose (0-8 hour and 8-24 hour urine collection intervals). As with plasma, urine samples collected from six animals were pooled for bioanalysis.

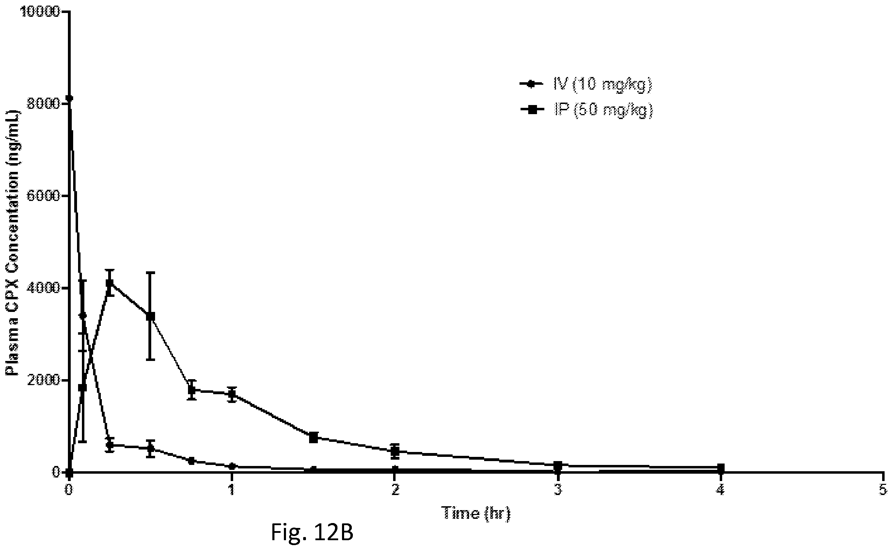

The 10 mg/kg IV and 50 mg/kg IP single doses of CPX-POM were well tolerated. The absolute bioavailability of IP CPX-POM was 65%. Consistent with the previous, pharmacokinetic proof of principle study, CPX-POM was not detectable at a BQL of 25 ng/mL in plasma following either treatment. As illustrated in Figure 12B, extensive systemic exposure to CPX is achieved following IV and IP administration of CPX-POM. Following 10 mg/kg IV bolus administration of CPX-POM, a peak plasma CPX concentration value of 8,098 ng/mL (39 µM) was observed, exceeding *in vitro* IC50 values by several fold. Following IP administration of 50 mg/kg CPX-POM, a Cmax value of 4,127 ng/mL (10.0 µM) was observed at 0.25 hours post-dose. Given the in vitro IC50 value of 2 µM (414 ng/mL) in muscle-noninvasive and muscle-invasive bladder cancer cell lines, 50 mg/kg IP CPX-POM achieves CPX Cmax values that are five-fold greater than the IC50.

Resultant plasma pharmacokinetic data are summarized in Table 2A. Consistent with previous study 208-02, the apparent elimination rate of CPX is rapid following IP administration, with a mean plasma terminal half-life of less than 1 hour (0.88 hours). The systemic clearance of CPX, following IV administration of CPX-POM in this study was 7,605 mL/hr/kg in the mouse. The volume of distribution for CPX following IV administration of CPX-POM was moderate in the mouse at approximately 9.0 L/kg, again, greater than the previous mouse study. Figure 12B shows the mean plasma ciclopirox (CPX) concentration-time rectilinear profiles following administration of 10 mg/kg IV and 50 mg/kg IP CPX-POM to three mice per time point per treatment group.

**Table 2A. Plasma CPX pharmacokinetic parameters in C57/B16 mice following single IV and IP doses of CPX-POM**

| **Treatment^{a}** | **IV** | **IP** |
|---|---|---|
| Mean Body Weight (kg) | 0.021 | 0.020 |
| CPX Dose (mg/kg)^{b} | 10 | 50 |
| C₀ (ng/mL)^{c} | 8098 | |
| Cmax (ng/mL) | | 4127 |
| Tmax (hr) | | 0.25 |
| Kₑₗ (hr⁻¹) | 0.544 | 0.785 |
| T_{½} (hr) | 1.27 | 0.88 |
| AUC₀∫⁶ (ng*hr/mL) | 1273 | 3981 |
| AUC₀∫^{∞} (ng*hr/mL) | 1328 | 4124 |
| AUMC₀∫⁶ (ng*hr²/mL) | 544 | |
| AUMC₀∫^{∞} (ng*hr²/mL) | 861 | |
| MRT (hr) | 0.649 | |
| Cl (mL/hr/kg) | 7605 | |
| V_{z} (mL/kg) | 13981 | |
| Vₛₛ (mL/kg) | 4887 | |
| F (%) | | 64.8 |

| | | |
|---|---|---|
| ^{a} N=3 mice per time point per treatment ^{b} Ciclopirox free acid dose administered, CPX-POM disodium salt was formulated in 25mM Phosphate Buffer pH7 at a strength of 2.69 mg/mL ^{c} C₀ corresponds to y-intercept value following IV bolus administration derived from non-parametric pharmacokinetic data analysis | | |

Urine CPX pharmacokinetic parameters obtained in mice following 10 mg/kg IV and 50 mg/kg IP CPX-POM are summarized in Table 3A below. Following single IV and IP doses of CPX-POM, less than 1% of the dose is excreted as CPX. However, urine CPX concentrations exceeded the in vitro IC50 value of 2 µM by several fold. In contrast to the IV route of administration, significant urinary concentrations of CPX were observed over the entire 24-hour period post-dose following IP administration. Twenty three to 26% of the CPX-POM dose was excreted as the inactive, glucuronide metabolite of ciclopirox.

**Table 3A. Urine CPX^{c} and CPX-G^{c} pharmacokinetic parameters in male C57/Bl6 mice following single doses of IV and IP CPX-POM**

| **Treatment^{a}** | **CPX-POM IV** | **CPX-POM IP** |
|---|---|---|
| Mean Body Weight (kg) | 0.021 | 0.022 |
| CPX Dose (mg/kg)^{b} | 10 | 50 |
| U_{CPX} (ng/mL) 0-4 hr | 1420 | 4740 |
| U_{CPX} (ng/mL) 4-8 hr | 3300 | 3690 |
| U_{CPX} (ng/mL) 8-24 hr | 0 | 7050 |
| U_{CPX} (µM) 0-4 hr | 6.86 | 22.9 |
| U_{CPX} (µM) 4-8 hr | 15.9 | 17.8 |
| U_{CPX} (µM) 8-24 hr | 0 | 34.1 |
| Xe_{CPX} (µg) 0-24 hr | 0.57 | 7.75 |
| Fe_{CPX} (%) | 0.27 | 0.60 |
| Xe/dt_{CPX} (ng/hr) 0-4 hr | 37.3 | 104 |
| Xe/dt_{CPX} (ng/hr) 4-8 hr | 58.0 | 80.9 |
| Xe/dt_{CPX} (ng/hr) 8-24 hr | 0 | 415 |
| Clᵣ (mL/hr) | 0.44 | |
| Clᵣ (mL/hr/kg) | 21.5 | |
| Fe_{CPX-G} (%) | 25.9 | 22.6 |

| | | |
|---|---|---|
| ^{a} N=6 mice per treatment ^{b} Ciclopirox free acid dose administered, CPX-POM disodium injectable formulation contained 5 mg/mL CPX in 25mM Phosphate pH7 with 50mM Captisol® ^{c} Ciclopirox (CPX); Ciclopirox glucuronide (CPX-G) | | |

### Study 11206. Ciclopirox-POM Prodrug Urine Pharmacokinetics following Single and Repeated IP Administration in the Mouse.

Urine ciclopirox (CPX) pharmacokinetics were characterized in mice following single (Day 1) and repeat (Day 10) dose (once daily for ten consecutive days) administration of 50, 100, and 200 mg/kg ciclopirox-POM prodrug (CPX-POM) to three separate groups of six mice each, are summarized in Table 3B below. This study was conducted to support in vivo preclinical proof of principle studies in validated models of bladder cancer by documenting ciclopirox exposure in the urine following administration of CPX-POM prodrug following a range of once daily IP doses. Following single and repeat dose IP administration of 50, 100, and 200 mg/kg CPX-POM, less than 1% of the dose was excreted as CPX over 24 hours. Despite the low percent dose recovery as ciclopirox, however, urine CPX concentrations exceeded the in vitro IC50 value of 2 µM by several-fold across the entire dose range. CPX-POM was not detected in any urine samples collected in this study suggesting complete metabolism of the prodrug. A significant portion of the administered dose was eliminated as the inactive ciclopirox glucuronide metabolite across the entire dose range. Based on these data, subchronic IP dosage regimens of 100 mg/kg and 200 mg/kg ciclopirox-POM prodrug were selected for subsequent proof of principle studies in validated models of bladder cancer.

**Table 3B. Urine CPX^{c} and CPX-G^{c} pharmacokinetic parameters in male C57/B16 mice following single and repeated once daily IP doses of CPX-POM**

| **Treatment^{a}** | **50 mg/kg IP** | | **100 mg/kg IP** | | **200 mg/kg IP** | |
|---|---|---|---|---|---|---|
| | Day 1 | Day 10 | Day 1 | Day 10 | Day 1 | Day 10 |
| Mean Body Weight (kg) | 0.021 | 0.023 | 0.0020 | 0.022 | 0.020 | 0.022 |
| CPX Dose (mg)^{b} | 1.1 | 1.1 | 2.1 | 2.2 | 4.0 | 4.4 |
| U_{CPX} (ng/mL) 0-8 hr | 5975 | 7855 | 5595 | 15000 | 8165 | 19350 |
| U_{CPX} (ng/mL) 8-24 hr | 2215 | 8220 | 4180 | 5555 | 5540 | 8740 |
| U_{CPX} (ng/mL) 0-24 hr | 2871 | 8142 | 4700 | 8010 | 6315 | 12149 |
| U_{CPX} (µM) 0-8 hr | 29 | 38 | 27 | 72 | 39 | 93 |
| U_{CPX} (µM) 8-24 hr | 11 | 50 | 20 | 27 | 27 | 42 |
| U_{CPX} (µM) 0-24 hr | 14 | 39 | 23 | 39 | 30 | 59 |
| Xe_{CPX} (µg) 0-24 hr | 2.72 | 4.12 | 6.44 | 3.03 | 2.91 | 2.51 |
| Fe_{CPX} (%) | 0.25 | 0.36 | 0.31 | 0.14 | 0.07 | 0.06 |
| Xe_{CPX-G} (mg) 0-24 hr | 7.46 | 5.24 | 18.69 | 22.73 | 28.42 | 15.57 |
| Fe_{CPX-G} (%)^{d} | 61.1 | 41.6 | 80.2 | 93.0 | 63.5 | 32.1 |

| | | | | | | |
|---|---|---|---|---|---|---|
| ^{a} N=6 mice per treatment group ^{b} Ciclopirox free acid dose administered, CPX-POM disodium injectable formulation contained 20 mg/mL CPX in 25mM Phosphate pH7 with 50mM Captisol® administered once daily IP for ten consecutive days ^{c} Ciclopirox (CPX); Ciclopirox glucuronide (CPX-G) ^{d} Fraction of dose excreted as CPX-G was corrected for molecular weight | | | | | | |

### Ciclopirox-POM Prodrug Pharmacokinetics in the Rat

**Study 10954. Bioavailability of Ciclopirox Following Intravenous Administration of Ciclopirox-POM Prodrug in the Rat.** The plasma and urine pharmacokinetics of ciclopirox (CPX) were characterized in the rat following IV administration of 10 mg/kg ciclopirox olamine (CPX-O) and ciclopirox-POM prodrug (CPX-POM). Intravenous ciclopirox olamine was used as the reference in characterizing the bioavailability of ciclopirox following IV ciclopirox-POM prodrug in this specie. Given the low oral bioavailability of CPX observed in the mouse following administration of CPX-O and CPX-POM, this route of administration was not investigated in this study.

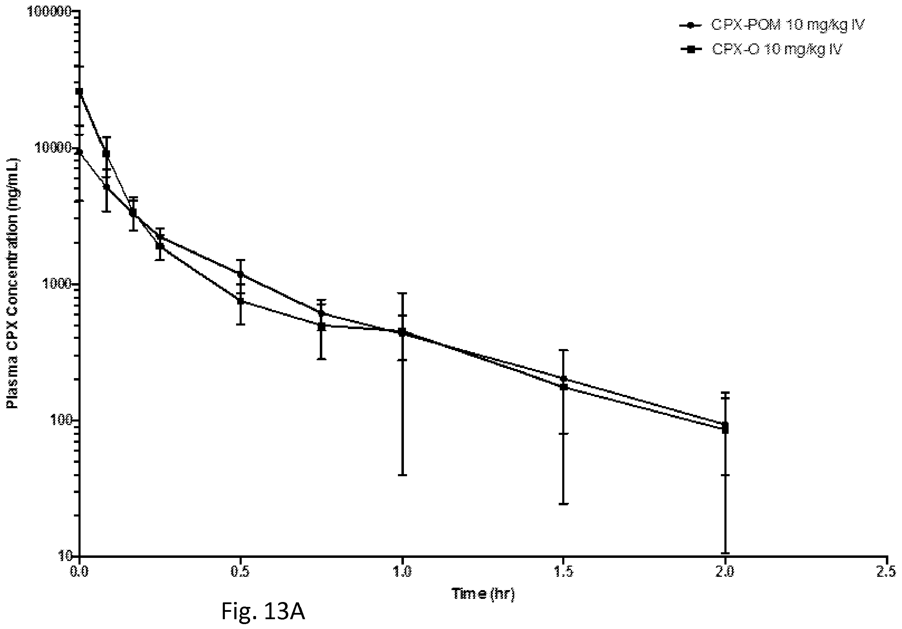

Resultant mean plasma ciclopirox concentration-time profiles following administration of IV CPX-POM and CPX-O are illustrated in Figure 13A. Plasma ciclopirox concentrations declined rapidly following IV administration of both treatments with an apparent elimination half-life of less than one hour. Resultant plasma ciclopirox pharmacokinetic parameters are summarized in Table 4A.

Following IV administration of CPX-POM, the systemic availability of CPX was 77% compared to intravenous administration of ciclopirox olamine. Plasma concentrations of the prodrug were observed in a few blood samples collected within minutes of IV bolus administration, however, we failed to detect CPX-POM in the majority of samples collected. CPX-POM was not detected in any urine samples collected. Extensive systemic exposure to CPX is achieved following IV administration of 10 mg/kg CPX-POM, with a mean initial plasma ciclopirox concentration value of 9,310 ng/mL immediately following IV bolus administration. Given the in vitro IC50 value of 2 µM (414 ng/mL) in muscle-noninvasive and muscle-invasive bladder cancer cell lines, 10 mg/kg IV CPX-POM achieves Cmax values of approximately twenty-fold greater than the IC50 (Cmax = 45 µM). Figure 13A shows the mean plasma ciclopirox (CPX) concentration-time semi-logarithmic profiles following administration of 10 mg/kg IV CPX-POM and CPX-O to six rats per treatment group.

**Table 4A. Plasma CPX pharmacokinetic parameters in male Sprague-Dawley rats following single IV doses of CPX-POM and CPX-O**

| **Treatment^{a}** | **CPX-POM** | **CPX-O** |
|---|---|---|
| Mean Body Weight (kg) | 0.26 ±0.01 | 0.27 ±0.0 1 |
| CPX Dose (mg/kg)^{b} | 9.12 ± 0.05 | 10.00 ± 0.06 |
| C₀ (ng/mL) | 9.310 ± 5,251 | 25,883 ± 13,321 |
| Kₑₗ (hr⁻¹) | 1.580 ± 0.286 | 1.453 ± 0.634 |
| T_{½} (hr) | 0.452 ± 0.087 | 0.615 ± 0.404 |
| AUC₀∫ⁿ (ng*hr/mL) | 2,121 ± 501 | 3,077 ± 427 |
| AUC₀∫^{∞} (ng*hr/mL) | 2,161 ± 477 | 3,141 ± 349 |
| AUMC₀∫ⁿ (ng*hr²/mL) | 827 ± 275 | 947 ± 608 |
| AUMC₀∫^{∞} (ng*hr²/mL) | 950 ± 229 | 1,291 ± 1,206 |
| MRT (hr) | 0.455 ± 477 | 0.446 ± 0.474 |
| Cl (mL/hr/kg) | 4,060 ± 399 | 3,225 ± 374 |
| Vd_{Z} (mL/kg) | 2,664 ± 670 | 2,983 ± 2,319 |
| Vₛₛ (mL/kg) | 1,853 ± 899 | 1,563 ± 1,853 |
| F (%) | 77.0 | |

| | | |
|---|---|---|
| ^{a} N=6 rats per treatment ^{b} Ciclopirox free acid dose administered, CPX-POM disodium injectable formulation contained 5 mg/mL CPX in 25mM Phosphate pH7 with 50mM Captisol® ^{c} C₀ corresponds to y-intercept value derived from non-parametric pharmacokinetic data analysis | | |

Urine CPX pharmacokinetic parameters obtained in rats following 10 mg/kg IV CPX-O and CPX-POM are summarized in Table 5A below. Following a single IV dose of CPX-POM and CPX-O, less than 1% of the dose is excreted as CPX. Urine CPX concentrations, however, exceeded the in vitro IC50 value of 2 µM.

**Table 5A. Urine CPX^{c} and CPX-G^{c} pharmacokinetic parameters in male Sprague-Dawley rats following single doses of IV CPX-POM and CPX-O**

| **Treatment** | **CPX-POM** | **CPX-O** |
|---|---|---|
| Mean Body Weight (kg)^{a} | 0.252 ± 0.006 | 0.238 ± 0.006 |
| CPX Dose (mg/kg)^{b} | 9.06 ±0.02 | 9.98 ±0.07 |
| U_{CPX} 0-8 hr (ng/mL) | 1,182 ± 498 | 921 ± 112 |
| U_{CPX} 8-24 hr (ng/mL) | 198 ± 172 | 118 ± 45 |
| U_{CPX} 0-8 hr (µM) | 4.45 ± 0.54 | 8.79 ± 2.41 |
| U_{CPX} 8-24 hr (µM) | 1.22 ± 0.32 | 2.64 ± 1.13 |
| dXe/dt_{CPX} 0-8hr (µg/hr) | 0.23 ± 0.09 | 0.76 ± 0.26 |
| dXe/dt_{CPX} 8-24hr (µg/hr) | 0.07 ± 0.01 | 0.13 ± 0.04 |
| Xe_{CPX} (µg) | 2.88 ± 0.78 | 8.18 ± 2.90 |
| Fe_{CPX} (%) | 0.13 ± 0.03 | 0.34 ± 0.12 |
| Clr (mL/hr) | 1.27 | 2.60 |
| Clr (mL/hr/kg) | 5.04 | 0.92 |
| Fe_{CPX-G} (%) | 27.75 ± 10.02 | 17.58 ± 2.46 |

| | | |
|---|---|---|
| ^{a} N=4 rats who received all three treatments in a complete, nonrandomized, crossover fashion ^{b} Ciclopirox free acid dose administered, CPX-POM disodium injectable formulation contained 5 mg/mL CPX in 25mM Phosphate pH7 with 50mM Captisol® ^{c} Ciclopirox (CPX); Ciclopirox glucuronide (CPX-G) | | |

**Study 11428. Bioavailability of Ciclopirox Following Subcutaneous Administration of Ciclopirox-POM Prodrug and Oral Ciclopirox Olamine in the Rat.** The plasma and urine pharmacokinetics of ciclopirox (CPX) were characterized in the rat following IV administration of 20 mg/kg ciclopirox-POM prodrug (CPX-POM), subcutaneous (SQ) administration of 30 mg/kg CPX-POM, and oral administration of 30 mg/kg ciclopirox olamine (CPX-O). Intravenous CPX-POM was used as the reference in characterizing the bioavailability of ciclopirox following SQ ciclopirox-POM prodrug in this specie. The oral bioavailability of CPX following administration of the olamine salt was determined to establish a pharmacokinetic "bridge" between injectable CPX-POM and preclinical safety data generated by Hoechst AG through registration studies conducted in support of the olamine topical antifungal product.

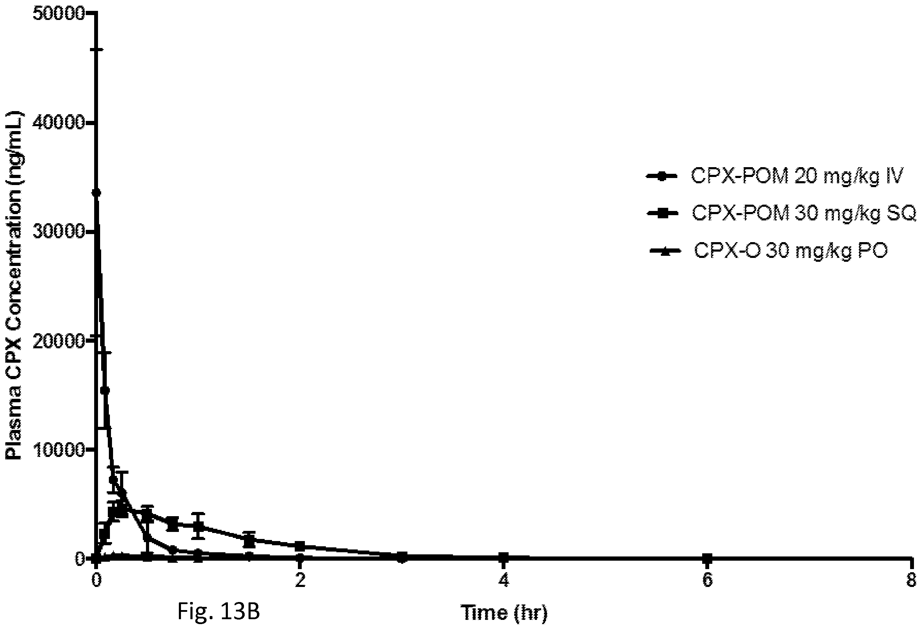

Resultant mean plasma ciclopirox concentration-time profiles following administration of IV and SQ CPX-POM as well as orally administered ciclopirox olamine are illustrated in Figure 13B. Consistent with Study 10954, plasma ciclopirox concentrations declined rapidly following IV administration of CPX-POM with an apparent elimination half-life of less than one hour. The plasma ciclopirox concentration-time profile resulting from SQ CPX-POM was drastically different than IV CPX-POM. Plasma ciclopirox concentrations following oral administration of the olamine salt of ciclopiro were comparatively quite low. Resultant plasma ciclopirox pharmacokinetic parameters are summarized in Table 6A.

SQ CPX-POM demonstrated excellent bioavailability compared to IV CPX-POM at 80%. IV and SQ administered CPX-POM resulted in plasma ciclopirox concentrations that exceeded the *in vitro* IC50 value of 2 µM by several-fold. Plasma ciclopirox concentrations of ciclopirox following oral administration of the olamine salt were quite low. The absolute bioavailability of this salt form administered orally was 4.6% compared to IV CPX-POM. CPX-POM was not detected in any blood or urine sample collected in this study. Figure 13B shows the mean plasma ciclopirox (CPX) concentration-time rectilinear profiles following administration of 20 mg/kg IV CPX-POM, 30 mg/kg SQ CPX-POM, and 30 mg/kg CPX-O to six rats per treatment group.

**Table 6A._Plasma CPX pharmacokinetic parameters in male Sprague-Dawley rats following single doses of IV CPX-POM, SQ CPX-POM, and oral CPX-O**

| | | | |
|---|---|---|---|
| **Treatment^{a}** | **CPX-POM IV** | **CPX-POM SQ** | **CPX-O Oral** |
| Mean Body Weight (kg) | 0.314 ± 0.012 | 0.315 ± 0.007 | 0.314 ± 0.013 |
| CPX Dose (mg/kg)^{b} | 19.98 ± 0.10 | 30.02 ± 0.06 | 30.17 ± 0.16 |
| C₀ (ng/mL) | 33,568 ± 13,124^{c} | | |
| Cmax (ng/mL) | | 5,005 ± 808 | 373 ± 158 |
| Tmax (hr) | | 0.264 ± 0.122 | 0.250 ± 0.129 |
| Kₑₗ (hr⁻¹) | 1.529 ± 0.680 | 1.039 ± 0.253 | 1.234 ± 0.742 |
| T_{½} (hr) | 0.537 ± 0.238 | 0.700 ± 0.163 | 0.801 ± 0.558 |
| AUC₀∫ⁿ (ng*hr/mL) | 5,449 ± 756 | 6,548 ± 1007 | 211.2 ± 84.5 |
| AUC₀∫^{∞} (ng*hr/mL) | 5,496 ± 756 | 6,633 ± 1,019 | 378.0 ± 140.4 |
| AUMC₀∫ⁿ (ng*hr²/mL) | 1,534 ± 457 | | |
| AUMC₀∫^{∞} (ng*hr²/mL) | 1,687 ± 478 | | |
| MRT (hr) | 0.285 ± 0.092 | | |
| Cl (mL/hr/kg) | 3,695 ± 531 | | |
| Vd_{z} (mL/kg) | 2,874 ± 1451 | | |
| Vₛₛ (mL/kg) | 1,167 ± 467 | | |
| F (%) | | 80.3 | 4.56 |

| | | | |
|---|---|---|---|
| ^{a} N=6 rats per treatment ^{b} Ciclopirox free acid dose administered, CPX-POM disodium injectable formulation contained 5 mg/mL CPX in 25mM Phosphate pH7 with 50mM Captisol® ^{c} C₀ corresponds to y-intercept value derived from non-parametric pharmacokinetic data analysis | | | |

Urine CPX pharmacokinetic parameters obtained in rats following 20 mg/kg IV CPX-POM, 30 mg/kg SQ CPX-POM, and 30 mg/kg oral CPX-O are summarized in Table 7A below. IV and SQ CPX-POM administration resulted in greater urine concentrations of ciclopirox compared to orally administered CPX-O. IV and SQ CPX-POM doses were well tolerated in these animals, and resulted in urine ciclopirox concentrations that exceeded the *in vitro* IC50 value by several-fold. SQ CPX-POM results in significant systemic and urinary tract exposure of ciclopirox.

**Table 7A. Urine CPX^{c} and CPX-G^{c} pharmacokinetic parameters in male Sprague-Dawley rats following single doses of IV CPX-POM, SQ CPX-POM, and oral CPX-O**

| **Treatment^{a}** | **CPX-POM IV** | **POX-POM SQ** | **CPX-O Oral** |
|---|---|---|---|
| Mean Body Weight (kg) | 0.276 ± 0.008 | 0.293 ± 0.007 | 0.289 ± 0.010 |
| CPX Dose (mg/kg)^{b} | 20.02 ± 0.07 | 29.97 ± 0.08 | 30.44 ± 0.18 |
| U_{CPX} (ng/mL) 0-24 hr | 20,623 ± 11,948 | 5,931 ± 5689 | 1,711 ± 1,427 |
| U_{CPX} (µM) 0-24 hr | 99.63 ± 57.72 | 28.65 ± 27.48 | 8.28 ± 6.89 |
| Xe_{CPX}(mcg) 0-24 hr | 190.5 ± 103.0 | 85.44 ± 65.89 | 22.30 ± 16.65 |
| Fe_{CPX} (%) | 3.46 ± 1.90 | 0.98 ± 0.76 | 0.25 ± 0.18 |
| Xe/dt_{CPX} (µg/hr) 0-8 hr | 18.36 ± 11.6 | 6.22 ± 6.50 | 0.63 ± 0.16 |
| Xe/dt_{CPX} (µg/hr) 8-24 hr | 2.76 ± 1.79 | 2.22 ± 2.76 | 1.08 ± 1.11 |
| Clᵣ (mL/hr) | 34.6 | | |
| Clᵣ (mL/hr/kg) | 126 | | |
| Fe_{CPX-G} (%) | 9.37 ± 9.08 | 13.73 ± 8.83 | 9.13 ± 4.28 |

| | | | |
|---|---|---|---|
| ^{a} N=4 rats who received all three treatments in a complete, nonrandomized, crossover fashion ^{b} Ciclopirox free acid dose administered, CPX-POM disodium injectable formulation contained 5 mg/mL CPX in 25mM Phosphate pH7 with 50mM Captisol® ^{c} Ciclopirox (CPX); Ciclopirox glucuronide (CPX-G) | | | |

### Ciclopirox-POM Prodrug Pharmacokinetics in the Dog

**Study 10953. Bioavailability of Ciclopirox Following Intravenous Administration of Ciclopirox-POM Prodrug in the Dog.** The plasma and urine pharmacokinetics of ciclopirox (CPX) were characterized in the rat following IV administration of 10 mg/kg ciclopirox olamine (CPX-O) and ciclopirox-POM prodrug (CPX-POM). Intravenous ciclopirox olamine was used as the reference in characterizing the bioavailability of ciclopirox following IV ciclopirox-POM prodrug in this specie. Given the low oral bioavailability of CPX observed in the mouse following administration of CPX-O and CPX-POM, this route of administration was not investigated in this study.

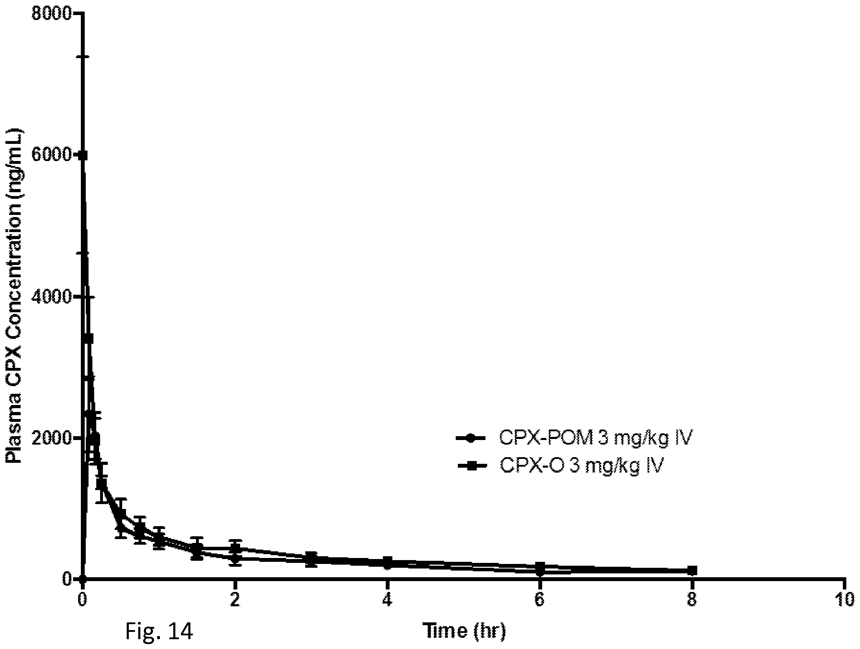

Figure 14 illustrates the mean plasma CPX concentration-time profiles following administration of 3 mg/kg IV CPX-POM and CPX-O to four male beagle dogs.

Following IV administration of CPX-POM, the systemic availability of CPX was 85%. Plasma CPX-POM concentrations were detected in very few blood samples collected within minutes following IV bolous administration, however, the prodrug was not detected in the majority of plasma samples assayed. CPX-POM was not detected in any urine sample. The absolute bioavailability of the active agent ciclopirox following IV CPX-POM, was excellent, with a value 85% with IV CPX-O as the reference. The 3 mg/kg IV dose of the prodrug achieved a mean initial plasma ciclopirox concentration of 2,907 ng/mL, which is equivalent to 14 µM. Given the in vitro IC50 value of 2 µM (414 ng/mL) in muscle-noninvasive and muscle-invasive bladder cancer cell lines, the observed mean Cmax value is approximately seven-fold greater than the IC50. Figure 14 shows the mean plasma ciclopirox (CPX) concentration-time rectilinear profiles following administration of 3 mg/kg IV CPX-POM and CPX-O to four male beagle dogs.

**Table 8A. Plasma CPX pharmacokinetic parameters in male beagle dogs following single IV doses of CPX-POM and CPX-O**

| **Treatment** | **CPX-POM** | **CPX-O** |
|---|---|---|
| Mean Body Weight (kg)^{a} | 9.65 ± 0.38 | 9.64 ± 0.34 |
| IV CPX Dose (mg/kg)^{b} | 2.82 ± 0.01 | 3.10 ± 0.02 |
| C₀ (ng/mL)^{c} | 2,970 ± 969 | 5,996 ± 1,387 |
| Kₑₗ (hr⁻¹) | 0.242 ± 0.030 | 0.185 ± 0.019 |
| T_{½} (hr) | 2.90 ± 0.329 | 3.77 ± 0.40 |
| AUC₀∫ⁿ (ng*hr/mL) | 2,571 ± 436 | 3,314 ± 655 |
| AUC₀∫^{∞} (ng*hr/mL) | 3,075 ± 591 | 4,008 ± 802 |
| AUMC₀∫ⁿ (ng*hr²/mL) | 5,485 ± 1,293 | 7,293 ± 1,520 |
| AUMC₀∫^{∞} (ng*hr²/mL) | 11,680 ± 3,682 | 16,665 ± 3,972 |
| MRT (hr) | 3.74 ± 0.58 | 2.20 ± 0.09 |
| Cl (mL/hr/kg) | 1,006 ± 209 | 777.2 ± 192.5 |
| Vd_{z} (mL/kg) | 4,133 ± 363 | 4,253 ± 1,266 |
| Vₛₛ (mL/kg) | 3,686 ± 431 | 3,218 ± 810 |
| F (%) | 85.1 ± 9.69 | |

| | | |
|---|---|---|
| ^{a} N=4 dogs receiving both treatments in a complete, crossover study design, animals were weighed prior to each treatment to determine dose volume for administration, hence, the slight differences in body weights for each treatment ^{b} Ciclopirox free acid dose administered, CPX-POM disodium injectable formulation contained 5 mg/mL CPX in 25mM Phosphate pH7 with 50mM Captisol® ^{c} C₀ corresponds to y-intercept value derived from non-parametric pharmacokinetic data analysis | | |

Urine CPX pharmacokinetic parameters obtained in dogs following 3 mg/kg IV CPX-O and CPX-POM are summarized in Table 9A below. Following a single IV dose of CPX-POM and CPX-O, less than 1% of the dose is excreted as CPX. However, urine CPX concentrations, however, exceeded the in vitro IC50 value of 2 µM at 3 mg/kg IV doses of both CPX-POM and CPX-O. The inactive ciclopirox glucuronide metabolite represented the major specie excreted in urine.

**Table 9A. Urine CPX pharmacokinetic parameters in male beagle dogs following single IV doses of CPX-POM and CPX-O**

| **Treatment** | **CPX-POM** | **CPX-O** |
|---|---|---|
| Mean Body Weight (kg)^{a} | 9.65 ± 0.38 | 9.64 ± 0.34 |
| CPX Dose (mg/kg) ^{b} | 2.82 ± 0.01 | 3.10 ± 0.02 |
| U_{CPX} 0-8 hr (ng/mL) | 838 ± 149 | 1,006 ± 370 |
| U_{CPX} 8-24 hr (ng/mL) | 112.0 ± 56.1 | 72.5 ± 54.5 |
| U_{CPX} 0-8 hr (µM) | 4.04 ± 0.72 | 4.86 ± 1.79 |
| U_{CPX} 8-24 hr (µM) | 0.541 ± 0.271 | 0.350 ± 0.263 |
| dXe/dt_{CPX} 0-8hr (µg/hr) | 9.88 ± 0.3.89 | 12.58 ± 5.61 |
| dXe/dt_{CPX} 8-24hr (µg/hr) | 0.97 ± 0.19 | 0.43 ± 0.42 |
| Xe_{CPX}(µg) | 94.5 ± 32.6 | 107.5 ± 39.9 |
| Fe_{CPX} (%) | 0.345 ± 0.111 | 0.358 ± 0.123 |
| Clr (mL/hr) | 31.90 ± 18.25 | 26.43 ± 9.63 |
| Clr (mL/hr/kg) | 3.284 ± 1.810 | 2.730 ± 0.916 |
| Fe_{CPX-G} (%) | 51.11 ± 5.31 | 62.67 ± 12.15 |

| | | |
|---|---|---|
| ^{a} N=4 dogs receiving both treatments in a complete, crossover study design, animals were weighed prior to each treatment to determine dose volume for administration, hence, the slight differences in body weights for each treatment ^{b} Ciclopirox free acid dose administered, CPX-POM disodium injectable formulation contained 5 mg/mL CPX in 25mM Phosphate pH7 with 50mM Captisol® | | |

**Study 11429. Bioavailability of Ciclopirox Following Subcutaneous Administration of Ciclopirox-POM Prodrug and Oral Ciclopirox Olamine in the Dog.** The plasma and urine pharmacokinetics of ciclopirox (CPX) were characterized in the dog following IV administration of 3 mg/kg ciclopirox-POM prodrug (CPX-POM), subcutaneous (SQ) administration of 9 mg/kg CPX-POM, and oral administration of 9 mg/kg ciclopirox olamine (CPX-O). Intravenous CPX-POM was used as the reference in characterizing the bioavailability of ciclopirox following SQ ciclopirox-POM prodrug in this specie. The oral bioavailability of CPX following administration of the olamine salt was determined to establish a pharmacokinetic "bridge" between injectable CPX-POM and preclinical safety data generated by Hoechst AG through registration studies conducted in support of the olamine topical antifungal product.

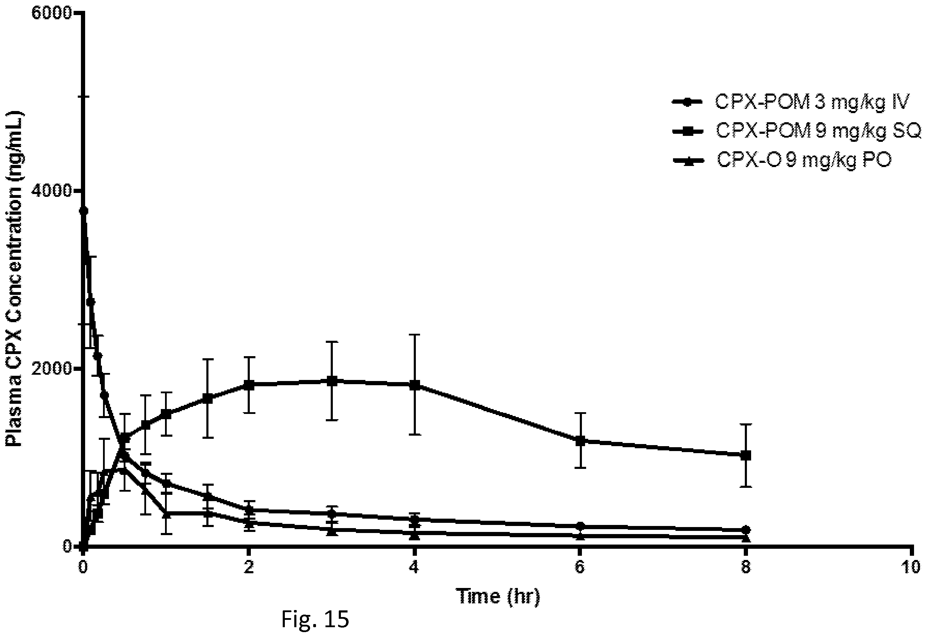

Resultant mean plasma ciclopirox concentration-time profiles following administration of IV and SQ CPX-POM as well as orally administered ciclopirox olamine to dogs are illustrated in Figure 15. Consistent with Study 10953, plasma ciclopirox concentrations declined rapidly following IV administration of CPX-POM with an apparent elimination half-life of less than one hour. The plasma ciclopirox concentration-time profile resulting from SQ CPX-POM was drastically different than IV CPX-POM. Plasma ciclopirox concentrations following oral administration of the olamine salt of ciclopiro were comparatively quite low. Resultant plasma ciclopirox pharmacokinetic parameters are summarized in Table 10A.

SQ CPX-POM demonstrated excellent bioavailability compared to IV CPX-POM at 106%. IV and SQ administered CPX-POM resulted in plasma ciclopirox concentrations that exceeded the *in vitro* IC50 value of 2 µM by several-fold. Plasma ciclopirox concentrations of ciclopirox following oral administration of the olamine salt were quite low. The absolute bioavailability of this salt form administered orally was 17.4% compared to IV CPX-POM. CPX-POM was not detected in any blood or urine sample collected in this study. Figure 15 shows the mean plasma ciclopirox (CPX) concentration-time rectilinear profiles following administration of 3 mg/kg IV CPX-POM, 9 mg/kg SQ CPX-POM, and 9 mg/kg Oral CPX-O to four male beagle dogs.

**Table 10A. Plasma CPX pharmacokinetic parameters in male beagle following single doses of IV CPX-POM, SQ CPX-POM, and oral CPX-O**

| **Treatment^{a}** | **CPX-POM IV** | **CPX-POM SQ** | **CPX-O Oral** |
|---|---|---|---|
| Mean Body Weight (kg) | 9.58 ± 0.49 | 9.91 ± 0.81 | 9.48 ± 0.64 |
| CPX Dose (mg/kg)^{b} | 3.10 ± 0.02 | 9.28 ± 0.09 | 9.39 ± 0.04 |
| C₀ (ng/mL) | 3,770 ± 1280^{c} | | |
| Cmax (ng/mL) | | 1,988 ± 374 | 1,017 ± 271 |
| Tmax (hr) | | 0.264 ± 0.122 | 0.250 ± 0.129 |
| Kₑₗ (hr⁻¹) | 0.153 ± 0.072 | 0.137 ± 0.031 | 0.126 ± 0.009 |
| T_{½} (hr) | 5.54 ± 2.92 | 5.27 ± 1.18 | 5.52 ± 0.39 |
| AUC₀∫ⁿ (ng*hr/mL) | 3,622 ± 255 | 11,518 ± 2,632 | 1,901 ± 650 |
| AUC₀∫^{∞} (ng*hr/mL) | 5,262 ± 1243 | 19,614 ± 6161 | 2,697 ± 1013 |
| AUMC₀∫ⁿ (ng*hr²/mL) | 8,836 ± 1,186 | | |
| AUMC₀∫^{∞} (ng*hr²/mL) | 38,965 ± 29,492 | | |
| MRT (hr) | 6.76 ± 3.76 | | |
| Cl (mL/hr/kg) | 615 ± 145 | | |
| Vd_{z} (mL/kg) | 4,486 ± 1366 | | |
| Vₛₛ (mL/kg) | 3,776 ± 1,266 | | |
| F (%) | | 106 ± 22.3^{d} | 17.4 ± 5.65^{d} |

| | | | |
|---|---|---|---|
| ^{a} N=4 dogs who received all three treatments in a nonrandomized, complete crossover fashion ^{b} Ciclopirox free acid dose administered, CPX-POM disodium injectable formulation contained 5 mg/mL CPX in 25mM Phosphate pH7 with 50mM Captisol® ^{c} C₀ corresponds to y-intercept value derived from non-parametric pharmacokinetic data analysis ^{d} Absolute bioavailability determined using AUC₀∫ⁿ given 28%, 39%, and 29% of AUC₀∫^{∞} was comprised of extrapolated AUC | | | |

Urine CPX pharmacokinetic parameters obtained in dogs following 3 mg/kg IV CPX-POM, 9 mg/kg SQ CPX-POM, and 9 mg/kg oral CPX-O are summarized in Table 11A below. IV and SQ CPX-POM administration resulted in comparatively greater urine concentrations of ciclopirox (dose adjusted) compared to orally administered CPX-O. IV and SQ CPX-POM doses were well tolerated in these animals, and resulted in urine ciclopirox concentrations that exceeded the *in vitro* IC50 value by several-fold. SQ CPX-POM results in significant systemic and urinary tract exposure of ciclopirox.

**Table 11A. Urine CPX^{d} and CPX-G^{d} pharmacokinetic parameters in male beagle dogs following single doses of IV CPX-POM, SQ CPX-POM, and oral CPX-O**

| **Treatment^{a}** | CPX-POM **IV** | **CPX-POM SQ^{C}** | **CPX-O Oral** |
|---|---|---|---|
| Mean Body Weight (kg) | 9.58 ± 0.49 | 9.91 ± 0.81 | 9.48 ± 0.64 |
| CPX Dose (mg/kg)^{b} | 3.10 ± 0.02 | 9.28 ± 0.09 | 9.39 ± 0.04 |
| U_{CPX} (ng/mL) 0-24 hr | 957 ± 508 | 2,530 ± 958 | 1,602 ± 750 |
| U_{CPX} (µM) 0-24 hr | 4.62 ± 2.45 | 12.2 ± 4.6 | 7.74 ± 3.62 |
| Xe_{CPX} (µg) 0-24 hr | 208 ± 117 | 339 ± 206 | 299 ± 208 |
| Fe_{CPX} (%) | 0.70 ± 0.38 | 0.38 ± 0.24 | 0.32 ± 0.21 |
| Xe/dt_{CPX} (µg/hr) 0-8 hr | 19.8 ± 15.2 | 23.0 ± 16.6 | 31.6 ± 26.2 |
| Xe/dt_{CPX} (µg/hr) 8-24 hr | 3.10 ± 3.33 | 9.67 ± 9.49 | 2.90 ± 1.70 |
| Clᵣ (mL/hr) | 43.2 ± 33.6 | | |
| Clᵣ (mL/hr/kg) | 4.51 + 3.51 | | |
| Fe_{CPX-G} (%) | 49.2 ± 34.4 | 43.0 ± 19.7 | 37.0 ± 18.7 |

| | | | |
|---|---|---|---|
| ^{a} N=4 dogs who received all three treatments in a nonrandomized, complete crossover fashion ^{b} Ciclopirox free acid dose administered, CPX-POM disodium injectable formulation contained 5 mg/mL CPX in 25mM Phosphate pH7 with 50mM Captisol® ^{c} N=3 ^{d} Ciclopirox (CPX); Ciclopirox glucuronide (CPX-G) | | | |

### In Vitro Stability of Ciclopirox Glucuronide in Bladder Cancer Patient Urine

Ciclopirox glucuronide (CPX-G) is the major urinary metabolite observed in mice, rats, dogs and humans following administration of CPX-POM as well as the olamine salt of cicloporix. CPX-G is not active as an anti-bladder cancer agent. CPX-G is metabolized by β-glucuronidase to form active ciclopirox, in effect, reactivating the active anti-bladder cancer agent. Based on human excretion data [Kellner et al, Arzneimittel-Forschung. 31 (8A):1337-1353], as well as evidence that bladder cancer patients have increased glucuronidase activity in urine [Paigen, K., Peterson, J., Paigen B. (1984) Role of urinary β-glucuronidase activity in human bladder cancer. Cancer Res 44:3620-3623], we conducted in vitro metabolism studies to characterize the stability of ciclopirox glucuronide in urine (at a concentration of 150 µM) obtained from bladder cancer patients compared to control urine collected from healthy volunteers.

Over a six-hour incubation of CPX-G in pooled patient urine obtained from six NMIBC patients, hydrolysis of 150 µM CPX-G in bladder cancer patient urine resulted in formation of 7.9 µM ciclopirox, a formation rate of 5%. The extent of ciclopirox formation was approximately 11.6-fold greater in bladder cancer patient urine compared to urine incubations with specimens obtained from healthy volunteers. The rate of β-glucuronidase-mediated hydrolysis of ciclopirox glucuronide ranged from 5.6 - 11.5-fold greater in pooled bladder cancer patient urine than in control urine. Urine β-glucuronidase activities were 69.5 U/mL and 1.39 U/mL in urine obtained from NMIBC patients and healthy volunteers, respectively (118-fold greater enzyme activity in bladder cancer patient urine). The selective hydrolysis of CPX-G in bladder cancer patient urine results in reactivation of the active anti-cancer agent ciclopirox.

### Prediction of Human Urine Concentrations of Ciclopirox Following IV Administration of Ciclopirox-POM Prodrug

Ciclopirox olamine (CPX-O), administered orally to humans, is rapidly and completely eliminated in the urine, with 87% of the dose excreted within 6 hours, and approximately 95% of the dose excreted within 12 hours of drug administration [Kellner et al, Arzneimittel-Forschung. 31 (8A):1337-1353]. Of the 95% of the dose recovered in urine, 83% was identified as ciclopirox (4%) and ciclopirox glucuronide (79%). CPX glucuronide does not possess activity in vitro.

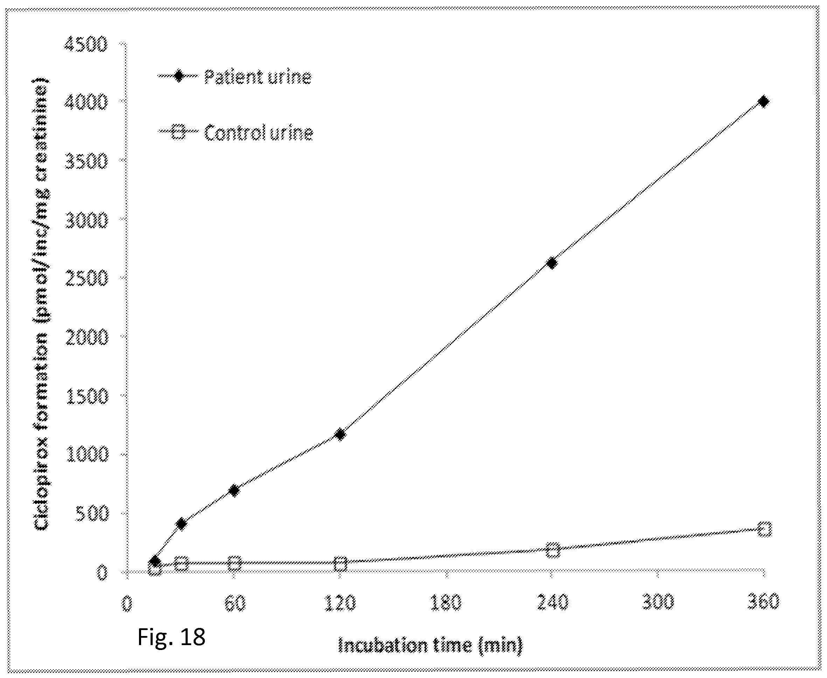

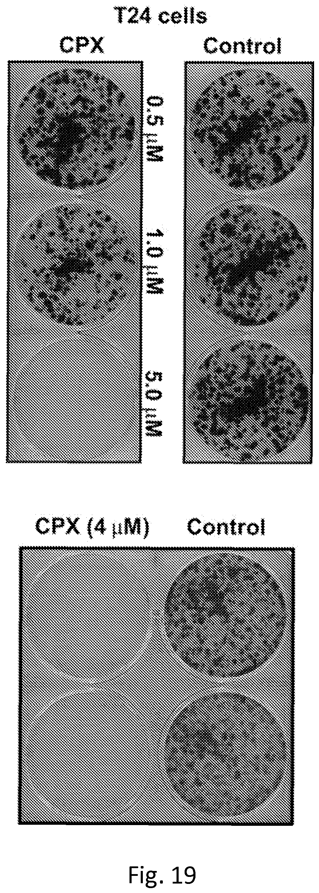

We simulated urine ciclopirox (CPX) concentration-time data based on the following assumptions: An intravenous dose of 32 mg/m² ciclopirox-POM prodrug (CPX-POM) will be well tolerated (note that 80 mg/m² of oral CPX-O was well tolerated when administered once daily); Four percent of an intravenous dose of 32 mg/m² CPX-POM will be excreted as ciclopirox, similar to the percent CPX excreted in urine following administration of a single oral dose of 10 mg C¹⁴ CPX-O; Five percent of CPX-G will be hydrolyzed to CPX in urine of bladder cancer patients; and urine production rates typical of newly diagnosed bladder cancer patients will be 1 mL/hr/kg. Figure 18 shows the formation of ciclopirox from the prodrug in patent urine and control urine. See also Figures 19-20.

### Systemic Administration of Ciclopirox-POM Prodrug Selectively Delivers CPX to the Entire Urinary Tract

Based on these assumptions, we predict that IV and SQ administration of ciclopirox-POM prodrug (CPX-POM) will result in urine CPX concentrations that exceed, several-fold, in vitro IC50 values in non-muscle invasive bladder cancer cell lines.

Injectable routes of administration for CPX-POM are preferred over oral administration to avoid gastrointestinal toxicity as well as poor oral bioavailability. It has been found that oral ciclopirox olamine (CPX-O) administered four times daily results in dose-limiting gastrointestinal toxicity (Minden et al, Am J Hematol 89(4):363-368, 2014).

*In vitro* studies have been conducted to understand the role of ciclopirox as a valid and novel therapeutic agent for the treatment of NMIBC and MIBC. Fully characterized human NMIBC and MIBC cell lines 253JBV and T24, respectively, were studied. Because cell culture media lack enzymes (phosphatases) necessary to metabolize CPX-POM to ciclopirox (CPX), mechanism of action studies were conducted by exposing bladder cancer cell lines to CPX by adding CPX-O to cell culture media.

To determine the effect of CPX on bladder cancer cell proliferation in T24 (MIBC) and 253-JBV (NMIBC) cells, 5,000 cells per well were seeded on to 96-well plates and grown overnight. The cells were then treated with increasing doses of CPX in RPMI media containing 10% FBS. Analysis of cell proliferation after the treatment period was estimated by the hexosaminidase assay. Briefly, the medium was removed and hexosaminidase substrate solution in citrate buffer pH 5 (7.5 mM), p-nitrophenol-N-acetyl-beta-D-glucosaminidase (Sigma Aldrich) was added at 75 *µ*L per well. The plate was incubated at 37°C in 100% humidity for 30 minutes. The reaction was stopped with 112.5 *µ*L of 50 mM glycine containing 5 mM of EDTA (pH 10.4). The absorbance was measured at 405 nm. Experiments were conducted at *n* = 6, and then repeated at least three times. The data were analyzed as percent of control, where the control wells were treated with equivalent amounts of DMSO alone. For ICso calculations, a plot between the drug concentration and hexosaminidase activity was generated and the data was fitted either linearly or exponentially. The best fit was used for further processing of data. IC₅₀ was obtained by determining the concentration of compounds resulting in 50% of cell death after 48 h of treatment.

### Ciclopirox Demonstrates Anticancer Activity in NMIBC and MIBC:

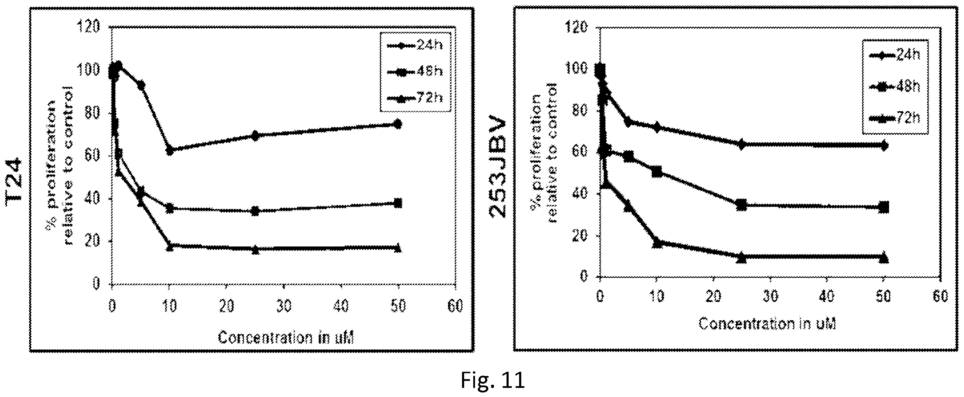

It was found that ciclopirox (CPX) inhibits bladder cancer cell proliferation. Figures 9, 10, and 11 show CPX inhibits proliferation of bladder cancer cell lines. Cells were incubated with the indicated dose of compound for up to 72 h. CPX treatment resulted in a significant dose- and time-dependent decrease in cell proliferation in both T24 and 253JBV cells. Results from this experiment, as illustrated in Figures 9 and 11, demonstrate that CPX significantly suppressed the proliferation of both cell lines in a dose and time dependent manner. This effect was observed within 24 h, which continued to remain for over the next 48 h. IC50 values for the compound was determined to be 2 and 4 µM at 48 h for 253JBV and T24 cells, respectively.

### Ciclopirox inhibits bladder cancer growth in 2 Dimensional and 3 Dimensional Model Systems:

We next determined the long-term effect of ciclopirox (CPX) on bladder cancer cells using the clonogenicity assay. For this assay, 6-well plates were seeded with 500 viable cells and were allowed to grow for overnight. The cells were then incubated in the presence or absence of various concentrations (1 and 5 µM) of CPX for 48 h. The CPX containing medium was then removed, and the cells were washed in PBS and incubated for an additional 10 days in complete medium. Each treatment was done in triplicate. The colonies obtained were washed with PBS and fixed in 10% formalin for 10min at room temperature and then washed with PBS followed by staining with crystal violet. The colonies were compared to untreated cells.

As illustrated in Figures 19-20, CPX inhibited the number and size of colonies compared to control, suggesting that short-term incubation of cells with the compound had long-term inhibitory effects on the viability of the cells. Figures 19-20 shows that CPX inhibits bladder cancer cell growth. T24 and 253-JBV bladder cancer cells were plated and treated with IC50 concentrations of CPX (dose indicated) for 48h. The fresh medium was replaced and allowed to grow for 10 days. The colonies were stained with crystal violet and compared to untreated cells.

### Ciclopirox inhibits bladder cancer spheroids:

Recent studies have demonstrated heterogeneity within a tumor, and that a small population of stem cells occur within the tumor with the capacity to self-renew and have the ability to generate differentiated descendants. The spheroid assay, which is sometimes also called the 3D assay was developed as a method to propagate epithelial stem cells *in vitro,* and is now a standard surrogate reporter for stem cell activity. The assay (in this case called bladdosphere assay) is based on the hypothesis that the unique, undifferentiated cells present in bladder cancers will survive in suspension culture, while the other cells will die by anoikis. Therefore, we characterized the effect of ciclopirox (CPX) in bladdosphere assay. Here, cancer cells are plated in ultra-low binding plates and the resulting spheroids are believed to represent growth from stem cells.

For formation of spheroids, cells were cultured in RPMI 1640 (Mediatech) supplemented with 20ng/mL bFGF (Invitrogen) 10 mL per 500 mL of 50X B27 supplement (Invitrogen) EGF 20 ng/mL (Invitrogen) and antibiotic and antimycotic solution. Cells were seeded at low densities (5000 cells/mL) in 6-well low adhesion plates. The cells were treated with increasing concentrations of CPX (0.5x, 1x, and 2x of IC₅₀ calculated from cell proliferation assay- 2 µM for 253JBV and 4 µM for T24). After 7 days the spheroids were photographed (I generation). Control spheroids and spheroids treated with CPX (IX IC50) were collected, trypsinized and re-plated for the secondary spheroid growth (II generation), followed by tertiary spheroid development (III generation).

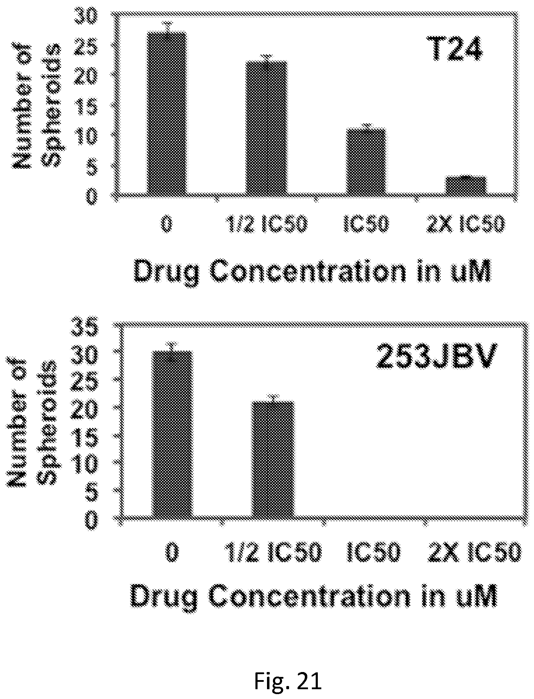

Figure 21 shows that CPX inhibits bladder cancer spheroids. Cells were seeded in an ultra-low attachment plates and treated with CPX and incubated with the indicated dose of compound. CPX treatment resulted in a significant decrease in size and number of spheroids formed by both T24 and 253-JBV bladder cancer cells (I generation). CPX treatment significantly reduced the growth of secondary and tertiary spheroids as well. Results from this experiment, shown in Figure 21, demonstrate that there was significant reduction in spheroid size and the number of T24 and 253JBV bladder cancer cell spheroids, when cells were treated with CPX. The secondary and tertiary spheroid developments were significantly inhibited by CPX treatment. These data suggest that the compound affects cancer stem cells within the bladder cancer. See also Figure 10.

### Ciclopirox arrests cells at S-phase of cell cycle:

Flow cytometry-based cell cycle analysis studies were conducted to determine the effect of ciclopirox (CPX) on cell cycle progression in T24 cells (data not shown). Studies demonstrated that CPX induces S-phase cell cycle arrest (data not shown). At 1X IC50 and 2X IC50 concentrations, CPX arrest cells at G0/G1 phase. Cells were treated with CPX and a flow cytometer based analysis was performed. CPX treatment significantly increased the percentage of cells getting arrested at S-phase and G0/G1-phase.

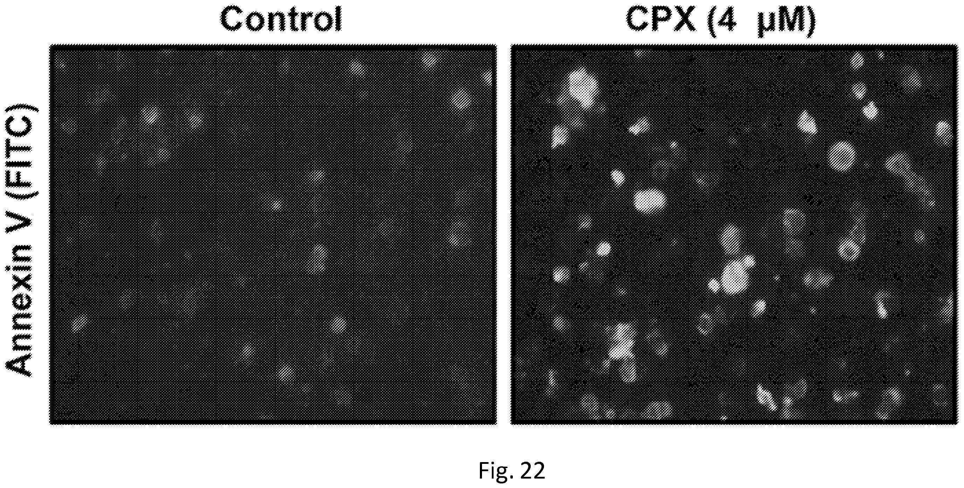

It was determined that CPX induces apoptosis. We next determined whether the compounds could induce cell death through the apoptotic pathway. For this we treated T24 bladder cancer cells with CPX (4 µM) and labeled the cells with FITC conjugated Annexin V to detect apoptotic cells. The results are illustrated in Figure 22 suggesting that CPX treatment had high levels of FITC+ cells implying that CPX induced cell death. Figure 22 shows that CPX induces cell death in bladder cancer cell lines. Cells were incubated with the indicated dose of compound for up to 48 h. CPX treatment resulted in a significant increase in cell death in T24 cells as evident from the increase in Annexin V -FITC labeled cells.

### Ciclopirox affects the cancer stem cell signaling pathways:

We performed an RT-PCR array analyses as a preliminary study to identify the pathways affected by ciclopirox (CPX). Expression analysis was performed using RT2 Profiler™ PCR Arrays (SA Biosciences, Frederick, MD, USA) per the manufacturer's instructions. The Human signal transduction pathway finder polymerase chain reaction (PCR) array was used (cat. no. PAHS-014ZA). Briefly, Total RNA isolated from T24 cells using TRIZOL reagent was reverse transcribed with Superscript II reverse transcriptase in the presence of random hexanucleotide primers (all from Invitrogen, Carlsbad, CA). Complementary DNA (2 µg) was mixed with 1,275 µL RT2 SYBR Green qPCR Master Mix (PA-010; SA Biosciences) to make up a total volume of 2,550 µL. Each well received 25 µL of this mix. qPCR was performed on an Bio-Rad CFX machine following detection protocols recommended by SA Biosciences. Changes in mRNA expression were expressed as fold change relative to control.

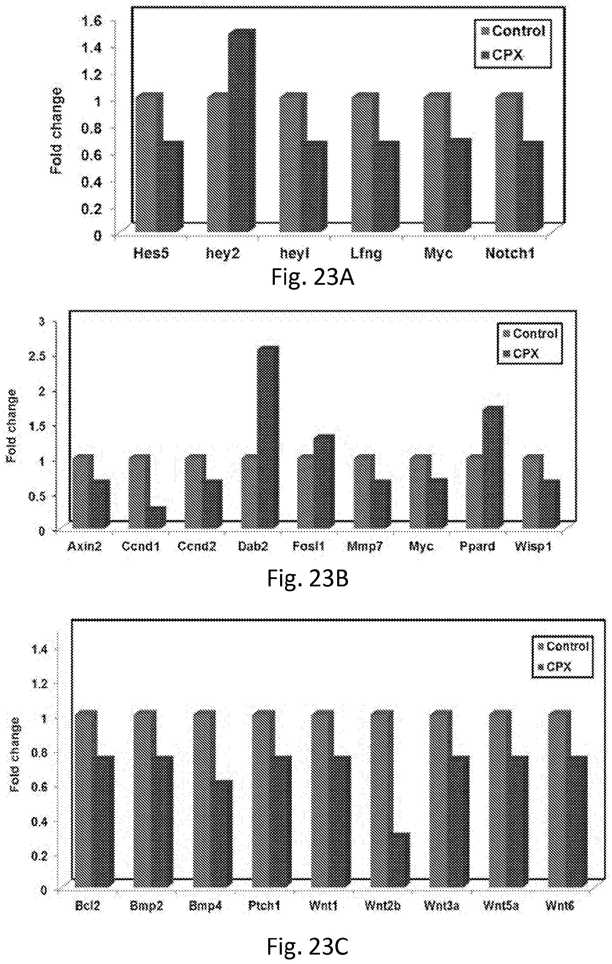

Figures 23A-23C show CPX inhibits CSC signaling pathways. CPX treatment significantly reduced the mRNA expression of Notch and its downstream signaling proteins. In addition, CPX inhibited expression of genes involved in Wnt and Hedgehog signaling pathways in T24 cells. CPX treatment significantly reduced the expression of genes (mRNA level) involved in CSC signaling pathways such as Notch, Wnt and Hedgehog as shown in Figure 23A-23C.

### Ciclopirox affects the Notch signaling pathway:

We performed an RT-PCR array analyses to identify the pathways affected by ciclopirox (CPX). Our preliminary studies identified that CPX treatment affected Notch signaling pathway. Notch signaling is initiated when a ligand interacts with a notch transmembrane receptor on adjacent cells. This initiates the γ-secretase mediated proteolytic release of the Notch intracellular domain (NICD), which in turn translocates into the nucleus of the cells, interacts with its transcriptional cofactor CBF1/RBPJ and transactivates target genes, leading to increased proliferation and metastasis. Notch signaling has been shown to play a critical role in cancer stem cell self-renewal and earlier studies have suggested that inhibiting notch pathway could be a good mechanism for stem cell based therapy.

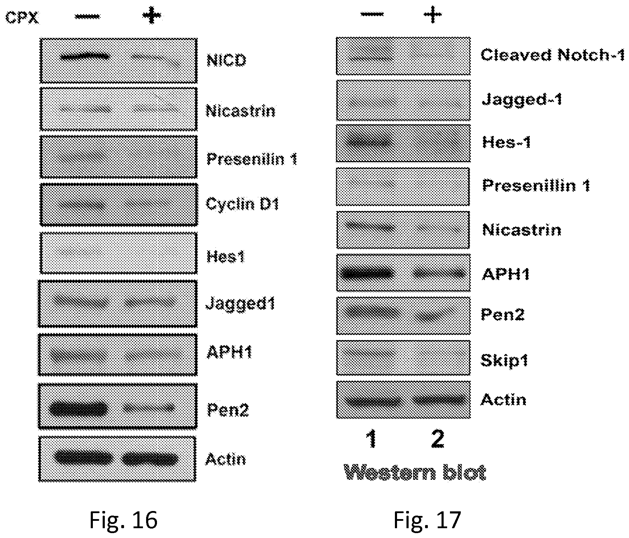

Previous studies have demonstrated that high levels of Notch1 activity is associated with bladder cancer tumor progression, and that Notch1 may be potential therapeutic target for treating bladder cancer. Western blot studies were performed to detect the effect of CPX on the expression of proteins involved in Notch signaling pathway. Cell lysates were subjected to polyacrylamide gel electrophoresis and blotted onto Immobilon polyvinylidene difluoride membranes (Millipore, Bedford, MA, USA). Antibodies were purchased from Cell Signaling Technology (Beverly, MA, USA), Abcam Inc. (Cambridge, MA, USA) and Santa Cruz Biotechnology Inc. (Santa Cruz, CA, USA) and specific proteins were detected by the enhanced chemiluminescence system (GE Healthcare, Piscataway, NJ, USA). CPX treatment in bladder cancer cell lines significantly inhibited the expression of the γ-secretase complex proteins such as Presenilin1, Nicastrin, APH-1 and PEN-2 thereby resulting in decreased cleaved activated Notch intracellular domain (NICD1). CPX also reduced Jagged1, a ligand for Notch receptor. In addition, the levels of downstream targets of the pathway including Hes1 and Cyclin-D1 were also significantly inhibited following CPX treatment. These data suggest that CPX significantly inhibits Notch intracellular signaling pathway proteins, thereby inhibiting CSC growth. See Figures 16-17.

Figures 16-17 show that CPX inhibits Notch1 signaling and γ-secretase complex proteins. CPX treatment significantly reduced Notch1 activation and its ligand Jagged1. Also it reduces the downstream target proteins Hes-1 and cyclin D1. In addition, CPX inhibited expression of γ-secretase complex proteins Presenilin1, Nicastrin, APH-1 and PEN-2 in T24 cells. On the right is shown the Notch signaling pathway.

### Ciclopirox inhibits cell growth through inactivation of the γ-secretase complex:

We determined whether ectopic expression of NICD reversed ciclopirox (CPX) mediated inhibition of cell proliferation. For this study, T24 Cells were transfected with plasmid EF.hICN1.CMV.GFP encoding the Notch-1 intracellular domain (NICD) or the empty vector EF.v-CMV.GFP (Addgene Inc.) and subsequently treated with CPX (4 µM). Cell proliferation was detected using hexoaminidase assay. Western blot analyses demonstrated increased expression of Notch-1 following ectopic expression of NICD in T24 cell lines. Ectopic expression of NICD reversed CPX mediated inhibition of cell proliferation. These data suggest that CPX inhibits the γ-secretase complex thereby affecting Notch signaling.

Notch-1 expression is associated with tumor progression, and is implicated in the carcinogenesis and progression of invasive bladder transitional cell carcinoma (J Clin Oncol 30, 2012 (suppl 5; abstr 299)). It has been suggested that Notch-1 represents a therapeutic target for the treatment of bladder cancer (Oncogene (2008) 27, 5132-5137). Notch-1 signaling may also represent a potential diagnostic marker for progression of NMIBC to MIBC (J Clin Oncol 30, 2012 (suppl 5; abstr 299)). Data generated in our laboratory suggest that CPX inhibits bladder cancer growth *in vitro* by suppressing Notch-1 signaling pathway.

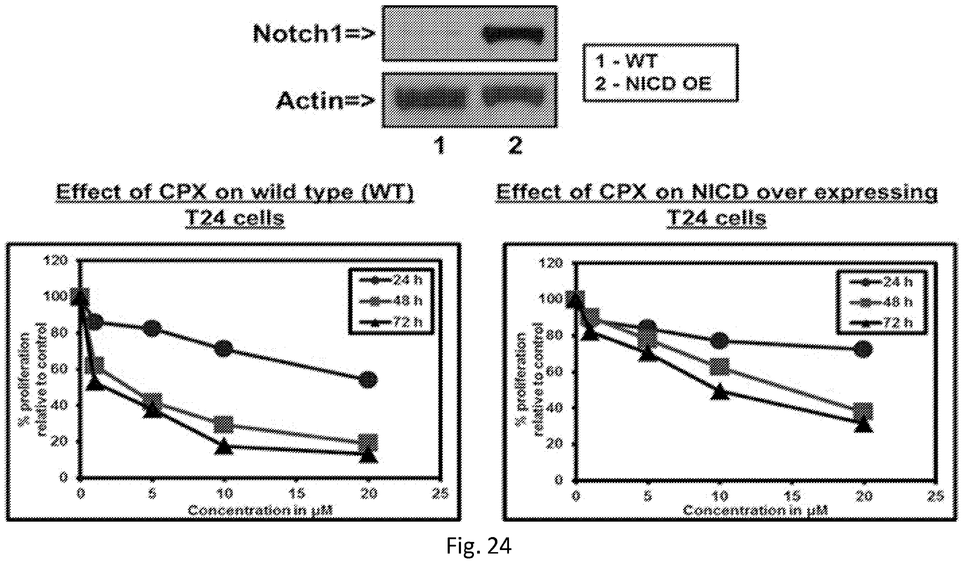

Figure 24 shows CPX inhibits cell growth through inactivation of γ-secretase complex proteins. Cells expressing NICD were treated with CPX and subsequently measured for proliferation. Ectopic expression of NICD rescued CPX mediated inhibition of cell proliferation.

ciclopirox-POM prodrug (CPX-POM) or ciclopirox glucuronide (CPX-G) show very less anticancer activity in vitro:

We determined and compared the effect of CPX with CPX-POM and CPX-glucuronide *in vitro* by performing proliferation assay. To determine the effect of CPX, CPX-POM and CPX-glucuronide on bladder cancer cell proliferation in T24 (MIBC) and 253-JBV (NMIBC) cells, 5,000 cells per well were seeded on to 96-well plates and grown overnight. The cells were then treated with increasing doses of CPX, CPX-POM and CPX-glucuronide in RPMI media containing 10% FBS. Analysis of cell proliferation after the treatment period was estimated by the hexosaminidase assay. Briefly, the medium was removed and hexosaminidase substrate solution in citrate buffer pH 5 (7.5 mM), p-nitrophenol-N-acetyl-beta-D-glucosaminidase (Sigma Aldrich) was added at 75 *µ*L per well. The plate was incubated at 37°C in 100% humidity for 30 minutes. The reaction was stopped with 112.5 *µ*L of 50 mM glycine containing 5 mM of EDTA (pH 10.4). The absorbance was measured at 405 nm.

Experiments were conducted at *n* = 6, and then repeated at least three times. The data were analyzed as percent of control, where the control wells were treated with equivalent amounts of DMSO alone. For IC₅₀ calculations, a plot between the drug concentration and hexosaminidase activity was generated and the data was fitted either linearly or exponentially. IC₅₀ was obtained by determining the concentration of compounds resulting in 50% of cell death after 48 h of treatment.

Results from this experiment demonstrate that CPX significantly suppressed the proliferation of both cell lines (T24 and 253JBV) in a dose and time dependent manner (IC₅₀=>4 µM and 3 µM respectively in T24 and 253JBV cells). Whereas CPX-POM show a very less effect on proliferation (IC₅₀=>45 µM and 48 µM respectively in T24 and 253JBV cells) and CPX-glucuronide did not show any significant effect at all up to 72h of treatment in both the cells. At the time of this summary, experiments evaluating the chemical stability of CPX-POM in cell culture media are ongoing to evaluate whether CPX-POM forms CPX in vitro.

Predicted CPX concentration in human urine show sufficient anticancer activity:

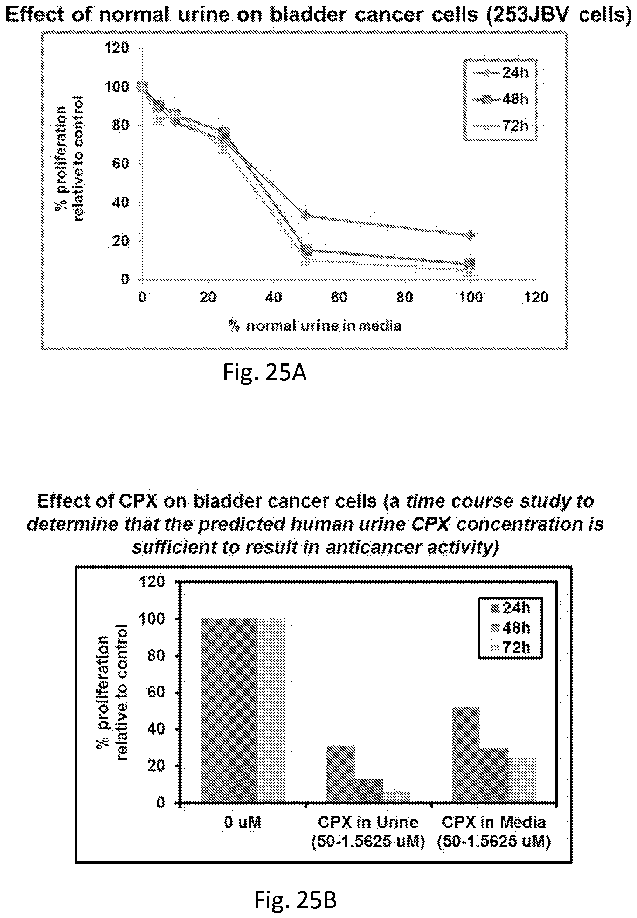

We determined whether the predicted CPX concentration in human urine is sufficient enough to result in anticancer activity. We treated the cells with CPX in the presence of normal urine (procured from the bio specimen repository of KUMC) to mimic the environment in bladder. Meanwhile, a percentage dose curve was performed to determine the percentage of urine that can be used for further studies which is non-toxic to the cells (Figure 25A). Figure 25A shows the effect of normal urine on cells. 253JBV Cells were incubated with the indicated percentage of normal urine for up to 72 h. Urine concentration of less than 20% is found to be non-toxic to the cells.

The 253JBV cells were incubated with various percentages of urine in complete RPMI media for up to 12 hours. After 12 hours the media was replaced with 100% complete RPMI media. The cells were allowed to grow up to 72h. Hexosaminidase assay was performed to determine the effect of urine at different time points (24h, 48h and 72h). It is determined that 20% normal urine can be used for further studies, as it is not found to be toxic on the cells for the given period of time (12h).

To determine whether the predicted CPX concentration in human urine is sufficient enough to result in anticancer activity, NMIBC cells were incubated with CPX in normal urine (20 % normal urine in complete RPMI media), in which cells are exposed to 50 uM CPX for two hours, followed by exposure to 25 uM CPX for two hours, followed by exposure to 12.5 uM CPX for two hours, followed by exposure to 6.25 uM CPX for two hours, followed by exposure to 3.125 uM CPX for two hours, and finally, followed by exposure to 1.5625 uM CPX for two hours. The effect of CPX was determined by hexosaminidase assay beyond the 12 hours of varying CPX concentration (24h, 48h, and 72h). The effect of CPX in normal urine is compared with effect of CPX in complete media.

Results from this experiment, as shown in Figure 25B demonstrate that the predicted urine concentration of CPX in human urine is sufficient enough to inhibit the proliferation of cells. CPX is found to be exhibiting its anti-proliferative effect beyond 12 hours and the effect continues up to 72h.

We next determined the effect of CPX in normal urine on T24 and 253JBV cells upon everyday treatment for up to 72h (3 days treatment). The idea behind this experiment is to see the effect of predicted concentration of CPX in human urine on bladder cancer cells as the patient's bladder will be exposed every day to the predicted concentration of CPX. Three sets of plates were used, one plate each for one day treatment, two days treatment and three days treatment of CPX (50-1.5625 uM). T24 and 253JBV cells were incubated with CPX in normal urine (20% normal urine in complete RPMI media). In one day treatment, the cells are exposed to 50 uM CPX for two hours, followed by exposure to 25 uM CPX for two hours, followed by exposure to 12.5 uM CPX for two hours, followed by exposure to 6.25 uM CPX for two hours, followed by exposure to 3.125 uM CPX for two hours, and finally, followed by exposure to 1.5625 uM CPX for two hours. The effect of CPX was determined by hexosaminidase assay beyond the 12 hours of varying CPX concentration (24h, 48h, and 72h). In two days treatment, the cells are exposed to 50 uM-1.5625 uM for two days and the effect was determined by hexosaminidase assay at 48h and 72h time points. In three days treatment, the cells are exposed to 50 uM-1.5625 uM for three days and the effect was determined by hexosaminidase assay at 72h time point.

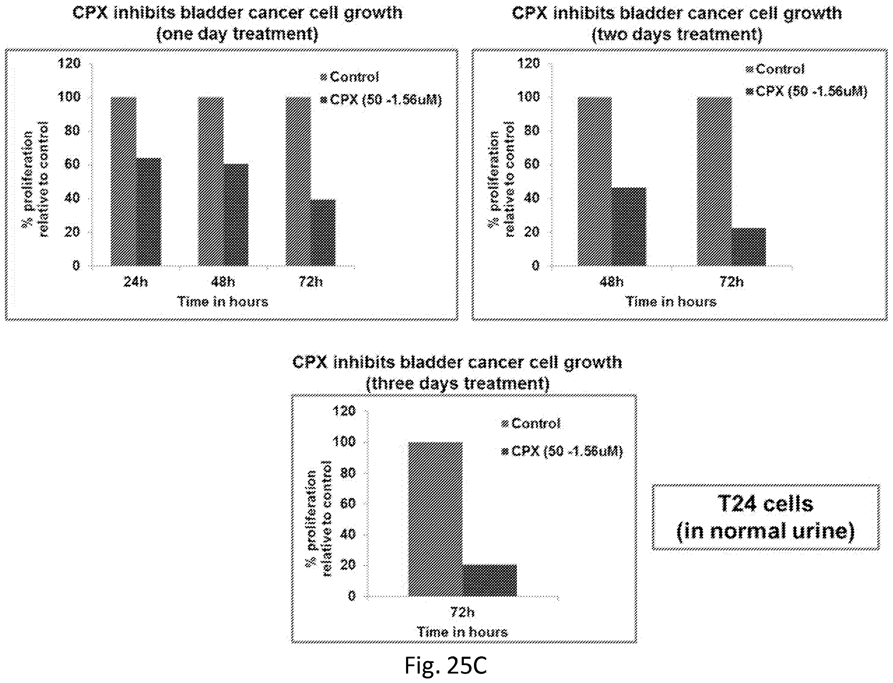

Figure 25C shows that every day treatment of T24 cells with predicted CPX concentration in human urine shows significant anticancer activity. T24 Cells were incubated with CPX in normal urine (20 % normal urine in complete RPMI media). In one day treatment, the cells are exposed to varying CPX concentration (50 uM-1.5625 uM) for 12h. The effect of CPX was determined by hexosaminidase assay beyond the 12 hours of varying CPX concentration (24h, 48h, and 72h). In two days treatment, the cells are exposed to 50 uM-1.5625 uM for two days and the effect was determined by hexosaminidase assay at 48h and 72h time points. In three days treatment, the cells are exposed to 50 uM-1.5625 uM for three days and the effect was determined by hexosaminidase assay at 72h time point. T24 cells incubated with CPX showed a time dependent reduction in the proliferation with cells treated for 3 days showing the maximum inhibition of proliferation (20%).

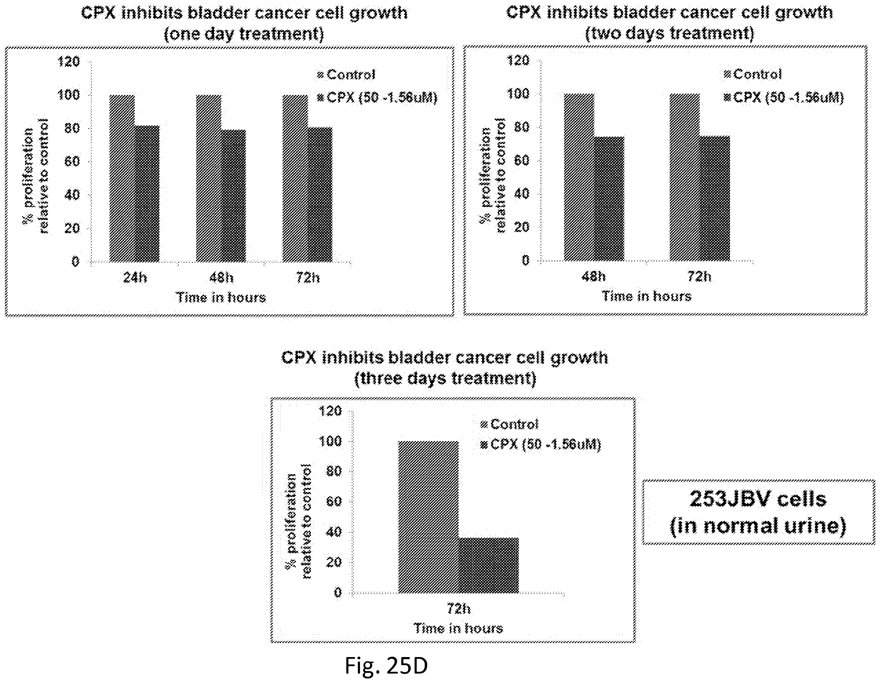

Figure 25D shows that every day treatment of 253JBV cells with predicted CPX concentration in human urine shows significant anticancer activity. 253JBV Cells were incubated with CPX in normal urine (20 % normal urine in complete RPMI media). In one day treatment, the cells are exposed to varying CPX concentration (50 uM-1.5625 uM) for 12h. The effect of CPX was determined by hexosaminidase assay beyond the 12 hours of varying CPX concentration (24h, 48h, and 72h). In two days treatment, the cells are exposed to 50 uM-1.5625 uM for two days and the effect was determined by hexosaminidase assay at 48h and 72h time points. In three days treatment, the cells are exposed to 50 uM-1.5625 uM for three days and the effect was determined by hexosaminidase assay at 72h time point. 253JBV cells incubated with CPX showed a time dependent decrease in the proliferation with cells treated for 3 days showing the maximum inhibition of proliferation (35%).

Results from this experiment, as shown in Figure 25C-25D demonstrate that the predicted urine concentration of CPX in human urine shows a time dependent decrease in the proliferation of cells. In both the cell lines such as T24 and 253JBV, CPX treatment for 3 days shows the maximum inhibition of proliferation 20% and 35% respectively.

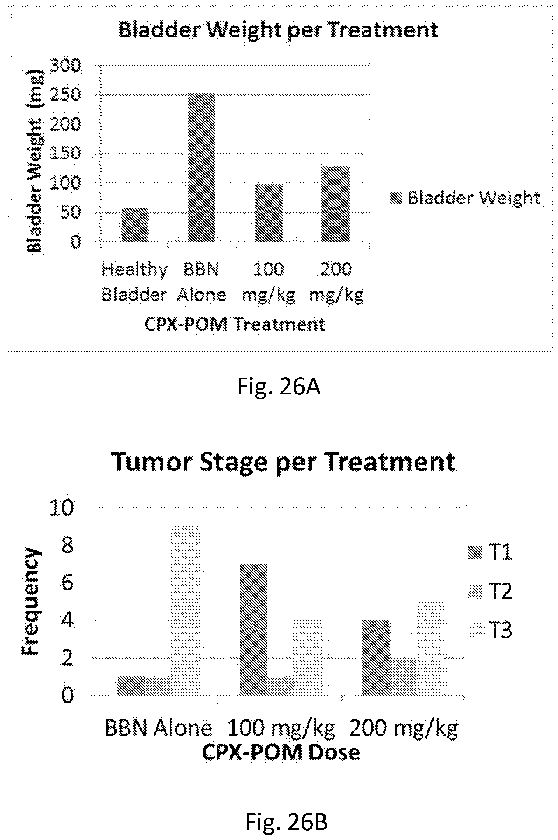

Figures 26A-26B both include a graph that shows CPX-POM reduces bladder weight of BBN induced bladder tumors. Following four weeks of once daily IP CPX-POM doses of 100 mg/kg and 200 mg/kg, bladder weights (as a measure of tumor volume) were significantly lower (p=0.013) in CPX-POM treated animals compared to controls. There were no significant differences in bladder weights between the two CPX-POM treatment groups. Animals given BBN alone showed an increase in the bladder weight, due to the induction and growth of tumor, when compared with control healthy bladder. CPX-POM treatment significantly reduced the bladder weight both at 100 and 200 mg/kg treatment doses.

There is a strong correlation between CPX-POM treatment and tumor stage. Bladder tissues obtained from control (BBN alone) animals contained predominantly high grade (T3) tumors while CPX-POM treated animals demonstrated a clear migration to lower stage tumors. Hematoxylin and Eosin stained bladder tissue sections of BBN alone group animals show a very high frequency of T3 stage tumors. Tissue sections from CPX-POM treated bladders show a significant reduction in the stage T3 frequency, indicating that CPX-POM arrests the tumor at its early stage.

Previous studies have demonstrated that high levels of Notch1 activity are associated with bladder cancer tumor progression, and that Notch1 may be a potential therapeutic target for treating bladder cancer. Tissue sections from CPX-POM treated bladder tissues significantly inhibited the expression of γ-secretase complex proteins such as Presenilin1, thereby resulting in decreased cleaved activated Notch intracellular domain (NICD1). In addition, the levels of downstream targets of the pathway including Hey1 were also significantly inhibited following CPX-POM treatment. These data suggest that CPX-POM significantly inhibits Notch intracellular signaling pathway proteins, thereby inhibiting cancer stem cell (CSC) growth *in vivo.*

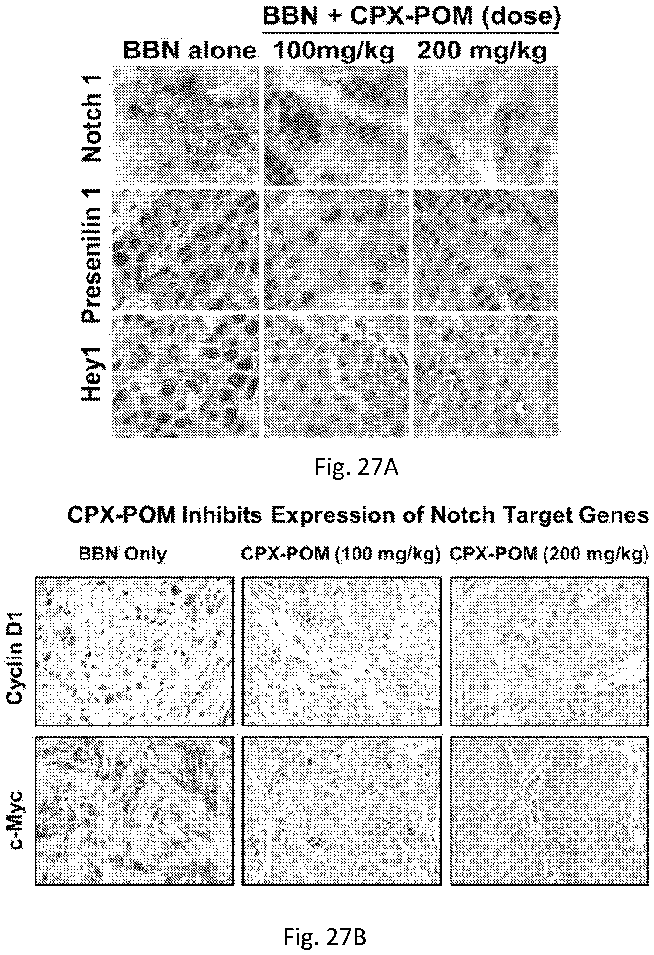

Figures 27A include images that show CPX-POM inhibits Notch1 signaling and γ-secretase complex proteins *in vivo.* CPX-POM treatment significantly reduced Notch1 activation and γ-secretase complex protein, Presenilin1, and reduces the downstream target protein such as Hey-1. Inhibition of the Notch signaling pathway was observed in both CPX-POM treatment groups.

Figure 27B includes images that show CPX-POM inhibits Notch downstream target proteins, Cyclin D1 and c-Myc in BBN induced bladder tumor model. CPX-POM significantly reduced the Notch downstream target proteins such as Cyclin D1 and c-Myc, which plays an important role in cell growth. As such, CPX-POM treatment also results in inhibition of Notch downstream target proteins, Cyclin D1 and c-Myc in the BBN induced bladder tumor model. These downstream target proteins play a major role in progression of cells through cell cycle. In conclusion, we established in vivo preclinical proof of principle in a validated mouse model of bladder cancer. We demonstrated that sufficient concentrations of ciclopirox are achieved at the target site of action (i.e., the urinary tract_, for sufficient length of time (i.e., over 24 hours) at steady-state, following injectable CPX-POM administration. Secondly, we have established that ciclopirox engages with the proposed targets (e.g., Notch signaling pathway), at the site of action. Thirdly, we have established that ciclopirox-target engagement at the site of action results in pharmacologic activity consistent with the proposed mechanism of action (tumor size reduction, tumor stage migration).

CPX-POM inhibits proliferating cell nuclear antigen (PCNA) both *in vitro* and *in vivo.* We examined the PCNA content in the Non-Muscle invasive bladder cancer cell lines and in CPX-POM treated bladder tumor tissues Western blot studies show a time dependent decrease in the level of PCNA in CPX-POM treated T24 cells. CPX-POM reduced the level of PCNA *in vivo.* Also, studies show a positive correlation between notch intracellular pathway and PCNA. Data generated in our laboratory suggest that CPX-POM inhibits bladder cancer growth *in vivo* by suppressing Notch-1 signaling pathway, Notch downstream target proteins and Proliferating cell nuclear antigen.

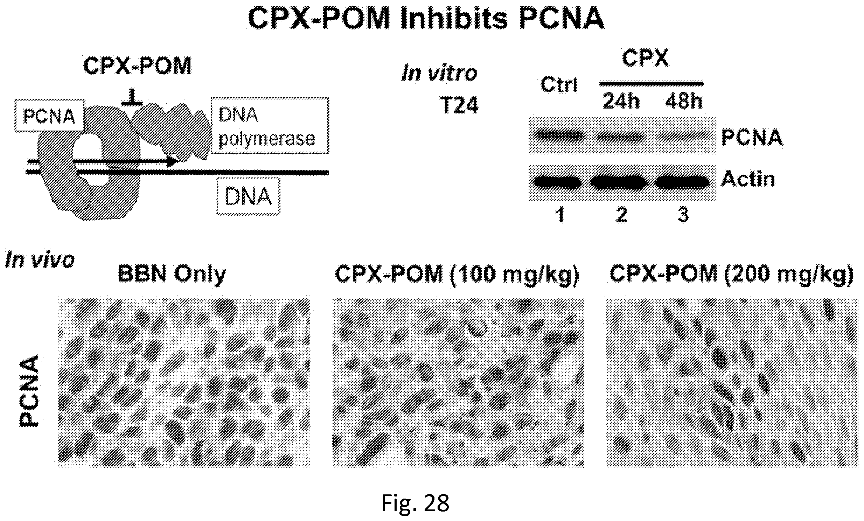

Figure 28 shows that CPX-POM inhibits proliferating cell nuclear antigen (PCNA) both *in vitro* and *in vivo.* The cartoon explains the role of PCNA in holding DNA polymerase together with DNA which plays an essential role in the DNA replication. Western blot studies show a time dependent decrease in the level of PCNA in CPX treated T24 cells. CPX-POM also reduced the level of PCNA *in vivo.*

Additionally, data from the provisional application (incorporated herein) shows: CPX suppresses Notch, Wnt and Hedgehog pathways in T24 cell lines; CPX suppresses NOTCH Pathway related genes in T24 cell lines; CPX suppresses Wnt signaling Pathway related genes in T24 cell lines; and CPX suppresses Hedgehog Pathway related genes in T24 cell lines.

The data provided herein provides the following information. The POM moiety is well understood to be metabolized by phosphatases that circulate in blood. Complete metabolism of CPX-POM occurs to form ciclopirox. Following administration of IV CPX-POM to mice, ciclopirox is completely bioavailable compared to IV bolus administration of ciclopirox olamine. Following administration of IV CPX-POM to rats, the bioavailability of ciclopirox is 77% compared to IV bolus administration of ciclopirox olamine. Following administration of IV CPX-POM to dogs, the bioavailability of ciclopirox is 85% compared to IV bolus administration of ciclopirox olamine. CPX-POM has rarely been detected in plasma and urine of mice, rats and dogs participating in pharmacokinetic studies following IV, SQ and oral administration of the prodrug. CPX-POM administration by injectable routes of administration at well tolerated doses result in concentrations of ciclopirox in plasma and urine that exceed reported in vitro IC50 values. Ciclopirox is bioavailable following administration of CPX-POM by the subcutaneous route of administration in rats and dogs. Plasma ciclopirox concentrations following well tolerated IV doses of CPX-POM exceed reported in vitro IC50 values. Plasma ciclopirox concentrations following well tolerated IV and SQ doses of CPX-POM exceed reported in vitro IC50 values. Urine ciclopirox concentrations, following IV, SQ and oral administration of CPX-POM exceed in vitro IC50 values for non-muscle invasive and muscle invasive bladder cancer in mice, rats, and dogs. IP CPX-POM doses of 50, 100 and 200 mg/kg to mice result in urine concentrations of ciclopirox that exceed in vitro IC50 values by several fold. IV 10 mg/kg and IP 50 mg/kg CPX-POM doses result in single dose urine concentrations of ciclopirox that exceed in vitro IC50 values by several fold. Following IV and SQ doses of CPX-POM to rats, urine concentrations of ciclopirox exceed in vitro IC50 values ranging from 2-4 µM at well tolerated doses. Following IV and SQ doses of CPX-POM to dogs, urine concentrations of ciclopirox exceed in vitro IC50 values ranging from 2-4 µM at well tolerated doses.

The predicted CPX concentration in human urine is sufficient enough to result in anticancer activity (e.g., anti-bladder cancer or other cancer described herein). Every day treatment procedure with predicted concentration of CPX in human urine show maximum inhibition of cells. Additionally, the compounds of the present invention are useful for treating upper urinary tract disease.

The data provided herein clearly shows that ciclopirox, ciclopirox olamine, or ciclopirox-POM prodrug can be used for the treatments described herein. Particularly, any of the bladder cancer treatments can be performed with ciclopirox, ciclopirox olamine, or ciclopirox-POM prodrug.

In summary, CPX-POM is rapidly and completely converted *in vivo* to the active agent ciclopirox after administration. Mouse Study 208-02 demonstrated that the extent of ciclopirox exposure is essentially the same (137%) whether administering IV CPX-POM or the olamine salt of ciclopirox. Rat Study 10954 demonstrated that the extent of ciclopirox exposure is essentially the same (77%) whether administering IV CPX-POM or the olamine salt of ciclopirox. Dog Study 10953 demonstrated that the extent of ciclopirox exposure is essentially the same (85%) whether administering IV CPX-POM or the olamine salt of ciclopirox. Also, as described across all studies, we can't detect CPX-POM in plasma nor in urine really in any study. These studies demonstrated that the IV route of administration for CPX-POM is a viable route.

The subcutaneous route of administration for CPX-POM is bioavailable. Rat Study 11428 demonstrated that the absolute bioavailability of ciclopirox following SQ administration of CPX-POM is 80%, and urine concentrations of ciclopirox following SQ CPX-POM exceeded in vitro IC50 values. Dog Study 11429 demonstrated that the absolute bioavailability of ciclopirox following SQ administration of CPX-POM is 106%, and urine concentrations of ciclopirox following SQ CPX-POM exceeded in vitro IC50 values. These studies, along with Mouse Study 208-02 also demonstrated that the oral bioavailability of ciclopirox olamine is quite low. This route of administration is also associated with lower urine concentrations of the drug, thereby establishing that the subcutaneous route of administration is beneficial, especially in the event we need to administer CPX-POM repeatedly (like a Type I diabetic would administer insulin).

The data established that CPX-POM can be used to treat baldder cancer as shown in a validated mouse model of bladder cancer. Mouse Study 11177 established that the intraperitoneal (IP) route of administration (to be used in the mouse bladder cancer study) is bioavailable. Mouse Study 11206 established that ciclopirox exposure (e.g., urine concentrations) at the site of action (i.e., urinary tract) using dosage regimens selected for the mouse bladder cancer study exceeded in vitro IC50 values by several fold.

The studies established that sufficient concentrations of active drug (ciclopirox) are achieved at the site(s) of action (i.e., blood, urine) following injectable administration of CPX-POM. The Mouse BBN Study established that 100 mg/kg and 200 mg/kg daily doses of CPX-POM resulted in significant reductions in bladder weight (a measure of tumor size and volume) as well as a migration toward lower stage tumors. The studies also established that, in bladder tissues obtained from CPX-POM treated animals, Notch signaling was inhibited, and downstream Notch target proteins were decreased. The analysis of bladder cancer tissues in CPX-POM treated animals established that ciclopirox engages with the target (e.g., Notch signaling pathway) at the site of action (bladder tissue). The effects seen in bladder cancer tissues are consistent with data generated in vitro in NMIBC and MIBC human cell lines. The studies also established that daily injectable administration of CPX-POM produced a pharmacologic effect (e.g., decreased tumor size and volume, lower tumor stage) consistent with the hypothesis that ciclopirox is an anticancer agent and that injectable administration of CPX-POM delivers sufficient active drug to the site of action to produce the anticancer response.

The term "alkyl" or "aliphatic" as used herein refers to a branched or unbranched saturated hydrocarbon group typically although not necessarily containing 1 to about 24 carbon atoms, such as methyl, ethyl, n-propyl, isopropyl, n-butyl, isobutyl, t-butyl, octyl, decyl, and the like, as well as cycloalkyl groups such as cyclopentyl, cyclohexyl, and the like. Generally, although again not necessarily, alkyl groups herein contain 1 to about 18 carbon atoms, or 1 to about 12 carbon atoms. The term "lower alkyl" intends an alkyl group of 1 to 6 carbon atoms. Substituents identified as "C₁ -C₆ alkyl" or "lower alkyl" contains 1 to 3 carbon atoms, and such substituents contain 1 or 2 carbon atoms (i.e., methyl and ethyl). "Substituted alkyl" refers to alkyl substituted with one or more substituent groups, and the terms "heteroatom-containing alkyl" and "heteroalkyl" refer to alkyl in which at least one carbon atom is replaced with a heteroatom, as described in further detail infra. If not otherwise indicated, the terms "alkyl" and "lower alkyl" include linear, branched, cyclic, unsubstituted, substituted, and/or heteroatom-containing alkyl or lower alkyl, respectively.

The terms "alkenyl" as used herein refers to a linear, branched or cyclic hydrocarbon group of 2 to about 24 carbon atoms containing at least one double bond, such as ethenyl, n-propenyl, isopropenyl, n-butenyl, isobutenyl, octenyl, decenyl, tetradecenyl, hexadecenyl, eicosenyl, tetracosenyl, and the like. Generally, although again not necessarily, alkenyl groups herein contain 2 to about 18 carbon atoms, or 2 to 12 carbon atoms. The term "lower alkenyl" intends an alkenyl group of 2 to 6 carbon atoms, and the specific term "cycloalkenyl" intends a cyclic alkenyl group, or having 5 to 8 carbon atoms. The term "substituted alkenyl" refers to alkenyl substituted with one or more substituent groups, and the terms "heteroatom-containing alkenyl" and "heteroalkenyl" refer to alkenyl in which at least one carbon atom is replaced with a heteroatom. If not otherwise indicated, the terms "alkenyl" and "lower alkenyl" include linear, branched, cyclic, unsubstituted, substituted, and/or heteroatom-containing alkenyl and lower alkenyl, respectively.

The term "alkynyl" as used herein refers to a linear or branched hydrocarbon group of 2 to 24 carbon atoms containing at least one triple bond, such as ethynyl, n-propynyl, and the like. Generally, although again not necessarily, alkynyl groups herein contain 2 to about 18 carbon atoms, or 2 to 12 carbon atoms. The term "lower alkynyl" intends an alkynyl group of 2 to 6 carbon atoms. The term "substituted alkynyl" refers to alkynyl substituted with one or more substituent groups, and the terms "heteroatom-containing alkynyl" and "heteroalkynyl" refer to alkynyl in which at least one carbon atom is replaced with a heteroatom. If not otherwise indicated, the terms "alkynyl" and "lower alkynyl" include linear, branched, unsubstituted, substituted, and/or heteroatom-containing alkynyl and lower alkynyl, respectively.

The term "alkoxy" as used herein intends an alkyl group bound through a single, terminal ether linkage; that is, an "alkoxy" group may be represented as -O-alkyl where alkyl is as defined above. A "lower alkoxy" group intends an alkoxy group containing 1 to 6 carbon atoms, and includes, for example, methoxy, ethoxy, n-propoxy, isopropoxy, t-butyloxy, etc. Substituents identified as "C₁-C₆ alkoxy" or "lower alkoxy" herein contain 1 to 3 carbon atoms, and such substituents contain 1 or 2 carbon atoms (i.e., methoxy and ethoxy).

The term "aryl" as used herein, and unless otherwise specified, refers to an aromatic substituent containing a single aromatic ring or multiple aromatic rings that are fused together, directly linked, or indirectly linked (such that the different aromatic rings are bound to a common group such as a methylene or ethylene moiety). Examples of aryl groups contain 5 to 20 carbon atoms, and aryl groups contain 5 to 14 carbon atoms. Exemplary aryl groups contain one aromatic ring or two fused or linked aromatic rings, e.g., phenyl, naphthyl, biphenyl, diphenylether, diphenylamine, benzophenone, and the like. "Substituted aryl" refers to an aryl moiety substituted with one or more substituent groups, and the terms "heteroatom-containing aryl" and "heteroaryl" refer to aryl substituent, in which at least one carbon atom is replaced with a heteroatom, as will be described in further detail infra. If not otherwise indicated, the term "aryl" includes unsubstituted, substituted, and/or heteroatom-containing aromatic substituents.

The term "aryloxy" as used herein refers to an aryl group bound through a single, terminal ether linkage, wherein "aryl" is as defined above. An "aryloxy" group may be represented as -O-aryl where aryl is as defined above. Examples of aryloxy groups contain 5 to 20 carbon atoms, and aryloxy groups contain 5 to 14 carbon atoms. Examples of aryloxy groups include, without limitation, phenoxy, o-halo-phenoxy, m-halo-phenoxy, p-halo-phenoxy, o-methoxy-phenoxy, m-methoxy-phenoxy, p-methoxy-phenoxy, 2,4-dimethoxy-phenoxy, 3,4,5-trimethoxy-phenoxy, and the like.

The term "alkaryl" refers to an aryl group with an alkyl substituent, and the term "aralkyl" refers to an alkyl group with an aryl substituent, wherein "aryl" and "alkyl" are as defined above. Examples of aralkyl groups contain 6 to 24 carbon atoms, and aralkyl groups contain 6 to 16 carbon atoms. Examples of aralkyl groups include, without limitation, benzyl, 2-phenyl-ethyl, 3-phenyl-propyl, 4-phenyl-butyl, 5-phenyl-pentyl, 4-phenylcyclohexyl, 4-benzylcyclohexyl, 4-phenylcyclohexylmethyl, 4-benzylcyclohexylmethyl, and the like. Alkaryl groups include, for example, p-methylphenyl, 2,4-dimethylphenyl, p-cyclohexylphenyl, 2,7-dimethyinaphthyl, 7-cyclooctylnaphthyl, 3-ethyl-cyclopenta-1,4-diene, and the like.

The term "cyclic" refers to alicyclic or aromatic substituents that may or may not be substituted and/or heteroatom containing, and that may be monocyclic, bicyclic, or polycyclic.

The terms "halo" and "halogen" are used in the conventional sense to refer to a chloro, bromo, and fluoro or iodo substituent.

The term "heteroatom-containing" as in a "heteroatom-containing alkyl group" (also termed a "heteroalkyl" group) or a "heteroatom-containing aryl group" (also termed a "heteroaryl" group) refers to a molecule, linkage or substituent in which one or more carbon atoms are replaced with an atom other than carbon, e.g., nitrogen, oxygen, sulfur, phosphorus or silicon, typically nitrogen, oxygen or sulfur. Similarly, the term "heteroalkyl" refers to an alkyl substituent that is heteroatom-containing, the term "heterocyclic" refers to a cyclic substituent that is heteroatom-containing, the terms "heteroaryl" and heteroaromatic" respectively refer to "aryl" and "aromatic" substituents that are heteroatom-containing, and the like. Examples of heteroalkyl groups include alkoxyaryl, alkylsulfanyl-substituted alkyl, N-alkylated amino alkyl, and the like. Examples of heteroaryl substituents include pyrrolyl, pyrrolidinyl, pyridinyl, quinolinyl, indolyl, pyrimidinyl, imidazolyl, 1,2,4-triazolyl, tetrazolyl, etc., and examples of heteroatom-containing alicyclic groups are pyrrolidino, morpholino, piperazino, piperidino, etc.

The term "hydrocarbyl" refers to univalent hydrocarbyl radicals containing 1 to about 30 carbon atoms, or 1 to about 24 carbon atoms, or 1 to about 18 carbon atoms, or about 1 to 12 carbon atoms, including linear, branched, cyclic, saturated, and unsaturated species, such as alkyl groups, alkenyl groups, aryl groups, and the like. "Substituted hydrocarbyl" refers to hydrocarbyl substituted with one or more substituent groups, and the term "heteroatom-containing hydrocarbyl" refers to hydrocarbyl in which at least one carbon atom is replaced with a heteroatom. Unless otherwise indicated, the term "hydrocarbyl" is to be interpreted as including substituted and/or heteroatom-containing hydrocarbyl moieties.

By "substituted" as in "substituted alkyl," "substituted aryl," and the like, as alluded to in some of the aforementioned definitions, is meant that in the alkyl, aryl, or other moiety, at least one hydrogen atom bound to a carbon (or other) atom is replaced with one or more non-hydrogen substituents.

In addition, the aforementioned functional groups may, if a particular group permits, be further substituted with one or more additional functional groups or with one or more hydrocarbyl moieties such as those specifically enumerated above. Analogously, the above-mentioned hydrocarbyl moieties may be further substituted with one or more functional groups or additional hydrocarbyl moieties such as those specifically enumerated.

When the term "substituted" appears prior to a list of possible substituted groups, it is intended that the term apply to every member of that group. For example, the phrase "substituted alkyl, alkenyl, and aryl" is to be interpreted as "substituted alkyl, substituted alkenyl, and substituted aryl." Analogously, when the term "heteroatom-containing" appears prior to a list of possible heteroatom-containing groups, it is intended that the term apply to every member of that group. For example, the phrase "heteroatom-containing alkyl, alkenyl, and aryl" is to be interpreted as "heteroatom-containing alkyl, heteroatom-containing alkenyl, and heteroatom-containing aryl."

It will be understood by those within the art that, in general, terms used herein, and especially in the appended claims (e.g., bodies of the appended claims) are generally intended as "open" terms (e.g., the term "including" should be interpreted as "including but not limited to," the term "having" should be interpreted as "having at least," the term "includes" should be interpreted as "includes but is not limited to," etc.). It will be further understood by those within the art that if a specific number of an introduced claim recitation is intended, such an intent will be explicitly recited in the claim, and in the absence of such recitation no such intent is present. For example, as an aid to understanding, the following appended claims may contain usage of the introductory phrases "at least one" and "one or more" to introduce claim recitations. However, the use of such phrases should not be construed to imply that the introduction of a claim recitation by the indefinite articles "a" or "an" limits any particular claim containing such introduced claim recitation to embodiments containing only one such recitation, even when the same claim includes the introductory phrases "one or more" or "at least one" and indefinite articles such as "a" or "an" (e.g., "a" and/or "an" should be interpreted to mean "at least one" or "one or more"); the same holds true for the use of definite articles used to introduce claim recitations. In addition, even if a specific number of an introduced claim recitation is explicitly recited, those skilled in the art will recognize that such recitation should be interpreted to mean at least the recited number (e.g., the bare recitation of "two recitations," without other modifiers, means at least two recitations, or two or more recitations). Furthermore, in those instances where a convention analogous to "at least one of A, B, and C, etc." is used, in general such a construction is intended in the sense one having skill in the art would understand the convention (e.g., " a system having at least one of A, B, and C" would include but not be limited to systems that have A alone, B alone, C alone, A and B together, A and C together, B and C together, and/or A, B, and C together, etc.). In those instances where a convention analogous to "at least one of A, B, or C, etc." is used, in general such a construction is intended in the sense one having skill in the art would understand the convention (e.g., " a system having at least one of A, B, or C" would include but not be limited to systems that have A alone, B alone, C alone, A and B together, A and C together, B and C together, and/or A, B, and C together, etc.). It will be further understood by those within the art that virtually any disjunctive word and/or phrase presenting two or more alternative terms, whether in the description, claims, or drawings, should be understood to contemplate the possibilities of including one of the terms, either of the terms, or both terms. For example, the phrase "A or B" will be understood to include the possibilities of "A" or "B" or "A and B."

In addition, where features or aspects of the disclosure are described in terms of Markush groups, those skilled in the art will recognize that the disclosure is also thereby described in terms of any individual member or subgroup of members of the Markush group.

As will be understood by one skilled in the art, for any and all purposes, such as in terms of providing a written description, all ranges disclosed herein also encompass any and all possible subranges and combinations of subranges thereof. Any listed range can be easily recognized as sufficiently describing and enabling the same range being broken down into at least equal halves, thirds, quarters, fifths, tenths, etc. As a non-limiting example, each range discussed herein can be readily broken down into a lower third, middle third and upper third, etc. As will also be understood by one skilled in the art all language such as "up to," "at least," and the like include the number recited and refer to ranges which can be subsequently broken down into subranges as discussed above. Finally, as will be understood by one skilled in the art, a range includes each individual member. Thus, for example, a group having 1-3 cells refers to groups having 1, 2, or 3 cells. Similarly, a group having 1-5 cells refers to groups having 1, 2, 3, 4, or 5 cells, and so forth.

## Claims

1. A pharmaceutical composition having ciclopirox or ciclopirox olamine or a ciclopirox-POM prodrug having a structure of Formula 1 or stereoisomer thereof or pharmaceutically acceptable salt thereof for use in the treatment of bladder cancer, wherein 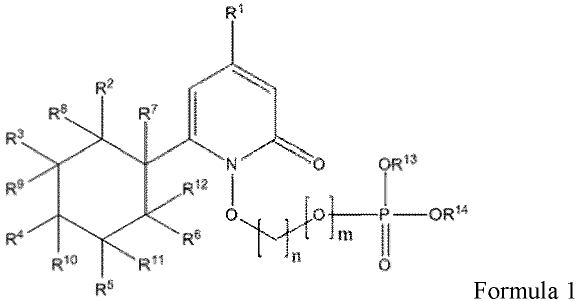 and wherein:
R¹-R¹² each independently include one or more of hydrogen, halogens, hydroxyls, alkoxys, straight aliphatics, branched aliphatics, cyclic aliphatics, substituted aliphatics, unsubstituted aliphatics, saturated aliphatics, unsaturated aliphatics, aromatics, polyaromatics, substituted aromatics, hetero-aromatics, amines, primary amines, secondary amines, tertiary amines, aliphatic amines, carbonyls, carboxyls, amides, esters, amino acids, peptides, polypeptides, substituted or unsubstituted, or combinations thereof;
R¹³-R¹⁴ each independently include one or more of a positive ion, sodium ion, hydrogen, halogens, hydroxyls, alkoxys, straight aliphatics, branched aliphatics, cyclic aliphatics, substituted aliphatics, unsubstituted aliphatics, saturated aliphatics, unsaturated aliphatics, aromatics, polyaromatics, substituted aromatics, hetero-aromatics, amines, primary amines, secondary amines, tertiary amines, aliphatic amines, carbonyls, carboxyls, amides, esters, amino acids, peptides, polypeptides, substituted or unsubstituted, or combinations thereof;
n is 0-20 substituted or unsubstituted; and
m is 0, 1 or 2.

2. The pharmaceutical composition for use according to claim 1, the compound represented by a structure of Formula 2 or Formula 3, or a stereoisomer Formula 2 or Formula 3, or pharmaceutically acceptable salt of Formula 2 or Formula 3.

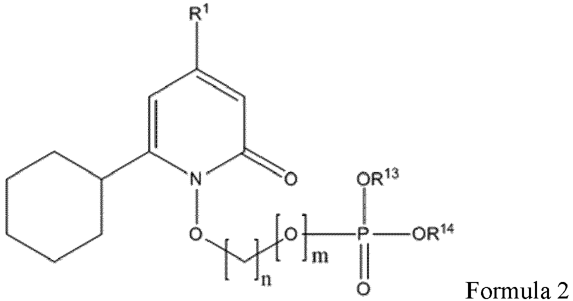

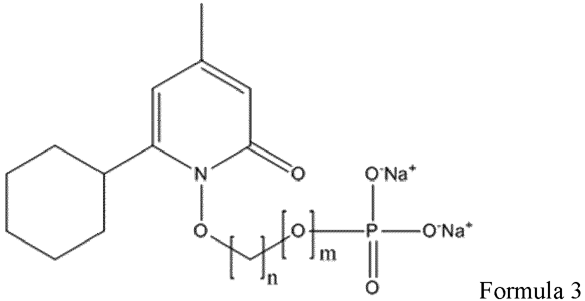

3. The pharmaceutical composition for use according to claim 1, the compound represented by a structure of Formula 4 or stereoisomer thereof or pharmaceutically acceptable salt thereof.

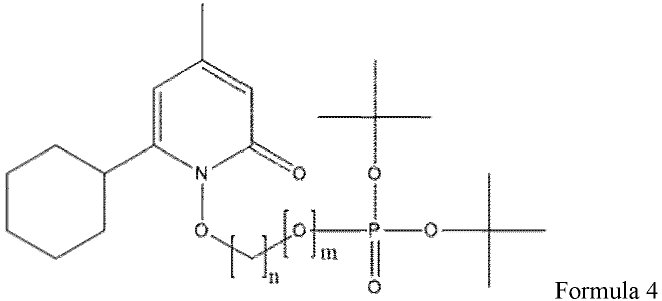

4. The pharmaceutical composition for use according to claim 1, the compound represented by a structure of Formula 5 or stereoisomer thereof or pharmaceutically acceptable salt thereof.

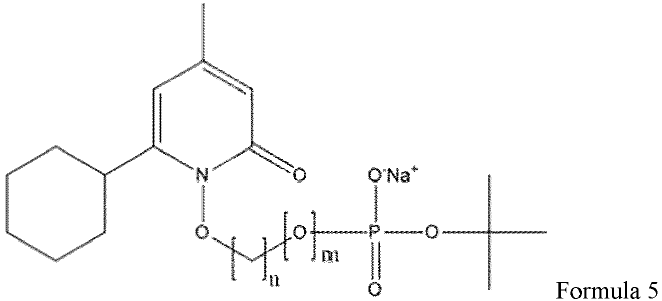

5. The pharmaceutical composition for use according to claim 1, the compound represented by a structure of Formula 6 or stereoisomer thereof or pharmaceutically acceptable salt thereof.

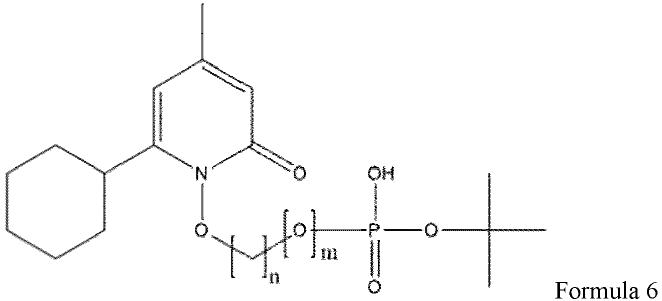

6. The pharmaceutical composition for use according to claim 1, the compound represented by a structure of Formula 7 or stereoisomer thereof or pharmaceutically acceptable salt thereof.

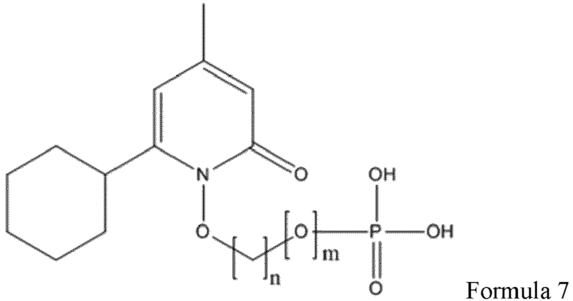

7. The pharmaceutical composition for use according to claim 1, the compound represented by a structure of Formula 8 or stereoisomer thereof or pharmaceutically acceptable salt thereof.

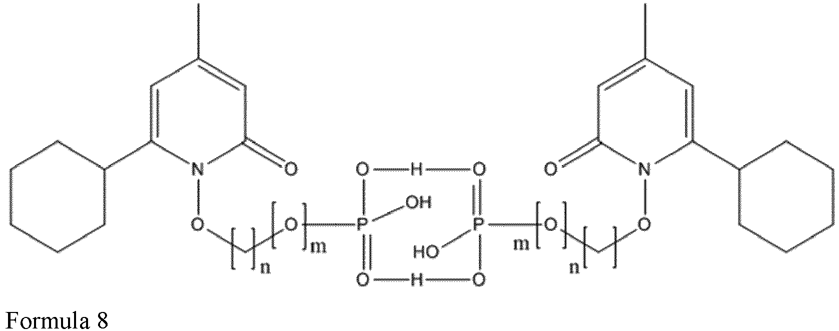

8. The pharmaceutical composition for use according to claim 1, the compound represented by a structure of Formula 9 or stereoisomer thereof or pharmaceutically acceptable salt thereof.

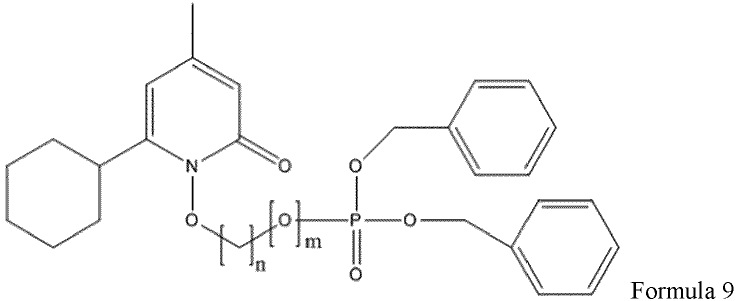

9. The pharmaceutical composition for use according to claim 1, the compound represented by a structure of Formula 10, 11, 12, or 13 or stereoisomer thereof or pharmaceutically acceptable salt thereof.

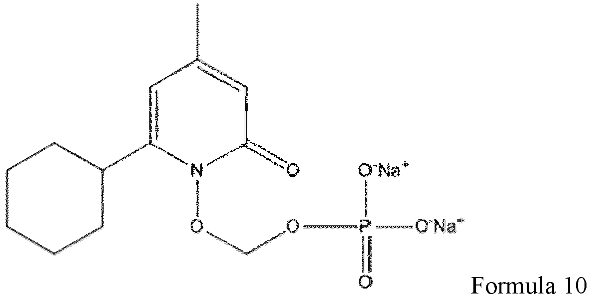

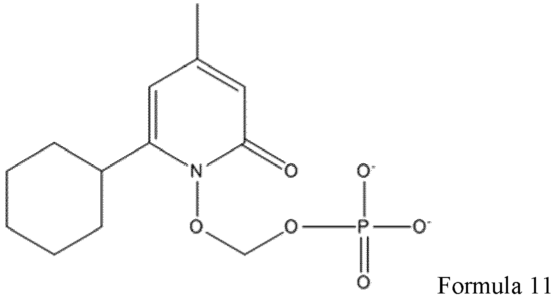

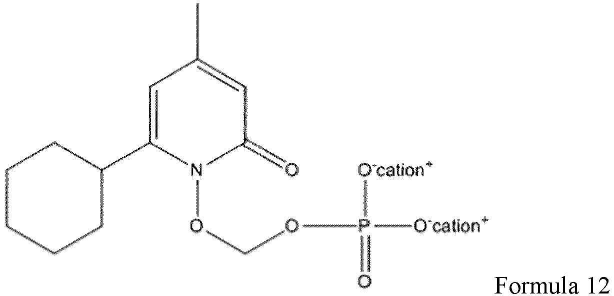

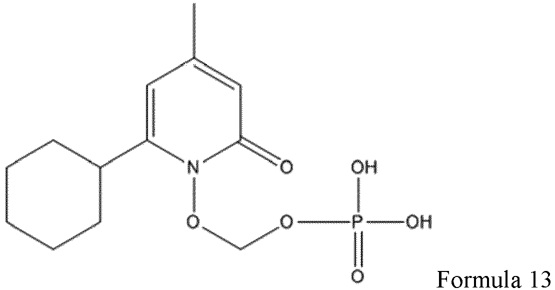

10. The pharmaceutical composition for use according to any one of claims 1 to 9, wherein the composition comprises the compound in a therapeutically effective amount and a pharmaceutically acceptable carrier.

11. The pharmaceutical composition for use according to any one of claims 1 to 9, wherein the composition is administered in an effective amount to provide a therapeutically effective amount of 6-cyclohexyl-1-hydroxy-4-methylpyridin-2(1H)-one.

12. The pharmaceutical composition for use according to claim 10 as dependent on claim 1, wherein the therapeutically effective amount is sufficient for inhibiting: the NOTCH pathway, the TGF-Beta pathway, the STAT3 pathway, growth of bladder cancer cells, DNA repair, ribonucleotide reductase, growth of bladder cancer stem cells, growth of bladder cancer cells at S-phase, bladder cancer spheroid formation, or bladder cancer cell proliferation.

13. The pharmaceutical composition for use according to any one of claims 1 to 9, wherein the bladder cancer is NMIBC (non-muscle invasive bladder cancer).

14. The pharmaceutical composition for use according to any one of claims 1 to 9, wherein the bladder cancer is MIBC (muscle invasive bladder cancer).

15. The pharmaceutical composition for use according to any one of claims 1 to 9, wherein the bladder cancer is in an upper urinary tract.

## Patentansprüche

1. Pharmazeutische Zusammensetzung, die Folgendes aufweist: Ciclopirox oder Ciclopirox-Olamin oder ein Ciclopirox-POM-Prodrug, das eine Struktur der Formel 1 oder ein Stereoisomer davon oder pharmazeutisch verträgliches Salz davon aufweist, für die Verwendung bei der Behandlung von Blasenkrebs, wobei: 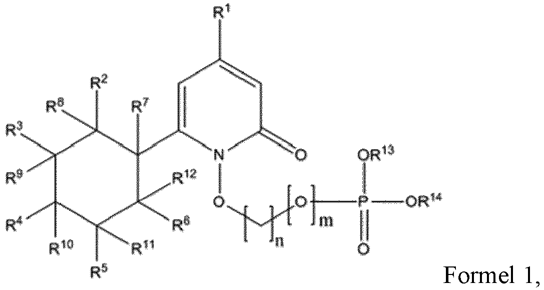 und wobei:
R¹-R¹² jeweils unabhängig eines oder mehrere von Folgenden einschließen: Wasserstoff, Halogenen, Hydroxylen, Alkoxylen, geraden Aliphaten, verzweigten Aliphaten, cyclischen Aliphaten, substituierten Aliphaten, unsubstituierten Aliphaten, gesättigten Aliphaten, ungesättigten Aliphaten, Aromaten, Polyaromaten, substituierten Aromaten, Heteroaromaten, Aminen, primären Aminen, sekundären Aminen, tertiären Aminen, aliphatischen Aminen, Carbonylen, Carboxylen, Amiden, Estern, Aminosäuren, Peptiden, Polypeptiden, substituiert oder unsubstituiert, oder Kombinationen davon;
R¹³-R¹⁴ jeweils unabhängig eines oder mehrere von Folgenden einschließen: einem positiven Ion, Natriumion, Wasserstoff, Halogenen, Hydroxylen, Alkoxylen, geraden Aliphaten, verzweigten Aliphaten, cyclischen Aliphaten, substituierten Aliphaten, unsubstituierten Aliphaten, gesättigten Aliphaten, ungesättigten Aliphaten, Aromaten, Polyaromaten, substituierten Aromaten, Heteroaromaten, Aminen, primären Aminen, sekundären Aminen, tertiären Aminen, aliphatischen Aminen, Carbonylen, Carboxylen, Amiden, Estern, Aminosäuren, Peptiden, Polypeptiden, substituiert oder unsubstituiert, oder Kombinationen davon;
n 0-20 ist, substituiert oder unsubstituiert; und
m 0, 1 oder 2 ist.

2. Pharmazeutische Zusammensetzung für die Verwendung nach Anspruch 1, die Verbindung, die dargestellt ist durch eine Struktur von Formel 2 oder 3 oder ein Stereoisomer von Formel 2 oder Formel 3 oder pharmazeutisch verträgliches Salz von Formel 2 oder Formel 3:

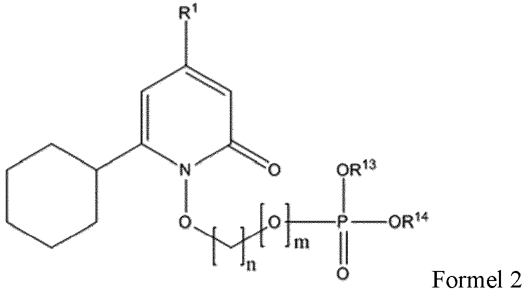

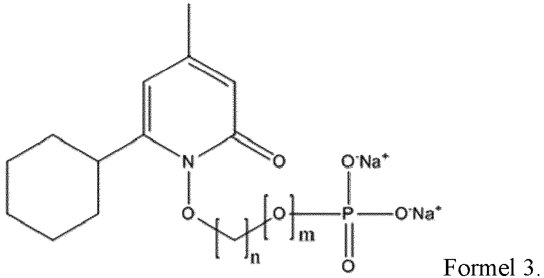

3. Pharmazeutische Zusammensetzung für die Verwendung nach Anspruch 1, die Verbindung, die dargestellt ist durch eine Struktur von Formel 4 oder ein Stereoisomer davon oder pharmazeutisch verträgliches Salz davon:

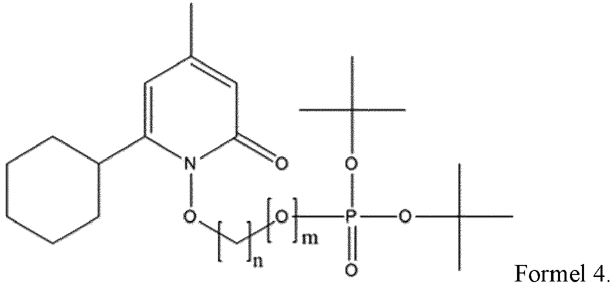

4. Pharmazeutische Zusammensetzung für die Verwendung nach Anspruch 1, die Verbindung, die dargestellt ist durch eine Struktur von Formel 5 oder ein Stereoisomer davon oder pharmazeutisch verträgliches Salz davon:

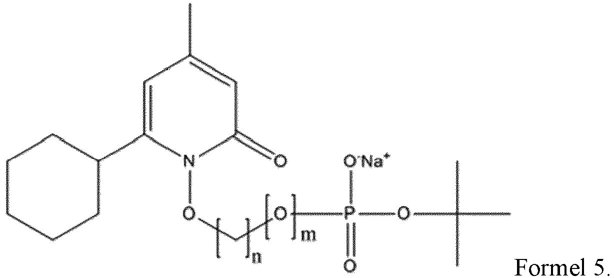

5. Pharmazeutische Zusammensetzung für die Verwendung nach Anspruch 1, die Verbindung, die dargestellt ist durch eine Struktur von Formel 6 oder ein Stereoisomer davon oder pharmazeutisch verträgliches Salz davon:

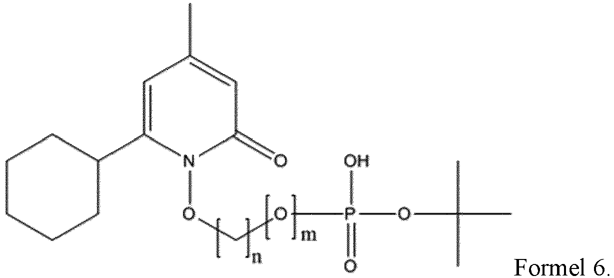

6. Pharmazeutische Zusammensetzung für die Verwendung nach Anspruch 1, die Verbindung, die dargestellt ist durch eine Struktur von Formel 7 oder ein Stereoisomer davon oder pharmazeutisch verträgliches Salz davon:

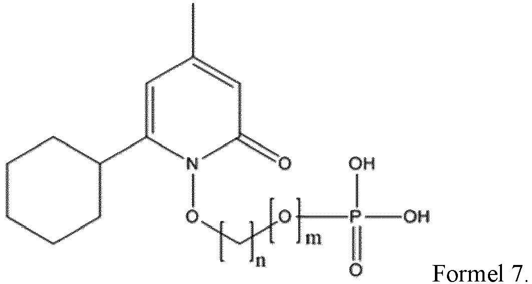

7. Pharmazeutische Zusammensetzung für die Verwendung nach Anspruch 1, die Verbindung, die dargestellt ist durch eine Struktur von Formel 8 oder ein Stereoisomer davon oder pharmazeutisch verträgliches Salz davon:

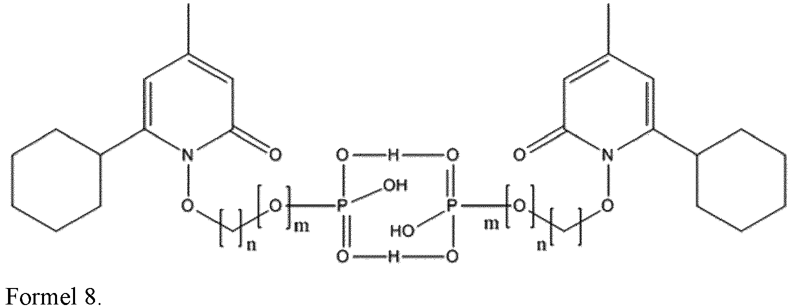

8. Pharmazeutische Zusammensetzung für die Verwendung nach Anspruch 1, die Verbindung, die dargestellt ist durch eine Struktur von Formel 9 oder ein Stereoisomer davon oder pharmazeutisch verträgliches Salz davon:

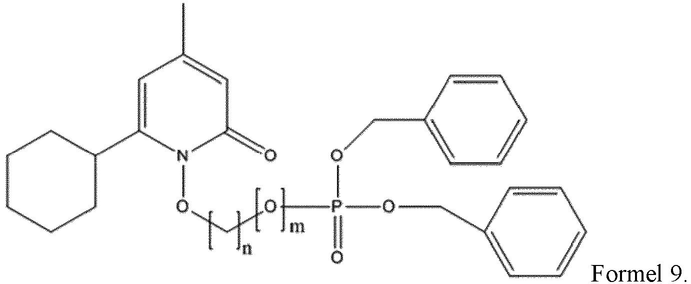

9. Pharmazeutische Zusammensetzung für die Verwendung nach Anspruch 1, die Verbindung, die dargestellt ist durch eine Struktur von Formel 10, 11, 12 oder 13 oder ein Stereoisomer davon oder pharmazeutisch verträgliches Salz davon:

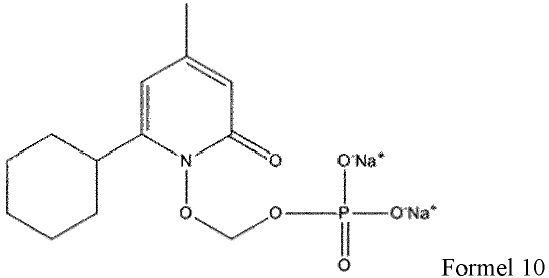

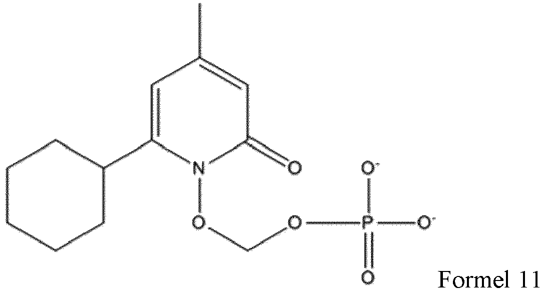

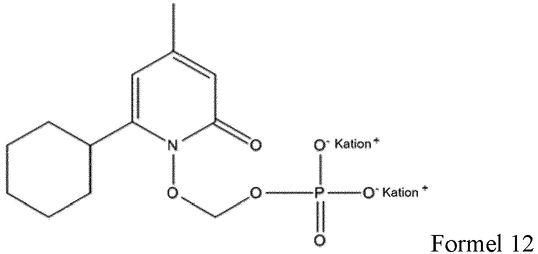

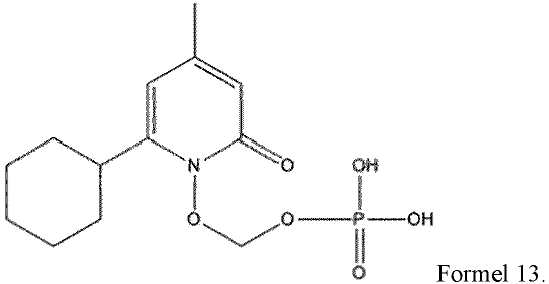

10. Pharmazeutische Zusammensetzung für die Verwendung nach einem der Ansprüche 1 bis 9, wobei die Zusammensetzung die Verbindung in einer therapeutisch wirksamen Menge und einen pharmazeutisch verträglichen Träger umfasst.

11. Pharmazeutische Zusammensetzung für die Verwendung nach einem der Ansprüche 1 bis 9, wobei die Zusammensetzung in einer wirksamen Menge verabreicht wird, um eine therapeutisch wirksame Menge von 6-Cyclohexyl-1-hydroxy-4-methylpyridin-2(1H)-on bereitzustellen.

12. Pharmazeutische Zusammensetzung für die Verwendung nach Anspruch 10 in Abhängigkeit von Anspruch 1, wobei die therapeutisch wirksame Menge zur Inhibierung von Folgenden ausreicht: dem NOTCH-Weg, dem TGF-Beta-Weg, dem STAT3-Weg, Wachstum von Blasenkrebszellen, DNA-Reparatur, Ribonukleotidreduktase, Wachstum von Blasenkrebs-Stammzellen, Wachstum von Blasenkrebszellen in der S-Phase, Blasenkrebs-Sphäroid-Bildung oder Blasenkrebszellproliferation.

13. Pharmazeutische Zusammensetzung für die Verwendung nach einem der Ansprüche 1 bis 9, wobei der Blasenkrebs NMIBC (nicht-muskelinvasiver Blasenkrebs) ist.

14. Pharmazeutische Zusammensetzung für die Verwendung nach einem der Ansprüche 1 bis 9, wobei der Blasenkrebs MIBC (muskelinvasiver Blasenkrebs) ist.

15. Pharmazeutische Zusammensetzung für die Verwendung nach einem der Ansprüche 1 bis 9, wobei der Blasenkrebs in einem oberen Harntrakt vorliegt.

## Revendications

1. Composition pharmaceutique contenant du ciclopirox ou du ciclopirox olamine ou un précurseur ciclopirox-POM ayant une structure de Formule 1 ou un stéréoisomère de celui-ci ou un sel pharmaceutiquement acceptable de celui-ci pour une utilisation dans le traitement d'une tumeur de la vessie, où 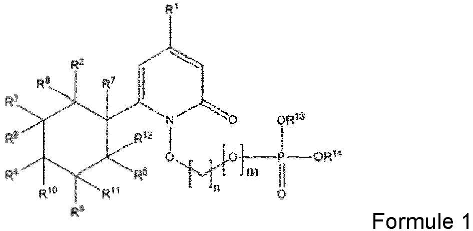 et où
R¹-R¹² incluent chacun indépendamment un ou plusieurs des suivants : hydrogène, halogènes, hydroxyles, alkoxys, aliphatiques linéaires, aliphatiques ramifiés, aliphatiques cycliques, aliphatiques substitués, aliphatiques non substitués, aliphatiques saturés, aliphatiques insaturés, aromatiques, polyaromatiques, aromatiques substitués, hétéroaromatiques, amines, amines primaires, amines secondaires, amines tertiaires, amines aliphatiques, carbonyles, carboxyles, amides, esters, acides aminés, peptides, polypeptides, substitués ou non substitués, ou combinaisons de ceux-ci ;
R¹³-R¹⁴ incluent chacun indépendamment un ou plusieurs des suivants : ion positif, ion sodium, hydrogène, halogènes, hydroxyles, alkoxys, aliphatiques linéaires, aliphatiques ramifiés, aliphatiques cycliques, aliphatiques substitués, aliphatiques non substitués, aliphatiques saturés, aliphatiques insaturés, aromatiques, polyaromatiques, aromatiques substitués, hétéroaromatiques, amines, amines primaires, amines secondaires, amines tertiaires, amines aliphatiques, carbonyles, carboxyles, amides, esters, acides aminés, peptides, polypeptides, substitués ou non substitués, ou combinaisons de ceux-ci ;
n représente 0-20 radicaux substitués ou non substitués ; et
m prend la valeur 0, 1 ou 2.

2. Composition pharmaceutique pour une utilisation selon la revendication 1, composé représenté par une structure de Formule 2 ou de Formule 3, ou stéréoisomère de Formule 2 ou de Formule 3 ou sel pharmaceutiquement acceptable de Formule 2 ou de Formule 3.

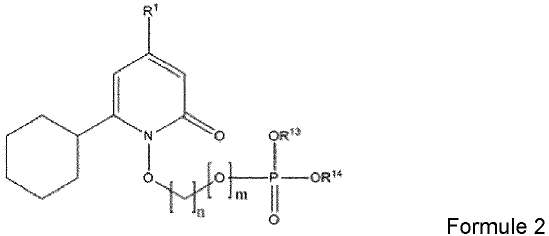

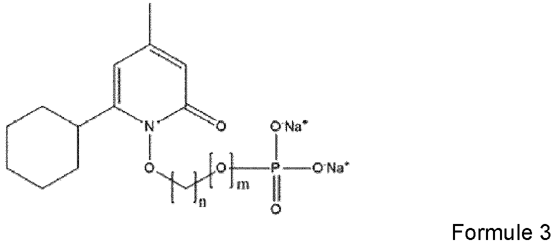

3. Composition pharmaceutique pour une utilisation selon la revendication 1, composé représenté par une structure de Formule 4 ou stéréoisomère de celui-ci ou sel pharmaceutiquement acceptable de celui-ci.

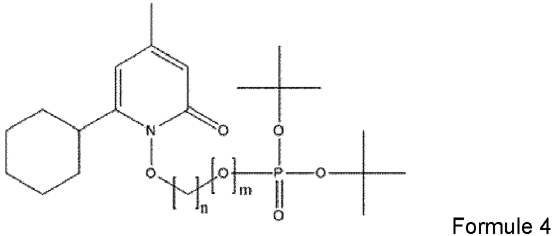

4. Composition pharmaceutique pour une utilisation selon la revendication 1, composé représenté par une structure de Formule 5 ou stéréoisomère de celui-ci ou sel pharmaceutiquement acceptable de celui-ci.

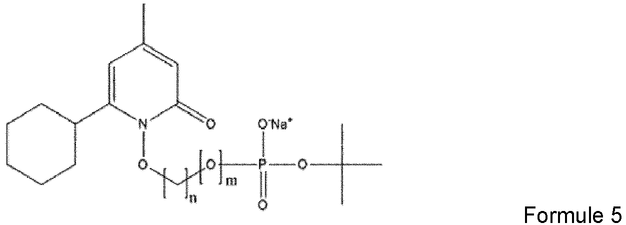

5. Composition pharmaceutique pour une utilisation selon la revendication 1, composé représenté par une structure de Formule 6 ou stéréoisomère de celui-ci ou sel pharmaceutiquement acceptable de celui-ci.

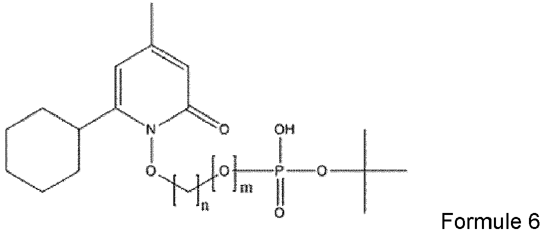

6. Composition pharmaceutique pour une utilisation selon la revendication 1, composé représenté par une structure de Formule 7 ou stéréoisomère de celui-ci ou sel pharmaceutiquement acceptable de celui-ci.

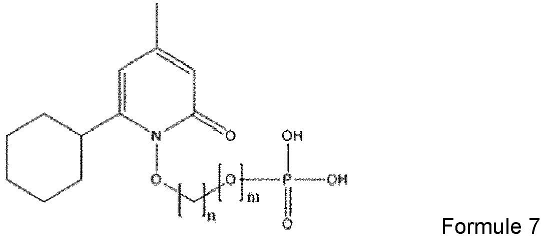

7. Composition pharmaceutique pour une utilisation selon la revendication 1, composé représenté par une structure répondant de Formule 8 ou stéréoisomère de celui-ci ou sel pharmaceutiquement acceptable de celui-ci.

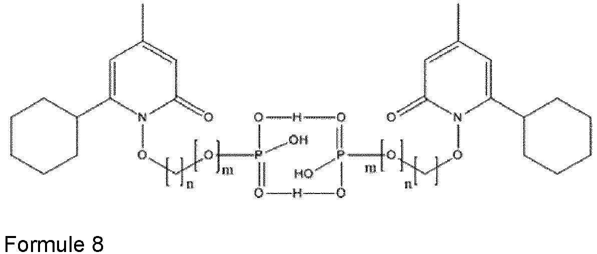

8. Composition pharmaceutique pour une utilisation selon la revendication 1, composé représenté par une structure de Formule 9 ou stéréoisomère de celui-ci ou sel pharmaceutiquement acceptable de celui-ci.

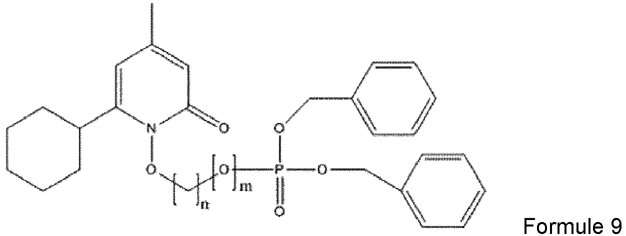

9. Composition pharmaceutique pour une utilisation selon la revendication 1, composé représenté par une structure de Formule 10, 11, 12 ou 13 ou stéréoisomère de celui-ci ou sel pharmaceutiquement acceptable de celui-ci.

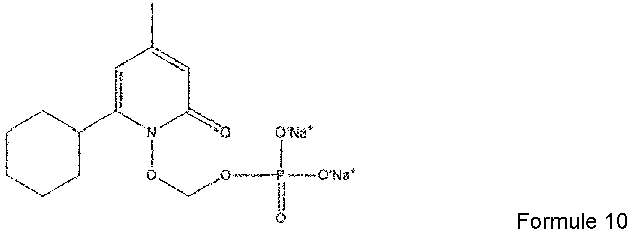

10. Composition pharmaceutique pour une utilisation selon l'une quelconque des revendications 1 à 9, où la composition comprend le composé à une quantité thérapeutiquement efficace et un véhicule pharmaceutiquement acceptable.

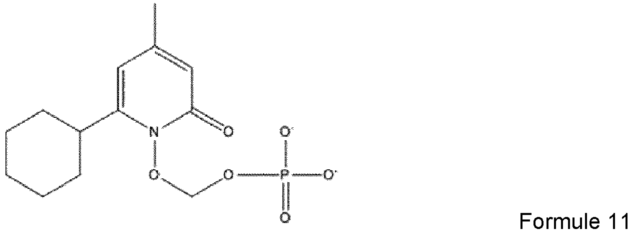

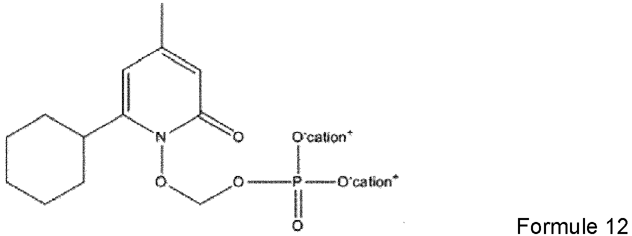

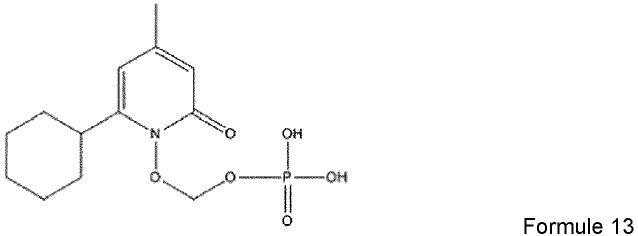

11. Composition pharmaceutique pour une utilisation selon l'une quelconque des revendications 1 à 9, où la composition est administrée à une quantité efficace pour fournir une quantité thérapeutiquement efficace de 6-cyclohexyl-1-hydroxy-4-méthylpyridin-2(1H)-one.

12. Composition pharmaceutique pour une utilisation selon la revendication 10 placée sous la dépendance de la revendication 1, où la quantité thérapeutiquement efficace est suffisante pour inhiber: la voie NOTCH, la voie TGF-bêta, la voie STAT3, la croissance de cellules vésicales tumorales, la réparation de l'ADN, la ribonucléotide réductase, la croissance de cellules souches vésicales tumorales, la croissance de cellules vésicales en phase S, la formation de sphéroïdes tumoraux dans la vessie ou la prolifération de cellules vésicales tumorales.

13. Composition pharmaceutique pour une utilisation selon l'une quelconque des revendications 1 à 9, où la tumeur de la vessie est une TVNIM (tumeur de la vessie n'infiltrant pas le muscle vésical).

14. Composition pharmaceutique pour une utilisation selon l'une quelconque des revendications 1 à 9, où la tumeur de la vessie est une TVIM (tumeur de la vessie infiltrant le muscle vésical).

15. Composition pharmaceutique pour une utilisation selon l'une quelconque des revendications 1 à 9, où la tumeur de la vessie affecte le haut appareil urinaire.
